(19) Europäisches Patentamt / European Patent Office / Office européen des brevets

(11) EP 1 689 445 B1

(12) EUROPÄISCHE PATENTSCHRIFT

(45) Veröffentlichungstag und Bekanntmachung des Hinweises auf die Patenterteilung:
**25.02.2015 Patentblatt 2015/09**

(21) Anmeldenummer: **04797900.0**

(22) Anmeldetag: **15.11.2004**

(51) Int Cl.:
***C12N 15/86*** $^{(2006.01)}$ ***A61K 48/00*** $^{(2006.01)}$
***C12N 15/861*** $^{(2006.01)}$

(86) Internationale Anmeldenummer:
**PCT/EP2004/012930**

(87) Internationale Veröffentlichungsnummer:
**WO 2005/051430 (09.06.2005 Gazette 2005/23)**

(54) **NEUE VERWENDUNG VON ADENOVIREN UND DAFÜR CODIERENDE NUKLEINSÄUREN**
NOVEL USE OF ADENOVIRUSES AND NUCLEIC ACIDS THAT CODE FOR SAID VIRUSES
NOUVELLE UTILISATION D'ADENOVIRUS ET D'ACIDES NUCLEIQUES LES CODANT

(84) Benannte Vertragsstaaten:
**AT BE BG CH CY CZ DE DK EE ES FI FR GB GR HU IE IS IT LI LU MC NL PL PT RO SE SI SK TR**

(30) Priorität: **14.11.2003 DE 10353262**
**14.04.2004 DE 102004018099**

(43) Veröffentlichungstag der Anmeldung:
**16.08.2006 Patentblatt 2006/33**

(73) Patentinhaber: **Holm, Per Sonne**
**82256 Fürstenfeldbruck (DE)**

(72) Erfinder: **Holm, Per Sonne**
**82256 Fürstenfeldbruck (DE)**

(74) Vertreter: **Bohmann, Armin K.**
**Bohmann**
**Anwaltssozietät**
**Nymphenburger Straße 1**
**80335 München (DE)**

(56) Entgegenhaltungen:
**EP-A- 0 931 830** **WO-A-00/22136**
**WO-A-03/033692** **WO-A-03/099859**
**US-A1- 2002 086 411**

- **WONG H K ET AL: "Complementary functions of E1a conserved region 1 cooperate with conserved region 3 to activate adenovirus serotype 5 early promoters" JOURNAL OF VIROLOGY, THE AMERICAN SOCIETY FOR MICROBIOLOGY, US, Bd. 68, Nr. 8, August 1994 (1994-08), Seiten 4910-4920, XP002260278 ISSN: 0022-538X**
- **HOWE J A ET AL: "Evaluation of E1-mutant adenoviruses as conditionally replicating agents for cancer therapy" MOLECULAR THERAPY, ACADEMIC PRESS, SAN DIEGO, CA,, US, Bd. 2, Nr. 5, November 2000 (2000-11), Seiten 485-495, XP002260276 ISSN: 1525-0016**
- **HOLM P S ET AL: "YB-1 relocates to the nucleus in adenovirus infected cells and facilitates viral replication E2 gene expression through the E2---late promoter" JOURNAL OF BIOLOGICAL CHEMISTRY, AMERICAN SOCIETY OF BIOLOGICAL CHEMISTS, BALTIMORE, MD, US, Bd. 277, Nr. 12, 22. März 2002 (2002-03-22), Seiten 10427-10434, XP002237181 ISSN: 0021-9258**
- **BARGOU R C ET AL: "NUCLEAR LOCALIZATION AND INCREASED LEVELS OF TRANSCRIPTION FACTOR YB-1 IN PRIMARY HUMAN BREAST CANCERS ARE ASSOCIATED WITH INTRINSIC MDR1 GENE EXPRESSION" NATURE MEDICINE, NATURE PUBLISHING, CO, US, Bd. 3, Nr. 4, April 1997 (1997-04), Seiten 447-450, XP001148855 ISSN: 1078-8956**
- **GANLY I ET AL: "Replication and cytolysis of an E1B-attenuated adenovirus in drug-resistant ovarian tumour cells is associated with reduced apoptosis" GENE THERAPY, MACMILLAN PRESS LTD., BASINGSTOKE, GB, Bd. 8, Nr. 5, März 2001 (2001-03), Seiten 369-375, XP002260275 ISSN: 0969-7128**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 689 445 B1

- HEISE ET AL: "ONYX-015, an E+B gene-attenuated adenovirus, causes tumor-specific cytolysis and antitumoral efficacy that can be augmented by standard chemotherapeutic agents" NATURE MEDICINE, NATURE PUBLISHING, CO, US, Bd. 3, Nr. 6, Juni 1997 (1997-06), Seiten 639-645, XP002095383 ISSN: 1078-8956
- MYMRYK JOE S ET AL: "Induction of gene expression by exon 2 of the major E1A proteins of adenovirus type 5", JOURNAL OF VIROLOGY, vol. 67, no. 12, 1993, pages 6922-6928, ISSN: 0022-538X
- CHU R L ET AL: "Use of Replicating Oncolytic Adenoviruses in Combination Therapy for Cancer", CLINICAL CANCER RESEARCH, THE AMERICAN ASSOCIATION FOR CANCER RESEARCH, US LNKD- DOI: 10.1158/1078-0432.CCR-0349-03, vol. 10, 15 August 2004 (2004-08-15), pages 5299-5312, XP003014156, ISSN: 1078-0432
- NOYA FRANCISCO ET AL: "Activation of adenovirus early promoters and lytic phase in differentiated strata of organotypic cultures of human keratinocytes.", JOURNAL OF VIROLOGY, vol. 77, no. 11, June 2003 (2003-06), pages 6533-6540, ISSN: 0022-538X
- HOLM PER S ET AL: "Multidrug-resistant cancer cells facilitate E1-independent adenoviral replication: Impact for cancer gene therapy", CANCER RESEARCH, AMERICAN ASSOCIATION FOR CANCER REREARCH, US LNKD- DOI:10.1158/0008-5472.CAN-0482-2, vol. 64, no. 1, 1 January 2004 (2004-01-01), pages 322-328, XP002387405, ISSN: 0008-5472
- "Effect of adenovirus dl520 as replicating agent in combination with fractionated radiotherapy in a glioma xenograft model", MOLECULAR THERAPY, ACADEMIC PRESS, SAN DIEGO, CA, US, vol. 9, 1 May 2004 (2004-05-01), page 232, XP004634978, ISSN: 1525-0016
- "The multidrug resistant phenotype in tumor cells facilitates adenoviral replication: new concepts for oncolytic vector development", MOLECULAR THERAPY, ACADEMIC PRESS, SAN DIEGO, CA, US, vol. 9, 1 May 2004 (2004-05-01), page 168, XP004634806, ISSN: 1525-0016
- KHURI F R ET AL: "a controlled trial of intratumoral ONYX-015, a selectively-replicating adenovirus, in combination with cisplatin and 5-fluorouracil in patients with recurrent head and neck cancer", NATURE MEDICINE, NATURE PUBLISHING GROUP, NEW YORK, NY, US, vol. 6, no. 8, 1 August 2000 (2000-08-01), pages 879-885, XP008126723, ISSN: 1078-8956, DOI: 10.1038/78638

## Beschreibung

Die vorliegende Erfindung betrifft die Verwendung eines Adenovirus zur Herstellung eines Medikamentes, die Verwendung einer für den Adenovirus codierenden Nukleinsäure zur Herstellung eines Medikamentes, die Verwendung eines mit YB-1 wechselwirkenden Mittels zur Charakterisierung von Zellen, Zellen eines Tumorgewebes oder Patienten, und ein Kit umfassend den Adenovirus gemäß den Ansprüchen.

Bei der Behandlung von Tumoren werden derzeit eine Vielzahl von Therapiekonzepten verfolgt. Neben der Verwendung chirurgischer Techniken stehen dabei die Chemotherapie und die Strahlentherapie im Vordergrund. All diese Techniken sind jedoch für den Patienten mit nicht unerheblichen Nebenwirkungen verbunden. Mit der Verwendung von replikationsselektiven onkolytischen Viren wurde eine neue Plattform für die Behandlung von Tumoren geschaffen. Dabei wird eine selektive intratumorale Replikation eines viralen Agens herbeigeführt, die in der Folge zur Virusreplikation, Lyse der infizierten Tumorzelle und Verbreitung des Virus auf benachbarte Tumorzellen führt. Infolge der Beschränkung der Replikationsfähigkeit des Virus auf Tumorzellen bleibt normales Gewebe von der Replikation und damit der Lyse durch das Virus verschont.

Derzeit finden verschiedene virale Systeme in klinischen Studien mit dem Ziel der Tumorlyse Anwendung. Ein Beispiel für einen derartigen Adenovirus ist d11520 (Onyx-015), der bereits erfolgreich in den klinischen Phasen I und II eingesetzt wurde (Khuri, F. et al. Nature Medicine 6, 879-885, 2000). Onyx-015 ist ein Adenovirus, bei dem das E1B-55kDA-Gen vollständig deletiert ist. Die vollständige Deletion des E1B55kDa-Proteins des Adenovirus beruht dabei auf der Beobachtung, dass im adenoviralen Vektor die Replikation und somit die Lyse von Zellen mit defektem p53 möglich ist (Kirn, D. et al., Proc. Am. Soc. Clin. Oncol. 17, 391a, 1998), wobei normale Zellen nicht geschädigt werden. Genauer ist das E1B-55kDa-Genprodukt beteiligt an der Inhibierung von p53, dem Transport viraler mRNA und dem Abschalten der Proteinsynthese der Wirtszelle. Die Inhibierung von p53 erfolgt dabei durch Ausbilden eines Komplexes aus p53 und dem adenoviral codierten E1B-55kDa Protein und/oder Komplex bestehend aus E1B-55kDA und E4orf6. Von p53, codiert von TP53, geht ein komplexer regulatorischer Mechanismus aus (Zambetti, G.P. et al., FASEB J. 7, 855-865, 1993), der u.a. auch dazu führt, dass eine effiziente Replikation von Viren wie Adenoviren in der Zelle unterdrückt wird. Das Gen TP 53 ist in etwa 50 % aller menschlichen Tumoren deletiert oder mutiert mit der Folge, dass es zu keiner - erwünschten - Apoptose infolge einer Chemotherapie oder einer Bestrahlungstherapie kommt und damit der Erfolg dieser Tumorbehandlungen im Normalfall unterbleibt.

Ein weiteres Konzept tumorlytischer Viren beruht auf der Beobachtung, dass wenn das E1A-Protein in einer bestimmten Weise deletiert vorliegt bzw. eine oder mehrere Mutationen aufweist, welche die Bindung von Rb/E2F und/oder p107/E2F und/oder p130/E2F nicht beeinflussen, solche Adenoviren den Eintritt der infizierten Zellen in die S-Phase nicht induzieren und zur Replikation in Tumorzellen befähigt sind, die kein funktionelles Rb-Protein aufweisen. Zudem kann das E1A-Protein am N-Terminus deletiert vorliegen bzw. eine oder mehrere Mutationen im Bereich der Aminosäurepositionen 1 bis 76 des E1A-Proteins umfassen, um die Bindung von E1A an p300 zu unterbinden, um somit eine selektivere Replikation in Tumorzellen zu bewerkstelligen. Diese Vorgehensweisen sind beispielhaft beschrieben in dem europäischen Patent EP 0 931 830. Beispiele derartiger Adenoviren sind Ad$\Delta$24, d1922 - 947, E1Ad/01/07 und CB016 (Howe, J. A. et al., Molecular Therapy 2, 485-495, 2000; Fueyo, J. et al., Oncogene 19, 2-12, 2000; Heise, C. et al., Nature Medicine 6, 11341139, 2001; Balague, C. et al., J. Virol. 75, 7602-7611, 2001). Diese im Stand der Technik bekannten adenoviralen Systeme zur Onkolyse weisen somit bestimmte Deletionen im E1A Protein auf, wobei diese Deletion vorgenommen worden war unter der Annahme, dass intakte Rb-Proteine bzw. Komplex bestehend aus intaktem Rb-Protein und E2F einer effiziente Replikation in vivo entgegenwirken würden und um eine adenovirale Replikation in vivo nur in Rb-negativen/mutierten Zellen sicher zu stellen. Diese adenoviralen Systeme nach dem Stand der Technik gehen auf E1A zurück, um mittels des frühen E2-Promotors (engl. E2 early Promotor) und freiem E2F die in vivo Replikation zu steuern (Dyson, N. Genes & Development, 12, 2245-2262, 1998).

Eine weitere Form von tumorlytischen adenoviralen Systemen beruht auf dem Einsatz selektiver Promotoren, um das virale Onkogen E1A spezifisch zu exprimieren, was eine selektive Replikation in Tumorzellen erlaubt (Rodriguez, R. et al., Cancer Res. 57,2559-2563, 1997).

Wie vorstehend beschrieben kommt es bei den verschiedenen Konzepten adenoviraler tumorlytischer Viren auf die Auswahl eines für das dem jeweiligen Konzept zugrundeliegenden Wirkmechanismus geeigneten zellulären Hintergrundes an. Mit anderen Worten, die verschiedenen derzeit bekannten adenoviralen Systeme zur Tumorlyse können nur bei Vorliegen bestimmter molekularbiologischer Voraussetzungen angewandt werden. Dies beschränkt die Anwendung derartiger Systeme auf bestimmte Patientenkollektive.

Ein besonderes Problem bei der Behandlung von Tumorerkrankungen stellt sich dann ein, wenn die Patienten eine sogenannte klassische Vielfachresistenz (engl. multidrug resistence (MDR)) entwickeln, die eine besonders gut untersuchte Resistenzform von Tumoren gegenüber Zytostatika darstellt (Gottesman und Pastan, Annu. Rev. Biochem. 62, 385-427, 1993). Sie beruht auf der Überexpression des membranständigen Transportproteins P-Glykoprotein, das zu den sogenannten ABC-Transportern gehört (Stein, U. et al., JBC 276, 28562-69, 2001, J. Wijnholds, Novartis Found Symp., 243, 69-79, 2002). Bargou, R. C. et al. und Oda, Y. et al (Bargou, R. C. et al., Nature Medicine 3, 447-450, 1997; Clin. Cancer Res. 4, 2273-2277, 1998) konnten zeigen, dass die Kernlokalisation des humanen Transkriptionsfaktors YB-1 unmittelbar an der Aktivierung der Expression des P-Glykoproteins beteiligt ist. Weitere Untersuchungen belegen, dass YB-1 durch verschiedene Streßbedingungen in den Zellkern gelangt, wie z.B. UV-Bestrahlung, Zytostatika-Applikation (Koike, K. et al., FEBS Lett 17, 390-394, 1997) und Hyperthermie (Stein, U. et al., JBC 276, 28562-69, 2001). Weitere Untersuchungen belegen, dass die nukläre Lokalisation von YB-1 einen Einfluss auf einen weiteren ABC-Transporter ausübt. Dieser ABC-Transporter wird als MRP (engl. multidrug resistance-related protein) bezeichnet und ist mit der Ausbildung des sogenannten atypischen, nicht P-Glykoproteinabhängigen Vielfachresistenz beteiligt (Stein, U. et al., JBC 276, 28562-69, 2001).

Die US-Patentanmeldung 2002/0086411 A1 offenbart Mittel zur Behandlung von malignen Erkrankungen unter Verwendung des Proteins YB-1.

Noya, F et al. (2003) (J.Virol. 77, No. 11, p. 6533 - 6540) beschreiben die Aktivierung des frühen adenoviralen Promoters und die lytische Phase in differenzierten Schichten organotypischer Kulturen menschlicher Keratinocyten.

Die internationale Patentanmeldung WO 03/033692 offenbart die Verwendung des adenoviralen E2-late Promotors.

Der vorliegenden Erfindung liegt die Aufgabe zugrunde, eine technische Lehre und insbesondere ein Mittel bereitzustellen, welches erlaubt, einen Organismus, speziell einen menschlichen Organismus bzw. ein Patientenkollektiv spezifisch mit tumorlytisch wirkenden Agenzien zu behandeln. Weiterhin ist es eine der vorliegenden Erfindung zugrundeliegenden Aufgabe, ein Mittel bereitzustellen, welches geeignet ist, bei Patienten mit Tumorerkrankungen eine Tumorlyse herbeizuführen, die gegenüber Zytostatika resistent sind, insbesondere solche, die eine Vielfachresistenz zeigen.

Die der vorliegenden Erfindung zugrunde liegende Aufgabe wird erfindungsgemäß gelöst durch den Gegenstand der beigefügten unabhängigen Ansprüche. Bevorzugte Ausführungsformen ergeben sich aus den beigefügten abhängigen Ansprüchen.

Erfindungsgemäß wird die Aufgabe in einem ersten Aspekt gelöst durch die Verwendung eines Adenovirus zur Herstellung eines Medikamentes, dadurch gekennzeichnet, dass der Adenovirus replikationsdefizient ist in Zellen, die YB-1 nicht im Kern aufweisen, der Adenovirus in Zellen repliziert, die YB-1 im Kern aufweisen, und der Adenovirus für ein virales Onkogenprotein E1A codiert, das zumindest ein adenovirales Gen transaktiviert, wobei das adenovirale Gen ausgewählt ist aus der Gruppe, die E1B55kDa, E4orf6, E4orf3 und E3ADP besteht, und das Medikament eine Kombination von mindestens zwei Mitteln umfasst, wobei ein jedes Mittel einzeln und unabhängig aus der Gruppe ausgewählt ist, die aus Cytostatika besteht, und wobei mindestens ein Mittel den Transport von YB-1 in den Zellkern vermittelt.

In einer Ausführungsform des ersten Aspektes ist vorgesehen, dass das virale Onkoprotein E1A nicht die nukleäre Lokalisation von YB-1 induziert.

In einer Ausführungsform des ersten Aspektes ist vorgesehen, dass das Medikament für Patienten ist, deren Zellen Rb-positiv oder Rb-negativ sind.

In einer Ausführungsform des ersten Aspektes ist vorgesehen, dass das Medikament für die Behandlung von Tumoren ist.

In einer Ausführungsform des ersten Aspektes ist vorgesehen, dass die Zellen, insbesondere die den Tumor oder Teile davon ausbildenden Zellen eine Resistenz, insbesondere Mehrfachresistenz gegen pharmakologische Wirkstoffe, bevorzugter Weise Antitumormittel und bevorzugtererweise Zytostatika, aufweisen.

In einer Ausführungsform des ersten Aspektes ist vorgesehen, dass die Zellen eine Expression, bevorzugterweise eine Überexpression des membranständigen Transportproteins P-Glykoprotein zeigen.

In einer Ausführungsform des ersten Aspektes ist vorgesehen, dass die Zellen p53-positiv oder p53-negativ sind.

In einer Ausführungsform des ersten Aspektes ist vorgesehen, dass das Onkogenprotein gegenüber dem Wildtyp-Onkogenprotein E1A eine oder mehrere Mutationen oder Deletionen aufweist, wobei die Deletion bevorzugt eine solche ist, die ausgewählt ist aus der Gruppe, die Deletionen des Bereichs CR3 und Deletionen des N-Terminus und Deletionen des C-Terminus umfasst.

In einer Ausführungsform des ersten Aspektes ist vorgesehen, dass das E1A-Onkogenprotein an Rb binden kann.

In einer Ausführungsform des ersten Aspektes ist vorgesehen, dass das Onkogenprotein gegenüber dem Wildtyp-Onkogenprotein eine oder mehrere Mutationen oder Deletionen aufweist, wobei die Deletion bevorzugt eine solche in der CR1-Region und/oder der CR2-Region ist.

In einer Ausführungsform des ersten Aspektes ist vorgesehen, dass das Onkogenprotein E1A nicht an Rb zu binden in der Lage ist.

In einer Ausführungsform des ersten Aspektes ist vorgesehen, dass das virale Onkogenprotein unter der Kontrolle eines Gewebes- und/oder Tumor-spezifischen Promotors steht.

In einer Ausführungsform des ersten Aspektes ist vorgesehen, dass der Adenovirus für YB-1 codiert.

In einer Ausführungsform des ersten Aspektes ist vorgesehen, dass YB-1 unter der Kontrolle eines Gewebe- und/oder Tumor-spezifischen Promotors steht.

In einer Ausführungsform des ersten Aspektes ist vorgesehen, dass der Adenovirus für mindestens ein Protein codiert, das ausgewählt ist aus der Gruppe, die aus E4orf6, E4orf3, EIB55k und adenoviralem E3ADP-Protein besteht.

In einer Ausführungsform des ersten Aspektes ist vorgesehen, dass die Zellen YB-1 im Kern aufweisen, insbesondere die den Tumor oder einen Teil davon ausbildenden Zellen YB-1 im Kern aufweisen.

In einer Ausführungsform des ersten Aspektes ist vorgesehen, dass der Tumor YB-1 im Kern nach Induktion des Tranports von YB-1 in den Kern enthält.

In einer Ausführungsform des ersten Aspektes ist vorgesehen, dass der Transport von YB-1 in den Kern ausgelöst wird durch zumindest eine Maßnahme, die ausgewählt ist aus der Gruppe, die aus Bestrahlung, Gabe von Zytostatika und Hyperthermie besteht.

In einer Ausführungsform des ersten Aspektes ist vorgesehen, dass der Virus, insbesondere der Adenovirus, ausgewählt ist aus der Gruppe, die aus AdΔ24, d1922-947, E1Ad/01/07, d11119/1131, CB 016, d1520, und Viren, denen ein exprimiertes virales Onkogen fehlt, das zur Bindung eines funktionellen Rb-Tumorsuppressor-Genprodukts fähig ist, besteht.

In einer Ausführungsform des ersten Aspektes ist vorgesehen, dass der Adenovirus E1B 19 kDa-defizient ist.

Erfindungsgemäß wird die Aufgabe in einem zweiten Aspekt gelöst durch die Verwendung einer Nukleinsäure codierend für einen Adenovirus gemäß dem ersten Aspekt der Erfindung zur Herstellung eines Medikamentes, insbesondere zur Herstellung eines Medikamentes für die Behandlung von Tumoren, wobei das Medikament eine Kombination von mindestens zwei Mitteln umfasst, wobei ein jedes Mittel einzeln und unabhängig aus der Gruppe ausgewählt ist, die aus Cytostatika besteht, und wobei mindestens ein Mittel den Transport von YB-1 in den Zellkern vermittelt.

In einer Ausführungsform des zweiten Aspektes ist vorgesehen, dass die Zellen, insbesondere die den Tumor oder Teile davon ausbildenden Zellen, eine Resistenz, insbesondere eine Mehrfachresistenz gegen pharmakologische Wirkstoffe, bevorzugterweise Antitumormittel, und bevorzugtererweise Zytostatika, aufweisen.

In einer Ausführungsform des zweiten Aspektes ist vorgesehen, dass der Adenovirus so ausgebildet ist, dass die Replikation durch YB-1 über die Aktivierung des E2-Late-Promotors gesteuert wird, bevorzugterweise überwiegend über die Aktivierung des E2-Late-Promotors.

Erfindungsgemäß wird die Aufgabe in einem dritten Aspekt gelöst durch die Verwendung eines mit YB-1 wechselwirkenden Mittels zur Charakterisierung von Zellen, Zellen eines Tumorgewebes oder Patienten, um zu bestimmen, ob diese(r) mit einem Adenovirus gemäß dem ersten Aspekt der Erfindung kontaktiert und/oder behandelt werden sollen, wobei das mit YB-1 wechselwirkende Mittel ausgewählt ist aus der Gruppe, die aus Antikörpern, Aptameren, und Spiegelmeren besteht.

In einer Ausführungsform des ersten Aspektes ist vorgesehen, dass der Virus eine für ein Transgen codierende Nukleinsäure umfasst.

In einer Ausführungsform des ersten Aspektes ist vorgesehen, dass der Virus das Translations- und/oder das Transkriptionsprodukt eines Transgens umfasst.

In einer Ausführungsform des zweiten Aspektes ist vorgesehen, dass die Nukleinsäure ein Transgen oder eine für ein Transgen codierende Nukleinsäure umfasst.

In einer Ausführungsform des ersten und des zweiten Aspektes ist vorgesehen, dass das Transgen ausgewählt ist aus der Gruppe, die aus Prodruggenen, Zytokinen, Apoptoseinduzierenden Genen, Tumorsuppressorgenen, Genen für Metalloproteinasen-Inhibitoren und Genen für Angiogene-Inhibitoren besteht.

In einer Ausführungsform des ersten und des zweiten Aspektes ist vorgesehen, dass das Transgen ausgewählt ist aus der Gruppe, die aus Nukleinsäuren für siRNA, für Aptamere, für Antisense-Moleküle und für Ribozyme besteht, wobei die siRNA, die Aptamere, die Antisense-Moleküle und/oder die Ribozyme gegen ein Zielmolekül gerichtet ist.

In einer Ausführungsform des ersten und des zweiten Aspektes ist vorgesehen, dass das Zielmolekül ausgewählt ist aus der Gruppe, die aus Resistenz-relevanten Faktoren, Anti-Apoptose-Faktoren, Onkogenen, Angiogenese-Faktoren, DNA-Synthese-Enzymen, DNA-Reparaturenzymen, Wachstumsfaktoren, Rezeptoren für Wachstumsfaktoren, Transkriptionsfaktoren, Metalloproteinasen, insbesondere Matrix-Metalloproteinkinasen, und Plasminogenaktivator vom Urokinase-Typ besteht.

In einer Ausführungsform des ersten und des zweiten Aspektes ist vorgesehen, dass mindestens zwei der Mittel an unterschiedlichen Zielmolekülen angreifen.

In einer Ausführungsform des ersten und des zweiten Aspektes ist vorgesehen, dass mindestens zwei der Mittel über einen unterschiedlichen Wirkmechanismus aktiv sind.

In einer Ausführungsform des ersten und des zweiten Aspektes ist vorgesehen, dass mindestens ein Mittel die Infizierbarkeit einer Zelle erhöht, in der das Virus repliziert.

In einer Ausführungsform des ersten und des zweiten Aspektes ist vorgesehen, dass das mindestens eine weitere Mittel die Verfügbarkeit einer Komponente der Zelle beeinflusst, bevorzugterweise die Verfügbarkeit der Komponente erhöht, wobei die Komponente die Aufnahme des Virus vermittelt.

In einer Ausführungsform des ersten und des zweiten Aspektes ist vorgesehen, dass das mindestens eine den Transport von YB-1 in den Zellkern vermittelnde Mittel den Transport von YB-1 erhöht.

In einer Ausführungsform des ersten und des zweiten Aspektes ist vorgesehen, dass mindestens ein Mittel ein Histon-Deacylase-Inhibitor ist.

In einer Ausführungsform des ersten und des zweiten Aspektes ist vorgesehen, dass der Histon-Deacylase-Inhibitor aus der Gruppe ausgewählt ist, die aus Trichostatin A, FR 901228, MS-27-275, NVP-LAQ824, PXD101 Apicidin und Scriptaid besteht.

In einer Ausführungsform des ersten und des zweiten Aspektes ist vorgesehen, dass mindestens ein Mittel aus der Gruppe ausgewählt ist, die aus Trichostatin A, FR 901228, MS-27-275, NVP-LAQ824, PXD101 Apicidin und Scriptaid besteht.

In einer Ausführungsform des ersten und des zweiten Aspektes ist vorgesehen, dass mindestens ein Mittel ein Topoisomerase-Inhibitor ist.

In einer Ausführungsform des ersten und des zweiten Aspektes ist vorgesehen, dass der Topoisomerase-Inhibitor aus der Gruppe ausgewählt ist, die aus Camptothecin, Irinotecan, Topotecan, DX-895If, SN-38, 9-aminocamptothecin, 9-nitrocamptothecin, Daunorubicn und Etoposid besteht.

In einer Ausführungsform des ersten und des zweiten Aspektes ist vorgesehen, dass die Kombination aus Trichostatin A und Irinotecan besteht.

In einer Ausführungsform des ersten und des zweiten Aspektes ist vorgesehen, dass der Adenovirus getrennt von den mindestens zwei Mitteln ist.

In einer Ausführungsform des ersten und des zweiten Aspektes ist vorgesehen, dass mindestens eine Einheitsdose des Virus von mindestens einer Einheitsdose von einem oder den mindestens zwei Mitteln getrennt ist.

Erfindungsgemäß wird die Aufgabe in einem vierten Aspekt gelöst durch die Kit umfassend einen Adenovirus gemäß dem ersten Aspekt der Erfindung und mindestens zwei Mittel, wobei ein jedes Mittel einzeln und unabhängig aus der Gruppe ausgewählt ist, die aus Cytostatika besteht, und wobei mindestens ein Mittel den Transport von YB-1 in den Zellkern vermittelt.

Der vorliegenden Erfindung liegt die überraschende Erkenntnis zugrunde, dass die DNA-Replikation von E1A-modifizierten Adenoviren in YB-1 Kern-positiven Tumorzellen auf die Aktivierung des E2-late Promotors zurückzuführen ist. Unter E1A-modifizierten Adenoviren sind dabei solche Adenoviren zu verstehen, die (a) keine Replikation oder eine verglichen mit dem jeweiligen Wildtyp verringerte, bevorzugterweise stark verringerte Replikation, in YB-1-Kern-negativen Zellen zeigen, (b) transaktivierend auf mindestens ein virales Gen wirken, wobei das Gen insbesondere ausgewählt ist aus der Gruppe, die E1B-55kDa, E4orf6, E4orf3 und E3ADP umfasst, und/oder (c) zelluläres YB-1 durch den Adenovirus nicht in den Kern transloziert. Optional weisen die verwendeten Adenoviren die weitere Eigenschaft auf, dass nämlich die Bindung des von dem Adenovirus kodierten E1A-Proteins die Bindung von E2F an RB stört bzw. den entsprechenden Komplex aus E2F und Rb aufzulösen in der Lage ist. Adenoviren, die eines oder mehrere der vorstehend genannten Merkmale a) bis c), bevorzugterweise alle Merkmale a) bis c) aufweisen, sind replikationsdefizient in Zellen, die YB-1 nicht im Kern aufweisen.

Unter einer stark verringerten Replikation hierin insbesondere eine solche Replikation verstanden, die gegenüber dem Wildtyp um den Faktor 2, bevorzugterweise um den Faktor 5, bevorzugtererweise um den Faktor 10 und am bevorzugtesten um den Faktor 100 verringert ist. Es ist vorgesehen, dass der Vergleich der Replikation bei Verwendung gleicher oder ähnlicher Zelllinien, gleicher oder ähnlicher Virustiter für die Infektion (engl. multiplicity of infection, MOI, oder engl. plaque forming unit, pfu) und/oder gleicher oder ähnlicher allgemeiner Versuchsbedingungen durchgeführt wird. Dabei wird unter Replikation insbesondere die Partikelbildung verstanden. Desweiteren kann jedoch als Mass für die Replikation der Umfang der Synthese viraler Nukleinsäure verstanden sein. Verfahren zur Bestimmung des Umfanges der Synthese viraler Nukleinsäuren sind ebenso wie Verfahren zur Bestimmung der Partikelbildung den Fachleuten auf dem Gebiet bekannt.

Die hierin beschriebenen Erkenntnisse bzw. die Verfahren, Verwendungen oder Nukleinsäuren, Proteine, Replikationssysteme und dergleichen sind nicht notwendigerweise auf Adenoviren beschränkt. Grundsätzlich gibt es auch bei anderen Viren derartige Systeme, die hiermit ebenfalls umfasst sind.

Bei Verwendung der Viren oder bei der Verwendung der hierin beschriebenen Viren kann eine dem Wildtyp vergleichbare Replikation bereits bei einer Infektionsrate von 1 bis 10 pfu/Zelle erreicht werden gegenüber 10 bis 100 pfu/Zelle gemäß dem Stand der Technik.

Unter zellulärem YB-1 soll hierin ein jedes YB-1 verstanden werden, welches von einer Zelle codiert und bevorzugterweise auch exprimiert wird, wobei dieses YB-1 in der Zelle insbesondere vor der Infektion der betreffenden Zelle mit einem Adenovirus, bevorzugterweise einem Adenovirus, und/oder einem Helfervirus, wie hierin beschrieben, vorhanden ist. Es ist jedoch auch im Rahmen der vorliegenden Erfindung, dass zelluläres YB-1 auch ein solches ist, welches erst durch exogene Maßnahmen, wie beispielsweise durch Infektion mit einem Virus, insbesondere mit einem Adenovirus, in die Zelle eingeführt bzw. von dieser produziert wird.

Ohne im folgenden darauf festgelegt sein zu wollen, geht der vorliegende Erfinder davon aus, dass der E2-early Promotor, d. h. der frühe E2-Promotor, im Rahmen der Replikation der hierin verwendeten Viren nicht über den humanen zellulären E2F-Transkriptionsfaktor eingeschaltet wird. Das Einschalten der Replikation ist dabei unabhängig vom Rb-Status der Zellen, d.h. dass die Tumorzellen, die unter Anwendung der hierin offenbarten Viren infiziert und in der Folge bevorzugt lysiert werden, sowohl funktionelle wie auch inaktive Rb-Proteine besitzen können. Zudem benötigt die adenovirale Replikation unter Verwendung der hierin offenbarten Adenoviren bzw. den hierin offenbarten Bedingungen kein funktionelles p53 Protein, wird jedoch auch durch dessen Vorhandensein nicht nachteilig beeinflusst. Insoweit wendet sich die technische Lehre von dem mit der Anwendung der onkolytischen oder tumorlytischen Adenoviren vom Typ AdΔ24, d1922-947, E1Ad/01/07, CB016 oder jener Adenoviren, wie sie beispielsweise im europäischen Patent EP 0 931 830 beschrieben sind, verfolgten Prinzip ab, bei denen eine und/oder mehrere Deletion(en) im E1A-Protein vorgenommen worden war(en) unter der Annahme, dass intakte funktionelle Rb-Proteine einer effizienten Replikation in vivo entgegenwirken und somit eine adenovirale Replikation in vivo nur in Rb-negativen bzw. Rb-mutierten Zellen sicher zu stellen. Diese adenoviralen Systeme nach dem Stand der Technik gehen auf E1A zurück, um mittels des frühen E2-Promotors (E2-early Promotor) und "freiem E2F" die in vivo Replikation von Adenoviren zu steuern. Gleichwohl können diese im Stand der Technik bekannten Viren erfindungsgemäß verwendet werden, d. h. zur Replikation in Zellen, die YB-1 unabhängig vom Zellzyklus im Kern enthalten.

Die in dem besagten europäischen Patent EP 0 931 830 beschriebenen Viren und insbesondere Adenoviren können dabei erfindungsgemäß verwendet werden. Konkret handelt es sich bei den in besagtem Patent beschriebenen Viren um solche, die eine Replikaktionsdefizienz aufweisen und denen ein exprimiertes virales Onkoprotein fehlt, das zur Bindung eines funktionellen Rb-Tumorsuppressor-Genprodukts fähig ist. Der Adenovirus kann dabei insbesondere ein solcher sein, dem exprimiertes virales E1A-Onkoprotein fehlt, das zur Bindung eines funktionellen Tumorsuppressor-Genprodukt, insbesondere Rb, fähig ist. Das virale E1A-Onkoprotein kann dabei eine inaktivierende Mutation aufweisen, beispielsweise in der CR1-Domäne an den Aminosäuren 30 bis 85 in Ad 5, den Nukleotidpositionen 697-790, und/oder der CR2-Domäne an den Aminosäuren 120 bis 139 in Ad 5, den Nukleotidpositionen 920 bis 967, welche an der Bindung von p105 Rb Protein, p130 und p107 Protein beteiligt sind. Dabei kann auch vorgesehen sein, dass der Adenovirus vom Typ 2 dl 312 oder Adenovirus vom Typ 5 NT dl 1010 ist

Bei der erfindungsgemäßen Verwendung von Adenoviren zur Herstellung eines Medikamentes, insbesondere zur Herstellung eines Medikamentes für die Behandlung von Tumorerkrankungen, und bei der Verwendung von Adenoviren zur Replikation in Zellen, die YB-1 im Kern aufweisen, erfolgt die Replikation letzten Endes in solchen Zellen, die YB-1 im Kern, bevorzugterweise unabhängig vom Zellzyklus, aufweisen, mithin YB-1-Kern-positiv sind. Dabei ist besonders beachtlich, dass die Adenoviren als solche in Zellen, die YB-1 nicht im Kern, sondern im Wesentlichen nur im Zytoplasma enthalten, nicht oder stark verringert replizieren. Insoweit ist es erforderlich, dass für eine erfolgreiche Replikation dieser Viren YB-1 im Kern vorhanden ist Dies kann beispielsweise, wie auch im Folgenden noch ausgeführt werden wird, durch Anlegen solcher Bedingungen an die Zellen realisiert werden, dass es zur Expression oder dem Vorhandensein von YB-1 im Kern kommt. Eine entsprechende Maßnahmen kann z. B. die Codierung bzw. Exprimierung von YB-1 durch die erfindungsgemäß verwendeten Adenoviren sein, die in Ergänzung zu den adenoviralen Genen auch eine genetische Information in sich tragen, die für YB-1 und insbesondere dessen Expression codiert. Andere Maßnahmen, die zum Transport, zur Induktion oder Expression von YB-1 im Kern der Zelle führen, sind das Anlegen von Stressbedingungen wie beispielsweise die Applikation von Zytostatika, Bestrahlung, Hyperthermie und dergleichen an die Zelle bzw. den eine - solche - Zelle enthaltenden Organismus.

Die im Rahmen der vorliegenden Erfindung, insbesondere zur Tumorlyse, verwendeten Adenoviren zeichnen sich weiterhin dadurch aus, dass sie in solchen Zellen, die YB-1 im Kern nicht aufweisen, mithin YB-1-Kern-negativ sind, nicht replizieren.

Ein weiteres Merkmal der erfindungsgemäß zu verwendenden Adenoviren besteht darin, dass sie für ein virales Onkoprotein, das hierin auch also Onkogenprotein bezeichnet wird, codieren, wobei es sich bei dem Onkogenprotein um E1A handelt, wobei das Onkogenprotein in der Lage ist, zumindest ein adeno virales Gen zu aktivieren, welches einen Einfluss auf die Replikation des Virus und/oder die Zellyse der von dem Virus infizierten Zelle haben kann. Dabei ist bevorzugt, dass der Einfluss auf die Replikation dergestalt ist, das der Virus bei Vorhandensein des Onkogenproteins besser repliziert als bei Fehlen des Onkogenproteins des jeweilige Virus. Dieser Vorgang wird hierin auch als transaktivierend bezeichnet bzw. als E1A-transaktivierend bezeichnet, da die Transaktivierung von E1A vermittelt wird. Die Bezeichnung "transaktivieren" oder "Transaktivierung" beschreibt dabei bevorzugt den Vorgang, auf die Expression und/oder auf die Transkription eines anderen oder mehrerer adenovirales Gene ausgewählt aus der Gruppe aus E1B55kDa, E4orf6, E4orf3 und E3ADP.

Ein weiteres, wenngleich bevorzugterweise optionales, Merkmal der erfindungsgemäß zu verwendenden Adenoviren ist deren Bindungsverhalten zu bzw. mit dem Tumor-Suppressor Rb. Es ist grundsätzlich im Rahmen der vorliegenden Erfindung, dass die erfindungsgemäß verwendeten Adenoviren an Rb binden oder nicht binden können. Die Verwendung der beiden alternativen Ausgestaltungsformen der Adenoviren ist dabei unabhängig vom Rb-Status der behandelnden Zelle möglich.

Um E1A die Fähigkeit zu verleihen, nicht an Rb binden zu können, sind beispielsweise folgende Deletionen am E1A-Onkoprotein möglich: Deletion in der CR1-Region (Aminosäurepositionen 30-85 in Ad5) und Deletion der CR2-Region (Aminosäurepositionen 120-139 in Ad5). Dabei bleibt die CR3-Region erhalten und kann ihre transaktivierende Funktion auf die anderen frühen viralen Gene ausüben.

Um E1A die Fähigkeit zu verleihen, an Rb binden zu können, sind dagegen folgende Deletionen am E1A-Onkoprotein grundsätzlich möglich: Deletion der CR3-Region (Aminosäurepositionen 140-185); Deletion des N-Terminus (Aminosäurepositionen 1-29); Deletion der Aminosäurepositionen 85-119; und Deletion des C-Terminus (Aminosäurepositionen 186-289).

Die hier aufgeführten Bereiche interferieren nicht mit der Bindung von E2F an Rb. Die transaktivierende Funktion bleibt erhalten, ist jedoch gegenüber vom Wildtyp-Ad5 verringert.

Derartige im Stand der Technik grundsätzlich bereits bekannte Viren gelten allgemein als replikationsdefizient. Es ist jedoch das Verdienst des vorliegenden Erfindes, erkannt zu haben, dass sie dennoch zur Replikation in einem geeigneten Hintergrund, insbesondere einem zellulären Hintergrund geeignet sind. Ein derartiger geeigneter zellulärer Hintergrund wird durch das Vorhandensein von YB-1 im Kern, bevorzugterweise eine Zellzyklus-unabhängige Anwesenheit von YB-1 im Kern, bedingt oder bereitgestellt. Der Begriff der Zellen oder zellulären Systeme, wie hierin verwendet, umfasst dabei Fragmente oder Fraktionen von Zellaufschlüssen ebenso wie Zellen, die in vitro, in vivo oder in situ vorliegen. Insoweit umfasst der Begriff zelluläre Systeme oder Zellen auch solche Zellen, die in einer Zellkultur, Gewebekultur, Organkultur oder in einem Gewebe oder Organ in vivo bzw. in situ, isoliert, in Gruppen oder als Teil von Geweben, Organen oder Organismen oder aber auch als solches in einem bevorzugterweise lebenden Organismus vorliegen. Bei dem Organismus handelt es sich bevorzugter Weise um einen Wirbeltier-Organismus und bevorzugterer Weise um ein Säugetier. Besonders bevorzugterweise ist der Organismus dabei ein menschlicher Organismus.

Darüber hinaus ist es im Rahmen der vorliegenden Erfindung, dass auf der Grundlage der hierin gegebenen technischen Lehre neue Viren erzeugt werden, die das Replikationsverhalten der hierin beschriebenen und im Stand der Technik bekannten Adenoviren in solchen Zellen zeigen, die YB-1-Kern-positiv sind. Mit anderen Worten, insbesondere bevorzugterweise ausgehend von den bereits bekannten Adenoviren können weitere Viren konzipiert werden, die die hierin definierten, für die erfindungsgemäße Verwendung erforderlichen Merkmale aufweisen.

Im Zusammenhang mit der vorliegenden Erfindung ist das modifizierte E1A-Onkoprotein der verschiedenen, erfindungsgemäß zu verwendenden Adenoviren in der Lage transaktivierend auf die adeno viralen Gene, E1B55K, E4orf3, E4orf6, E3ADP, in YB-1 kernpositiven Zellen zu wirken. Dabei liegt sonst bevorzugterweise keine Veränderung im viralen Genom vor und der entsprechende Adenovirus kann insoweit ansonsten einen Adenoviren vom Wildtyp oder Abwandlung davon entsprechen.

Zu den hierin offenbarten Viren, die für ein transaktivierendes Onkogenprotein im Sinne der vorliegenden Erfindung codieren oder ein solches umfassen, gehören die Adenoviren AdΔ24, d1922-947, E1Ad/01/07, CB106 und/oder die im europäischen Patent EP 0931 830 beschriebenen Adenoviren, die jeweils in der Lage sind, transaktivierend auf die frühen Gene z.B. E1B, E2, E3 und/oder E4 zu wirken, und ist vergleichbar mit Adenovirus vom Wildtyp, insbesondere vom Wildtyp Ad5 aufweisen. Verantwortlich für die Transaktivierung ist in diesen Fällen ein bestimmter Bereich des E1A Proteins. Innerhalb von verschiedenen Adenovirus-Serotypen kommen 3 hoch konservierte Bereiche im E1A Protein vor. Der Bereich CR1 von 41-80 AS, CR2 von 120-139 und CR3 von 140-188 AS. Die transaktivierende Funktion beruht hauptsächlich auf das Vorhandensein der CR3-Region im E1A Protein. Die AS-Sequenz von CR3 liegt in den oben genannten Adenoviren unverändert vor. Dies führt dazu, dass die Transaktivierung der frühen Gene E1B, E2, E3 und E4 unabhängig davon erfolgt, ob YB-1 im Kern oder im Zytoplasma vorhanden ist.

Im rekombinanten Adenovirus d1520 ist dahingegen der CR3-Bereich deletiert worden. Damit exprimiert d1520 ein sogenanntes E1A12S Protein, welches nicht die AS-Sequenz der CR3-Region aufweist. Dies führt dazu, dass d1520 nur eine ganz schwache transaktivierende Funktion ausüben kann, insbesondere auf die E2-Region, und somit in YB-1 Kern-negativen Zellen nicht repliziert. In YB-1 Kern-positiven Zellen übernimmt YB-1 die Transaktivierung der E2-Region, und ermöglicht somit eine effziente Replikation d1520. Darauf beruht die Verwendung von Systemen wie d1520 bzw. auf der Grundlage von d1520 zu den hierin offenbarten Zwecken. Ein weiterer wichtiger Unterschied zwischen beiden vorstehend beschriebenen Gruppen von Adenoviren, d. h. delta 24 (hierin auch als AdΔ24 bezeichnet) und d1520 besteht darin, dass bei d1520 die frühen Gene E1B, E3 und E4 stärker in YB-1 Kern-positiven Zellen als in YB-1 Kernnegativen Zellen transaktiviert werden. Bei delta 24 bestehen hingegen keine bzw. nur geringfügige Unterschiede. Die Transalctivierung von d1520 bzw. genauer des E1A12S Proteins ist allerdings erheblich schwächer im Vergleich zum Wildtyp-Adenovirus. Diese Transaktivierung reicht allerdings aus, um eine effiziente Replikation in YB-1 kernpositiven Zellen durchzuführen, wie dies auch in Beispiel 10 gezeigt ist. Die hierin und speziell in diesem Zusammenhang beschriebene Ausgestaltung des E1A-Proteins bzw. der dafür codierenden Nukleinsäure in der Form, dass das E1A-Protein gegenüber dem Wildtyp-Onkogenprotein E1A eine oder mehrere Deletionen und/oder Mutationen aufweist, wobei die Deletion bevorzugt eine solche ist, die ausgewählt ist aus der Gruppe, die Deletionen des Bereiches CR3 und Deletionen des N-Terminus und Deletionen des C-Terminus umfasst, einschließlich und besonders bevorzugt jener Ausgestaltungen des E1A-Proteins wie im Zusammenhang mit d1520 oder AdΔ24, d1922 bis 947, E1Ad/01/07, CB106 und/oder die im europäischen Patent EP 0 931 830 beschriebenen Adenoviren, stellen Ausführungsformen von Viren, insbesondere von Adenoviren dar, deren Replikation durch YB-1 über die Aktivierung des E2-late-Promotors gesteuert wird, bevorzugterweise überwiegend über die Aktivierung des E2-late-Promotors. Weitere Ausgestaltungen des E1A-Proteins, die diese Form der Replikation von Adenoviren erlauben, können auf der Grundlage der hierin gemachten Offenbarung von den Fachleuten auf dem Gebiet hergestellt werden.

Bei weiteren, neu zu konstruierenden rekombinanten Adenoviren, die hierin auch als Abwandlungen bezeichnet werden und die verwendet werden können, liegt typischerweise eine E1-Deletion, eine E1/E3-Deletion und/oder eine E4-Deletion vor, d. h. die ensprechenden Adenoviren sind nicht in der Lage, funktional aktive E1- und/oder E3- und/oder E4-Expressionsprodukte bzw. entsprechende Produkte zu erzeugen, oder mit anderen Worten, diese Adenoviren lediglich in der Lage sind, funktional inaktive E1-, E3- und/oder E4-Expressionsprodukt zu erzeugen, wobei ein funktional inaktives E1-, E3- und/oder E4-Expressionsprodukt ein solches, welches entweder gar nicht als Expressionsprodukt, sei es auf der Ebene der Transkription und/oder der Translation, oder in einer Form vorliegt, bei der es zumindest eine der ihm im Adenovirus vom Wildtyp zukommende Funktion nicht aufweist. Diese dem Expressionsprodukt im Adenovirus vom Wildtyp zukommenden Funktion(en) ist/sind den Fachleuten auf dem Gebiet bekannt und beispielsweise beschrieben in Russell, W. C., Journal of Virology, 81, 2573-2604, 2000. Russell (a.a.O.) beschreibt im übrigen auch Prinzipien der Konstruktion von Adenoviren und adenoviralen Vektoren, die hierin durch Bezugnahme aufgenommen werden. Es ist auch im Rahmen der vorliegenden Erfindung, dass das modifizierte E1A-Onkoprotein, E1B-55K, E4orf6 und/oder E3ADP (adenoviral death protein (ADP)) (Tollefson, A. et al., J. Virology, 70, 2296-2306, 1996.) in einem solchen Vektor einzeln oder in beliebiger Kombination zur Expression gebracht wird/werden. Dabei können die einzelnen genannten Gene ebenso wie die hierin offenbarten Transgene und/oder therapeutischen Transgene unabhängig von einander entweder in der E1 und /oder E3 und/oder E4-Region einkloniert und mit Hilfe eines geeigneten Promoters oder unter Kontrolle eines geeigneten Promotors zur Expression gebracht werden. Grundsätzlich sind die Regionen E1, E3 und E4 als Klonierungsstellen innerhalb der adenoviralen Nukleinsäure gleichermaßen geeignet, wobei die für die Klonierung nicht benutzten Regionen, einzeln oder insgesamt, entweder vorhanden, teilweise und/oder ganz deletiert sein können. Für den Fall, dass diese Regionen vorhanden sind, insbesondere vollständig vorhanden sind, ist es im Rahmen der vorliegneden Erfindung, dass diese entweder intakt ist und bevorzugterweise ein Translationsprodukt und/oder ein Transkriptionsprodukt liefert, und/oder nicht intakt ist und bevorzugterweise kein Translationsprodukt und/oder Transkriptionsprodukt liefert. Geeignete Promotoren sind u. a. jene, wie sie hierin im Zusammenhang mit der Steuerung bzw. Expression von E1A, insbesondere des modifizierten E1A, offenbart sind.

Schließlich ist in einer Ausführungsform vorgesehen, dass die erfindungsgemäß verwendeten Adenoviren hinsichtlich E1B 19 kda-defizient sind. Dabei wird hierin allgemein unter dem Begriff defizient ein Zustand verstanden, bei dem E1B die im Wildtyp inhärente Gesamtheit der Eigenschaften nicht aufweist und mindestens eine dieser Eigenschaften fehlen. Das Adenovirus BCL2-Homolog E1B19k verhindert die E1A induzierte Apoptose durch Interaktion mit den pro-apoptotischen Proteinen Bak und Bax. Dadurch wird eine maximale Replikation und/oder Partikelbildung in infizierten Zellen ermöglicht (Ramya Sundararajan und Eileen White, Journal of Virology 2001, 75, 7506-7516). Das Fehlen von E1B 19k führt zur besseren Freisetzung der Viren, da es falls vorhanden die Funktion des adenoviralen Death-Proteins minimiert. Durch eine solche Deletion wird der Virus induzierte cytopathische Effekt erhöht (Ta-Chiang Liu et al., Molecular Therapy, 2004) und führt somit zur stärkeren Lyse von infizierten Tumorzellen. Zudem bewirkt das Fehlen von E1B19k, dass TNF-alpha auf die Replikation von solchen rekombinanten Adenoviren in Tumorzellen keinen Einfluß ausübt, währen in normalen Zellen die Behandlung zu einer geringeren Replikation und Freisetzung von infektiösen Viren führt. Somit wird die Selektivität und Spezifität erhöht (Ta-Chiang Liu et al., Molecular Therapy 2004, 9, 786-803).

Die Adenoviren, wie sie gemäß der hierin offenbarten Erfindung verwendet werden, sind grundsätzlich im Stand der Technik zumindest in einigen Ausführungsformen bekannt. Bei den erfindungsgemäß verwendeten Adenoviren handelt es sich bevorzugter Weise um rekombinante Adenoviren, insbesondere auch dann, wenn eine Veränderung gegenüber dem Wildtyp vorgenommen wurde im Sinne der hierin gegebenen technischen Lehre. Es ist im Rahmen der Kenntnisse der Fachleute auf diesem Gebiet, für die Erfindung unwesentliche adenoviralen Nukleinsäuresequenzen zu deletieren bzw. zu mutieren. Derartige Deletionen können z. B die für einen Teil der E3 und E4 codierende Nukleinsäure betreffen wie hierin auch beschrieben. Bei einer Deletion von E4 ist dabei besonders bevorzugt, wenn sich diese nicht auf das Protein E4orf6 erstreckt, mithin der erfindungsgemäß zu verwendende Adenovirus E4orf6 codiert. In bevorzugten Ausführungsformen können diese adenoviralen Nukleinsäuren noch in das virale Kapsid verpackt werden und damit infektiöse Partikel ausbilden. Gleiches gilt für die erfindungsgemäße Verwendung der Nukleinsäuren. Generell gilt es auch noch festzuhalten, dass die adenoviralen Systeme hinsichtlich einzelner oder mehrerer Expressionsprodukte defizient sein können. Dabei ist zu berücksichtigen, dass dies zum einen darauf beruhen kann, dass die für das Expressionsprodukt codierende Nukleinsäure vollständig oder in dem Maße mutiert oder deletiert ist, dass im wesentlichen kein Expressionsprodukt mehr gebildet wird, oder darauf, dass regulative bzw. die Expression steuernde Elemente wie Promotoren oder Transkriptionsfaktoren fehlen oder anders als im Wildtyp aktiv sind, sei es auf der Ebene der Nukleinsäure (Fehlen eines Promotors; cis-wirkende Element) oder auf der Ebene des Translations- bzw. Transkriptionssystems (trans-wirkende Elemente). Gerade der letztere Aspekt kann dabei vom jeweiligen zellulären Hintergrund abhängen.

Neben der Verwendung von an und für sich bereits bekannten Adenoviren gemäß der vorliegenden Erfindung können auch neue Adenoviren in dem Umfang verwendet werden, wie dies für die anderen hierin beschriebenen Adenoviren bereits offenbart ist. Die erfindungsgemäßen neuen Adenoviren ergeben sich aus der hierin offenbarten technischen Lehre. Besonders bevorzugte Vertreter sind dabei beispielsweise die hierin in Fig. 16 und Fig. 17 dargestellten Viren Xvir03 und Xvir03/01, deren Konstruktionsprinzip auch in den Beispielen 11 und 12 weiter veranschaulicht ist.

Im Falle des Vektors Xvir03 wurde in der E1-Region ein CMV-Promotor kloniert, der die durch eine IRES-Sequenz getrennten Nukleinsäuren für E4orf6 und E1B 55k codiert. Dabei kann die E3-Region teilweise oder vollständig deletiert und/oder intakt vorhanden sein. Infolge der Einklonierung dieser beiden Gene bzw. der daraus hergestellten Genprodukte kommt es zu einer hohen Replikationseffizienz, die bevorzugterweise nahezu derjenigen von Wildtyp-Viren entspricht, wobei die Selektivität der Replikation in Zellen, insbesondere Tumorzellen, insoweit beibehalten wird, als dass eine Replikation, insbesondere in YB-1-Kern-positiven Zellen und insbesondere in solchen Zellen erfolgt, bei denen YB-1 dereguliert vorliegt. Zellen, in denen YB-1 dereguliert vorliegt, sind dabei bevorzugt solche, die eine erhöhte Expression von YB-1, bevorzugt Kompartiment-unabhängig, aufweisen verglichen mit normalen oder Nicht-Tumorzellen. Das Einklonieren von E1B 55k und E4orf6 kann aber auch in der E4-Region erfolgen, wobei die E3-Region intakt oder/und teilweise oder vollständig deletiert sein kann.

Eine Weiterentwicklung des Virus Xvir03 stellt der Virus Xvir03/01 dar, bei dem unter der Kontrolle eines spezifischen Promotors, insbesondere eines Tumor- oder Gewebe-spezifischen Promotors, therapeutische Gene oder Transgene in einer bevorzugten Ausführungsform einkloniert sind. Weiterhin ist es im Rahmen eines derartigen Virus, dass auch die Region E4 funktional inaktiv ist, bevorzugterweise deletiert ist. Die hierin beschriebenen Transgene können dabei auch in die E4-Region kloniert werden, wobei dies alternativ oder ergänzend zum Klonieren der Transgene in die E3-Region erfolgen kann bzw. die E3-Region teilweise oder vollständig intakt bleibt. Transgene, wie hierein verwendet, können virale Gene, bevorzugterweise adenovirale Gene sein, die bevorzugterweise im Wildtyp-Adenovirus nicht im Genom bzw. an der Stelle im Genom vorkommen, an der sie in dem konkreten Virus nun vorliegen, oder therapeutische Gene.

Therapeutische Gene können dabei Prodrug-Gene, Gene für Zytokine, Apoptose-induzierende Gene, Tumorsuppressorgene, Gene für Metalloproteinasen-Inhibitoren und/oder Angiogenese-Inhibitoren sein. Ferner können siRNA, Aptameren, Antisense und Ribozyme exprimiert werden die gegen Krebs-relevante Zielmoleküle gerichtet sind. Bevorzugterweise ist das einzelne oder die mehreren Zielmoleküle aus der Gruppe ausgewählt, die Resistenz-relevante Faktoren, Anti-Apoptose-Falctoren, Onkogene, Angiogenese-Faktoren, DNA-Synthese-Enzyme, DNA-Reperaturenzyme, Wachstumsfaktoren und deren Rezeptoren, Transkriptionsfaktoren, Metalloproteinasen, insbesondere Matrix-Metalloproteinasen, und Plasminogenaktivator vom Urokinase-Typ umfasst. Bevorzugte Ausführungsformen davon sind hierin bereits offenbart.

Mögliche Prodrug-Gene, wie sie in bevorzugten Ausführungsformen verwendet werden können, sind beispielsweise *Cytosine deaminase, Thymidin kinase, Carboxypeptidase, Uracil phosphoribosyltransferase; Purin Nukleosid Phosphorylase (PNP);* Kirn et al, Trends in Molecular Medicine, Volume 8, No.4 (Suppl), 2002; Wybranietz W.A. et al., Gene Therapy, 8, 1654-1664, 2001; Niculescu-Duvaz et al., Curr. Opin. Mol. Therapy, 1, 480.486, 1999; Koyama et al., Cancer Gene Therapy, 7, 1015-1022, 2000; Rogers et al., Human Gene Therapy, 7, 2235-2245, 1996; Lockett et al., Clinical Cancer Res., 3, 2075-2080, 1997; Vijayakrishna et al., J. Pharmacol. And Exp. Therapeutics, 304, 1280-1284, 2003.

Mögliche Zytokine, wie sie in bevorzugten Ausführungsformen verwendet werden können, sind beispielsweise *GM-CSF, TNF-alpha, Il-12, Il-2, Il-6, CSF, Interferon-Gamma;* Gene Therapy, Advances in Pharmacology, Volume 40, Editor: J. Thomas August, Academic Press; Zhang und Degroot, Endocrinology, 144, 1393-1398, 2003; Descamps et al., J. Mol. Med., 74, 183-189, 1996; Majumdar et al., Cancer Gene Therapy, 7, 1086-1099, 2000.

Mögliche Apoptose-induzierende Gene, wie sie in bevorzugten Ausführungsformen verwendet werden können, sind beispielsweise Decorin: Tralhao et al., FASEB J, 17, 464-466, 2003; Retinoblastoma 94: Zhang et al., Cancer Res.,63, 760-765, 2003; Bax und Bad: Zhang et al, Hum. Gene Ther., 20, 2051-2064, 2002; Apoptin: Noteborn und Pietersen, Adv. Exp. Med. Biol., 465, 153-161, 2000); ADP: Toth et al., Cancer Gene Therapy, 10, 193-200, 2003; bcl-xs: Sumantran et al., Cancer Res, 55, 2507-2512, 1995; E4orf4: Braithwaite und Russell, Apoptosis, 6, 359-370, 2001; FasL, Apo-1 und Trail: Boehringer Manheim, Guide to Apoptotic Pathways, Arai et al., PNAC, 94, 13862-13867, 1997; Bims; Yamaguchi et al., Gene Therapy, 10, 375-385, 2003; GNR163: Oncology News, 17 Juni, 2000, sind.

Mögliche Tumorsuppressor-Gene, wie sie in bevorzugten Ausführungsformen verwendet werden können, sind beispielsweise *E1A, p53, p16, p21, p27, MDA-7.* Opalka et al., Cell Tissues Organs, 172, 126-132, 2002 , Ji et al., Cancer Res., 59, 3333-3339, 1999, Su et al., Oncogene, 22,1164-1180,2003.

Mögliche Angiogenese-Inhibitoren, wie sie in bevorzugten Ausführungsformen verwendet werden können, sind beispielsweise *Endostatin, angiostatin :* Hajitou et al., FASEB J., 16, 1802-1804, 2002, und Antikörper gegen VEGF (Ferrara, N., Semin Oncol 2002 Dec; 29 (6 Suppl 16): 10-4.

Mögliche Metalloproteinase-Inhibitoren, wie sie in bevorzugten Ausführungsformen verwendet werden können, sind beispielsweise *Timp-3,* Ahonen et al., Mol Therapy, 5, 705-715,2002; *PAI*-1; Soff et al., J. Clin. Invest., 96, 2593-2600, 1995 ; *Timp-1,* Brandt K. Curr. Gene Therapy, 2, 255-271, 2002.

siRNA (short interfering RNA), wie sie im Rahmen der vorliegenden Erfindunge verwendet werden kann, besteht aus zwei, bevorzugt zwei getrennten RNA-Strängen, die infolge Basenkomplementarität miteinander hybridisieren, d.h. im wesentlichen basengepaart vorliegen, und weist bevozugterweise eine Länge von bis zu 50 Nukleotiden auf, bevorzugterweise zwischen 18 und 30 Nukleotiden, bevorzugtererweise weiniger als 25 Nukleotide und am bevorzugtesten 21, 22 oder 23 Nukleotide, wobei sich diese Zahlenwerte auf einen Einzelstrang der siRNA, insbesondere auf die Länge des Bereiches eines Einzelstranges, der mit einem, genauer dem zweiten Einzelstrang hybridisiert bzw. basenpaart, beziehen. siRNA induziert oder vermittelt spezifisch den Abbau von mRNA. Die dazu erforderliche Spezifität wird durch die Sequenz der siRNA und damit ihren Bindungsort vermittelt. Die abzubauende Zielsequenz ist dabei im wesentlichen komplementär zu dem ersten oder zu dem zweiten die siRNA aufbauenden Strang. Obwohl die genauen Wirkmechanismen noch unklar sind, wird vermutet, dass siRNA für Zellen eine biologische Strategie darstellt, während der Entwicklung bestimmte Allele zu hemmen und sich vor Viren zu schützen. Die durch siRNA vermittelte RNA-Interferenz wird als Verfahren zur spezifischen Unterdrückung oder gar völligen Ausschaltung der Expression eines Proteins durch Einbringen einer genspezifischen doppelsträngigen RNA benutzt. Für höhere Organismen ist eine 19 bis 23 Nukleotid lange siRNA deshalb besonders geeignet, weil sie nicht zur Aktivierung der unspezifischen Abwehrreaktion, zur sogenannten Interleukin-Antwort führt. Die direkte Transfektion von doppelsträngiger RNA aus 21 Nukleotiden mit symmetrischen 2-nt 3' Überhängen konnte eine RNA-Interferenz in Säugetierzellen vermitteln und zeigte im Vergleich zu anderen Technologien wie Ribozymen und Antisense-Molekülen eine hohe Effizienz (Elbashir, S. Harborth J. Lendeckel W. Yalvcin, A. Weber K Tuschl T: Duplexes of 21-nucleotide RNAs mediate RNA interference in cultured mammalian cells. Nature 2001, 411: 494-498). Nur wenige siRNA-Moleküle genügten, um die Expression des Zielgens zu unterdrücken. Um die Limitierungen exogen zugeführter siRNA, die insbesondere in der transienten Natur des Interferenz-Phänomens und der spezifischen Abgabe (engl. delivery) der siRNA-Moleküle liegen, zu umgehen, werden im Stand der Technik auch Vektoren eingesetzt, die eine endogene siRNA-Expression erlauben. Hierfür werden beispielsweise Oligonukleotide mit einer Länge von 64 Nukleotiden, die die 19 Nukleotide lange Zielsequenz einschließen, sowohl in sense- als auch in antisense-Orientierung, getrennt durch eine beispielsweise 9 Nukleotide lange Spacer-Sequenz, in den Vektor eingebaut. Das resultierende Transkript faltet sich zu einer Haarnadelstruktur mit einer Stammstruktur (engl.: stem) von beispielsweise 19 Basenpaaren. In der Zelle wird die Schleife rasch abgespaltet, so daß eine funktionelle siRNA entsteht (Brummelkamp et al., Science, 296, 550-553, 2002)

Die Aktivität von pRb bzw. E2F wird durch Phosphorylierung reguliert. Die hypophosphorylierte Form von pRb tritt hauptsächlich in der G1- und M-Phase auf. Dagegen tritt die hyperphosphorylierte Form von pRb in der S- und G2-Phase auf. Durch die Phosphorylierung von pRb wird E2F aus dem Komplex aus E2F und hypophosphoryliertem pRb freigesetzt. Die Freisetzung von E2F aus dem Komplex aus E2F und hypophosphoryliertem pRb führt zur Transkription von E2F-abhängigen Genen. Das E1A-Protein bindet nur an die hypophosphorylierte Form des pRb, wobei die Bindung von E1A an pRb hauptsächlich über die CR2-Region des E1A-Proteins erfolgt. Zudem bindet es auch an die CR1-Region, allerdings mit einer schwächeren Affinität (Ben-Israel and Kleiberger, Frontiers in Bioscience, 7, 1369-1395, 2002; Helt und Galloway, Carcinogenesis, 24, 159-169, 2003).

Die für YB-1 codierende Nukleinsäure, die in einer Ausführungsform der erfindungsgemäß zu verwendenden Adenoviren Bestandteil der Adenoviren sein kann, kann dabei eine einen Kerntransport von YB-1 vermittelnde Nukleinsäuresequenz umfassen. Als Adenoviren bzw. adenovirale Systeme und damit die entsprechenden Nukleinsäuren können im Zusammenhang damit und in Kombination mit diesen erfindungsgemäßen Nukleinsäuren die erfindungsgemäßen Nukleinsäuren, Adenoviren und adenoviralen Systeme sowie die im Stand der Technik bekannten Adenoviren wie beispielsweise Onyx-015, AdΔ24, d1922-947, E1Ad/01/07, CB016, dl 520 und die im Patent EP 0931 830 beschriebenen Adenoviren verwendet werden. Geeignete, den Kerntransport vermittelnde Nukleinsäuresequenzen sind den Fachleuten auf dem Gebiet bekannt und beispielsweise beschrieben in (Whittaker, G.R. et al., Virology, 246, 1-23, 1998; Friedberg, E.C., TIBS 17, 347, 1992; Jans, D.A. et al., Bioessays 2000 Jun; 22(6): 532-44; Yoneda, Y., J. Biocehm. (Tokyo) 1997 May; 121(5): 811-7; Boulikas, T., Crit. Rev. Eukaryot. Gene Expr. 1993; 3(3): 193-227; Lyons RH, Mol. Cell Biol., 7, 2451-2456, 1987). Bei den Kerntransport vermittelnden Nukleinsäuresequenzen können verschiedene Prinzipien verwendet werden. Ein derartiges Prinzip besteht beispielsweise darin, dass, dass YB-1 als Fusionsprotein mit einem Signalpeptid ausgebildet wird und infolge des Signalpeptids YB-1 in den Zellkern geschleust wird und damit die erfindungsgemäße Replikation der Adenoviren erfolgt.

Ein weiteres Prinzip, welches bei der Ausgestaltung der erfindungsgemäß verwendeten Adenoviren zur Anwendung gelangen kann, besteht darin, dass YB-1 mit einer Transportsequenz versehen wird, die dazu führt, dass YB-1, bevorzugter Weise ausgehend von einer Synthese im Cytoplasma, in den Zellkern geschleust oder transloziert wird und dort die virale Replikation befördert. Ein Beispiel für eine besonders wirksame, den Kerntransport vermittelnde Nukleinsäuresequenz stellt die TAT-Sequenz von HIV dar, die neben weiteren geeigneten derartigen Nukleinsäuresequenzen beispielsweise beschrieben ist in Efthymiadis, A., Briggs, LJ, Jans, DA., JBC 273, 1623-1628, 1998. Dabei ist es Rahmen der vorliegenden Erfindung, dass die erfindungsgemäß verwendeten Adenoviren die Nukleinsäuresequenzen umfassen, die für die den Kerntransport codierenden Peptide codieren.

Es ist im Rahmen der vorliegenden Erfindung, dass YB-1 in vollständiger Länge vorliegt, insbesondere in einer Form, die dem Wildtyp von YB-1 entspricht. Es ist möglich, dass YB-1 als Derivat, zum Beispiel, in verkürzter oder trunkierter Form verwendet wird oder vorliegt. Ein YB-1-Derivat, wie es verwendet wird, oder vorliegt, ist dabei ein solches, das an den E2 late-Promotor zu binden in der Lage ist und dadurch die Genexpression von der adenoviralen E2-Region aktiviert. Derartige Derivate umfassen insbesondere die hierin offenbarten YB-1-Derivate. Weitere Derivate können durch Deletion einzelner oder mehrerer Aminosäuren am N-Terminus, am C-Terminus oder innerhalb der Aminosäuresequenz erzeugt werden.

Hinsichtlich der vorstehend genannten verschiedenen weiteren, von den Adenoviren codierten bzw. exprimierten Genen und Genprodukten ist es auch möglich, dass diese in beliebiger Kombination codiert bzw. exprimiert werden.

Die Verwendung der hierin offenbarten Adenoviren als Medikamente und insbesondere bei systemischer Anwendung kann durch ein geeignetes Targeting der Adenoviren verbessert werden. Die Infektion von Tumorzellen durch Adenoviren hängt bis zu einem bestimmten Umfang unter anderem vom Vorhandensein des Coxackievirus-Adenovirus Rezeptor CAR und bestimmten Integrinen ab. Sobald diese stark in Zellen, insbesondere Tumorzellen, exprimiert werden, ist eine Infektion bereits bei sehr geringen Titern (pfu/Zelle) möglich. Verschiedene Strategien sind bisher durchgeführt worden, um ein sogenanntes Re-targeting der rekombinanten Adenoviren zu erzielen, z. B. durch Insertion von heterologen Sequenzen in der fiber knob region, Verwendung von bi -spezifischen Antikörpern, beschichten der Adenoviren mit Polymeren, Einfügen von Liganden im Ad fiber, die Substitution des Serotyps 5 knop bzw. 5 fiber shaft und knop mit dem Serotyp 3 knop bzw. Ad 35 fiber shaft and knob und Modifikationen des penton base (Nicklin S. A. et al., Molecular Therapy 2001, 4, 534-542; Magnusson, M. K. et. al., J. of Virology 2001, 75, 7280-7289; Barnett B. G. et al., Biochimica et Biophysica Acta 2002, 1575, 1-14).

Der vorliegende Erfinder hat weiterhin überraschend gefunden, dass die Wirksamkeit der hierin beschriebenen und insbesondere der erfindungsgemäß verwendeten Viren dadurch erhöht werden kann, dass er in Kombination mit mindestens zwei Mitteln verwendet wird, wobei ein jedes der mindestens zwei Mittel einzeln und unabhängig aus der Gruppe ausgewählt ist, die Cytostatika umfasst.

Wie hierin in einer bevorzugten Ausführungsform verwendet sind Cytostatika insbesondere chemische oder biologische Verbindungen, die während oder nach der Verabreichung an eine Zelle oder einen Organismus, der eine oder diese Zelle enthält, dazu führten, dass die Zelle nicht mehr wächst und/oder sich nicht mehr teilt bzw. die Zellteilung und/oder das Zellwachstum verlangsamen. Cytostatika sind dabei auch solche Verbindungen, die erst in der Zelle oder in dem diese enthaltenden Organismus zu einem Cytostatikum im vorstehend beschriebenen Sinne werden. Insoweit umfasst der Begriff der Cytostatika auch Prä-Cytostatika.

Cytostatika werden bevorzugt nach ihrem Wirkmechanismus eingeteilt. Dabei werden die folgenden Gruppen verschieden:

- Alkylanzien, d.h. chemische Verbdinungen, die ihre zytotoxische Wirkung durch Alkylierung von Phosphat-, Amio-, Sulfhydryl-, Carboxyl- und Hydroxylgruppen der Nukleinsäure sowie Protein bedingen. Derartige Verbindungen wirken selbst oft kanzerogen. Typische Beispiele dieser Gruppe von Cytostatika sind Cisplatin bzw. Platin-Derivate, Cyclophosphamid, Dacarbazin, Mitomycin, Procarbazin.

- Antimetabolite, d.h. Verbindungen, die aufgrund struktureller Ähnlichkeit oder Fähigkeit zur Bindung einen Stoffwechselprozess blockieren oder beeinträchtigen. Innerhalb der Gruppe der Antimetabolite unterscheidet man wiederum zwischen strukturähnlichen Antimetaboliten, strukturverändernden Antimetaboliten und den indirekt wirkenden Antimetaboliten. Bei den strukturähnlichen Antimetabolite konkurrieren diese aufgrund chemischer Ähnlichkeit mit dem Metaboliten, ohne Übernahme dessen Funktion. Strukturverändernde Antimetaboliten binden den Metaboliten, was dessen Funktion oder Resorption verhindert oder den Metaboliten chemisch modifiziert. Indirekt wirkende Antimetaboliten beeinträchtigen die Funktion des Metaboliten, beispielsweise über Bindung von Ionen. Typische Beispiele dieser Gruppe sind Folsäureantagonisten wie z.B. Methotrexat, Pyrimidinanaloga wie z.B. Fluoruracil, Purinanaloga wie z.B. Azathioprin und Mercaptopurin.

- Mitosehemmstoffe, d.h. die Zellteilung hemmen. Innerhalb der Gruppe der Mitosehemmstoffe erfolgt eine Unterscheidung in Zellteilungsgifte, Spindelgifte und Chromosomengifte. Typische Beispiels dieser Gruppe sind Taxane und Vinca-Alkaloide. Die Taxana können dabei wiederum in die beiden Hauptgruppen Taxole und Taxotere eingeteilt werden, wobei ein besonders bevorzugtes Taxol Paclitaxel ist und ein besonders bevorzugtes Taxoter Docetaxel ist.

- Antibiotika mit hemmender Wirkung auf die DNA-abhängige RNA-Polymerase. Typische Beispiels dafür sind die Anthrazykline, so zum Beispiel Bleomycin, Daunorubicin, Doxorubicin und Mitomycin.

- Topoisomerase-Hemmer, insbesondere Topoisomerase-I-Hemmer. Topoisomerase-Hemmer sind chemische Verbindungen, die die Tertiärstruktur der DNA bestimmen, indem sie in einem dreistufigen Prozess die Veränderung der DNA-Verweindungszahl katalysieren. Dabei werden im wesentlichen zwei Formen von Topoisomerasen unterschieden. Topoisomerasen vom Typ I spalten nur einen DNA-Strang und sind ATP-unabhängig, wohingegen Topoisomerasen vom Typ II beide Stränge einer DNA spalten, wobei diese ATP-abhängig sind. Typische Beispiele hierfür sind Irinotecan und Topotecan für Topoisomerase-I- -Inhibitoren und Etoposid und Daunorubicin für Topoismerase II-Inhibitoren.

Im Rahmen der vorliegenden Erfindung werden mindestens zwei Mittel aus der vorstehenden Gruppe ausgewählt, wobei mindestens ein Mittel den Transport von YB-1 in den Zellkern vermittelt. Es können auch auch drei, vier oder fünf verschiedene Mittel ausgewählt werden. Die folgenden Ausführungen werden für die Ausführungsform der vorliegenden Erfindung gemacht, bei der lediglich zwei Mittel zusammen mit dem Virus verwendet werden. Die Ausführungen gelten sinngemäß auch für die Ausführungsform, bei der mehr als zwei Mittel verwendet werden.

Bevorzugterweise unterscheiden sich die Mittel darin, dass sie an unterschiedlichen Ziehnolkülen angreifen, oder in der Literatur an verschiedene Moleküle angreifend beschrieben werden. Es ist im Rahmen der vorliegenden Erfindung, dass das Mittel auch zwei oder mehr verschiedene Verbindungen umfasst, die an das gleiche Zielmolekül binden. Es ist jedoch auch im Rahmen der vorliegenden Erfindung, dass das eine Mittel an einem ersten Ort des Zielmoleküls bindet, während das zweite Mittel an einem zweiten Ort des Zielmoleküls bindet.

Es ist ebenfalls im Rahmen der vorliegenden Erfindung, dass mindestens zwei der Mittel über einen unterschiedlichen Wirkmechanismus aktiv sind. Aktiv bedeutend in einer bevorzugten Ausführungsform, dass die das Zellwachstum und/oder Zellteilung inhibierende oder verzögernde Wirkung der chemischen Verbindung jeweils über einen unterschiedlichen Wirkmechanismus aktiv ist. In einer besonders bevorzugten Ausführungsform bedeutet der Begriff aktiv, dass dadurch die Effizienz der Replikation eines Virus, insbesondere des erfindungsgemäßen Virus, der hierin beschriebenen Viren und/oder der hierin erfindungsgemäß zu verwendenden Viren, erhöht wird verglichen mit einer Situation, bei der eines und/oder beide der Mittel nicht verwendet werden. Als Maß für die Effizienz der Replikation des Virus wird bevorzugterweise die Anzahl der für die Zelllyse erforderliche Anzahl von Viren, bevorzugtererweise ausgedrückt als pfu/Zelle.

In einer besonders bevorzugten Ausführungsform ist eines der mindestens zwei Mittel ein solches, das die Infizierbarkeit der Zelle, in der die Replikation des Virus erfolgen soll, bevorzugt selektiv erfolgen soll, mit dem Virus, bevorzugterweise dem hierin beschriebenen Virus und/oder dem hierin erfindungsgemäß zu verwendenden Virus, erhöht. Dies kann beispielweise dadurch erfolgen, dass die Aufnahme des Virus durch die Zelle erhöht wird. Die Aufnahme des Virus, insbesondere von Adenovirus, wird beispielsweise durch den Coxsackievirus-Adenovirus Rezeptor vermittelt (CAR) (Mizuguchi und Hayakawa, GENE 285, 69-77, 2002) vermittelt. Eine erhöhte Expression von CAR wird beispielsweise durch Trichostatin A bedingt (Vigushin et al., Clinical Cancer Research, 7,971-976,2001).

In einer weiteren Ausführungsform ist eines der mindestens zwei Mittel ein solches, welches die Verfügbarkeit einer Komponente innerhalb der Zelle erhöht, wobei die Komponente eines solche ist, die die Replikation des Virus, bevorzugterweise dem hierin beschriebenen Virus und/oder dem hierin erfindungsgemäß zu verwendenden Virus, erhöht.

Erfindungsgemäß ist eines der mindestens zwei Mittel ein solches, welches den Transport von YB-1 in den Zellkern vermittelt. Ein derartiges Mittel kann aus der Gruppe ausgewählt sein, die Topoisomerase-Hemmer, Alkylanzien, Antimetabolite und Mitose-Hemmstoffe umfasst. Bevorzugte Topoisomerase-Hemmer sind Camptothecin, Irinotecan, Etoposide und deren jeweilige Analoga. Bevorzugte Mitose-Hemmstoffe sind Daunorubicin, Doxorubicin Paclitaxel und Docetaxel. Bevorzugte Alkylanzien sind Cisplatin und deren Analoga-Bevorzugte Antimetabolite sind Fluoruracil und Methotrexat.

In einer besonders bevorzugten Ausführungsform ist das eine der mindestens zwei Mittel ein solches, das die Infizierbarkeit der Zelle erhöht, insbesondere die Expression von CAR erhöht, und das zweite der mindestens zwei Mittel ein solches, das den Transport von YB-1 in den Zellkern erhöht, wobei bevorzugtererweise als chemische Verbindung eine solche verwendet wird, die die entsprechende erforderliche Eigenschaft aufweist, wie bevorzugt vorstehend erwähnt.

In einer weiteren Ausführungsform ist vorgesehen, dass das eine der mindestens zwei Mittel ein Histon-Deacylase-Inhibitor ist. Ein bevorzugter Histon-Deacylase-Inhibitor ist ein solcher, der aus der Gruppe ausgewählt ist, die Trichostatin A, FR901228, MS-27-275, NVP-LAQ824 und PXD101 umfasst. Trichostatin A ist beispielsweise beschrieben in Vigushin et al., Clinical Cancer Research, 7, 971-976, 2001; FR901228 ist beispielsweise beschrieben in Kitazono et al., Cancer Res., 61, 6328-6330, 2001; MS-27-275 beschrieben in Jaboin et al., Cancer Res., 62, 6108-6115, 2002; PXD101 beschrieben in Plumb et al., Mol. Cancer Ther., 8, 721-728, 2003; NVP-LAQ824 beschrieben in Atadja et al., Cancer Res., 64, 689-695, 2004.

In einer noch weiteren Ausführungsform ist vorgesehen, dass das eine der mindestens zwei Mittel ein Topoisomerase-Inhibitor, bevorzugterweise in Topoisomerase I-Inhibitor ist. Ein bevorzugter Topoismerase-Inhibitor ist ein solcher, der aus der Gruppe ausgewählt ist, die Camptothecine, Irinotecan, Topotecan, SN-38, 9-aminocamptothecin, 9-nitrocamptothecin DX-895If und Daunorubicin umfasst. Irinotecan und SN-38 ist beispielsweise beschrieben in Gilbert et al., Clinical Cancer Res., 9, 2940-2949, 2003; DX-895IF beschrieben in van Hattum et al., British Journal of Cancer, 87, 665-672, 2002; Camptothecin beschrieben in Avemann et al., Mol. Cell. Biol., 8, 3026-3034, 1988; 9-aminocamptothecin, 9-nitrocamptothecin beschrieben in Rajendra et al., Cancer Res., 63, 3228-3233, 2003; Daunorubicin beschrieben in M. Binaschi et al., Mol. Pharmacol., 51, 1053-1059.

In einer besonders bevorzugten Ausführungsform ist das eine der mindestens zwei Mittel ein Histon-Deacylase-Inhibitor und das andere der mindestens zwei Mittel ein Topoismerase-Inhibitor.

In einer Ausführungsform ist vorgesehen, dass das erfindungsmäße Mittel und/oder das erfindungsgemäß hergestellte Mittel den Virus getrennt von einem oder mehreren der mindestens zwei Mittel, die mit dem Virus erfindungsgemäß kombiniert sind, enthält. Dabei ist bevorzugt, dass der Virus von einem jeglichen Mittel, das mit dem Virus kombiniert wird, getrennt. Bevorzugterweise ist die Trennung eine räumliche Trennung. Die räumliche Trennung kann dabei so erfolgen ,dass der Virus in einer anderen Verpackung vorliegt als die Mittel. Bevorzugterweise handelt es sich bei der Verpackung um eine Einzeldose, d.h. der Virus und die Mittel sind in Einzeldosen verpackt. Die Einzeldosen können wiederum zu einer Packung zusammengefasst sein. Es ist jedoch auch im Rahmen der vorliegenden Erfindung, dass die Einzeldosen des Virus mit einer oder mehreren Einzeldosen eines oder mehrerer der Mittel kombiniert oder verpackt ist.

Die Art der Verpackung hängt dabei in der den Fachleuten auf dem Gebiet bekannten Art von der Art der Verabreichung ab. Bevorzugterweise wird der Virus als Lyophilisat oder in einer geeigneten flüssigen Phase vorliegen. Bevoruigterweise werden die Mittel in fester Form vorliegen, z.B. als Tabletten oder Kapseln, sind jedoch nicht darauf beschränkt. Alternativ können die Mittel auch in flüssiger Form vorliegen.

Es ist im Rahmen der vorliegenden Beschreibung dass der Virus systemisch oder lokak verabreicht wird. Es ist auch im Rahmen der vorliegenden Erfindung, dass die mit dem Virus kombinierten Mittel einzeln unabhängig voneinander oder gemeinsam systemisch oder lokal verabreicht werden. Den Fachleuten auf dem Gebiet sind weitere Verabreichungsformen bekannt.

Weiterhin kann der Virus und die mit ihm kombinierten Mittel zeitlich getrennt oder gleichzeitig verabreicht werden. Bei zeitlich getrennter Verabreichung ist es bevorzugt, dass die Mittel vor der Verabreichung des Virus verabreicht werden. Die Zeitspanne, wie lange vorher die Mittel vorher verabreicht werden, hängt von der Art der verwendeten Mittel ab und ergibt sich für den Fachmann aus der Wirkungsweise der verwendeten Mittel. Dabei kann auch die Verabreichung der mindestens zwei Mittel wiederum gleichzeitig oder zu unterschiedlichen Zeitpunkten erfolgen. Bei zeitlich unterschiedlicher Verabreichung ergeben sich die Zeitpunkte wiederum aus den den Mitteln zugrundeliegenden Wirkmechanismen und können von den Fachleuten auf der Grundlage davon festgelegt werden.

Die vorstehenden Ausführungen, die im Zusammenhang mit den Medikamenten gemäß der vorliegenden Erfindung, die hierin auch als pharmazeutische Zusammensetzungen offenbart und/oder bezeichnet werden, gemacht wurden, gelten sinngemäß auch für eine jegliche Zusammensetzung, so auch für Zusammensetzungen, wie sie für die Replikation von Viren verwendet werden, bevorzugterweise für die *in vitro* Replikation von Viren. Die vorstehenden Ausführungen gelten auch für den erfindungsgemäßen Kit bzw. den erfindungsgemäß verwendeten Kit, der neben der hierin beschrieben Viren bzw. den erfindungsgemäß verwendeten Viren, auch ein oder eine Kombination von Mitteln, wie hierin beschrieben, umfassen kann. Derartige Kits umfassen den Virus und/oder das oder die Mittel in einer für die Anwendung fertigen Form und bevorzugterweise noch eine Gebrauchsanweisung. Weiterhin gelten die vorstehenden Ausführungsformen auch für die hierin beschriebenen offenbarten Nukleinsäuren bzw. verwendeten Nukleinsäuren, und die erfindungsgemäßen Replikationssysteme und die dafür codierenden Nukleinsäuren bzw. die erfindungsgemäß verwendeten Replikationssysteme und die erfindungsgemäß verwendeten dafür codierenden Nukleinsäuren.

Im Rahmen der vorliegenden Erfindung sollen hierin die Begriffe Adenovirus und adenovirale Systeme als im wesentlichen die gleiche Bedeutung aufweisend verstanden werden. Unter Adenovirus soll dabei insbesondere das vollständige Viruspartikel verstanden werden umfassend das Kapsid und die Nukleinsäure. Der Begriff adenovirales System stellt insbesondere darauf ab, dass die Nukleinsäure gegenüber dem Wildtyp verändert ist. Bevorzugt umfassen derartige Änderungen solche im Aufbau des Genoms des Adenovirus wie sie durch Deletieren und/oder Hinzufügen und/oder Mutieren von Promotoren, regulativen Sequenzen und/oder codierende Sequenzen wie beispielsweise Leserahmen entstehen. Der Begriff adenovirale Systeme wird darüber hinaus bevorzugt in dem Zusammenhang verwendet, dass es sich dabei um einen Vektor handelt, der beispielsweise in der Gentherapie verwendet werden kann.

Die vorstehend gemachten Ausführungen, einschließlich jeglicher Verwendungen sowie die Ausbildungen der Adenoviren bzw. adenoviralen Systeme gelten im gleichen Maße für die dafür codierenden Nukleinsäuren und umgekehrt.

Im Zusammenhang mit der vorliegenden Erfindung ist es möglich, dass die erfindungsgemäß verwendeten Adenoviren bzw. die für sie codierenden Nukleinsäuren eine jede entsprechende adenovirale Nukleinsäure ist, die zu einem Replikationsereignis für sich oder in Verbindung mit weiteren Nukleinsäuresequenzen führt. Dabei ist es möglich, wie hierin ausgeführt, dass mittels Helferviren die für die Replikation erforderlichen Sequenzen und/oder Genprodukte bereitgestellt werden. Sofern hierin auf codierende Nukleinsäuresequenzen Bezug genommen wird und es sich dabei um solche Nukleinsäuresequenzen handelt, die bekannt sind, ist es im Rahmen der Erfindung, dass nicht nur die identische Sequenz verwendet wird, sondern auch hiervon abgeleitete Sequenzen. Unter abgeleiteten Sequenzen sollen hierin insbesondere solche Sequenzen verstanden sein, die noch zu einem Genprodukt, sei es eine Nukleinsäure oder ein Polypeptid, führen, das eine Funktion aufweist, die einer oder der Funktion der nicht abgeleiteten Sequenz entspricht. Dies kann durch einfache, dem Fachmann geläufige Routinetests festgestellt werden. Ein Beispiel für derartige abgeleitete Nukleinsäuresequenzen sind jene Nukleinsäuresequenzen, die für das gleiche Genprodukt, insbesondere für die gleiche Aminosäuresequenz kodieren, jedoch infolge der Degeneriertheit des genetischen Codes eine andere Basenabfolge aufweisen.

Hinsichtlich des erfindungsgemäßen Adenovirus und/oder des erfindungsgemäßen adenoviralen Replikationssystems, bzw. deren erfindungsgemäße Verwendung ist dabei in einer Ausführungsform vorgesehen, dass die adenovirale Nukleinsäure für die Expression des Onkogenproteins, insbesondere des E1A-Proteins defizient ist, d.h. entweder für das 12S E1A-Protein nicht codiert oder für das 13S E1A-Protein nicht codiert, oder modifiziert ist, wie hierin definiert, und das adenovirale Replikationssystem weiter eine Nukleinsäure eines Helfervirus umfasst, wobei die Nukleinsäure des Helfervirus eine Nukleinsäuresequenz umfasst, die für das Onkogenprotein, insbesondere das E1A-Protein codiert, welches die folgenden Eigenschaften aufweist bzw. dem Adenovirus die folgenden Eigenschaften verleiht, nämlich dass dieser bevorzugterweise nicht replizierend in YB-Kern-negativen Zellen aber sehr wohl in vom Zellzyklus unabhängig YB-1-Kern-positiven Zellen replizierend ist, transaktivierend auf ein adeno virales Gen, ausgewählt aus E1B55kDa, E4orf6, E4orf3 und/oder E3ADP, in YB-1 kernpositiven Zellen wirkt, und/oder zelluläres YB-1 nicht in den Kern transloziert. Es ist im Rahmen der vorliegenden Erfindung, dass die hierin beschriebenen Transgene einzeln oder gemeinsam von dem Helfervirus codiert und/oder exprimiert werden.

Weiterhin ist bei einem derartigen adenoviralen Replikationssystem vorgesehen, dass die adenovirale Nukleinsäure und/oder die Nukleinsäure des Helfervirus als replizierbarer Vektor vorliegt.

Dabei ist es weiter im Rahmen, dass die für die Adenoviren, die verwendet werden, codierende Nukleinsäure(n) in einem Vektor, bevorzugter Weise in einem Expressionsvektor vorliegt/vorliegen und dieser Expressionsvektor verwendet wird.

Auch offenbart ist eine Vektorgruppe umfassend mindestens zwei Vektoren, wobei die Vektorgruppe insgesamt ein adenovirales Replikationssystem umfasst, wie hierin beschrieben, und die Vektorgruppe erfindungsgemäß verwendet wird. Dabei kann vorgesehen sein, dass eine jede Komponente des adenoviralen Replikationssystems auf einem eigenen Vektor, bevorzugter Weise einem Expressionsvektor angeordnet ist.

Ebenso offenbart ist die Verwendung einer Zelle, die eine oder mehrere der Nukleinsäuren, wie sie für die erfindungsgemäße Verwendung der hierin beschriebenen Adenoviren, die erfindungsgemäß verwendet werden sollen, codiert, und/oder ein entsprechendes adenovirales Replikationssystem und/oder einen entsprechenden Vektor und/oder eine erfindungsgemäße Vektorgruppe umfasst, zu denselben Zwecken, wie hierin für die Adenoviren beschrieben

Die vorstehend beschriebenen Konstrukte von Adenoviren und insbesondere deren Nukleinsäuren bzw. die dafür codierenden Nukleinsäuren können auch in eine Zelle, insbesondere eine Tumorzelle, in Teilen eingebracht werden, wobei dann infolge der Anwesenheit der verschiedenen Einzelkomponenten diese so zusammenwirken, als stammten die Einzelkomponenten von einer einzelnen Nukleinsäure bzw. einem einzelnen oder mehreren Adenoviren.

Die verwendeten, für Adenoviren, adenovirale Systeme oder Teile davon codierenden Nukleinsäuren können als Vektoren vorliegen. Bevorzugter Weise handelt es sich um virale Vektoren. Im Falle der adenovirale Nukleinsäuren umfassenden Nukleinsäuren ist das Viruspartikel dabei bevorzugterweise der Vektor. Die besagten Nukleinsäuren können in einem Plasmidvektor vorliegen. In einem jeden Fall weist der Vektor Elemente auf, die für die Vermehrung der inserierten Nukleinsäure, d. h. Replikation und ggf. Expression der inserierten Nukleinsäure sorgen bzw. diese steuern. Geeignete Vektoren, insbesondere auch Expressionsvektoren, und entsprechende Elemente sind den Fachleuten auf dem Gebiet bekannt und beispielsweise beschrieben in Grunhaus, A., Horwitz, M.S., 1994, Adenoviruses as cloning vectors. In Rice, C., Hrsg., Seminars in Virology, London: Saunders Scientific Publications.

Der oben beschriebenen Ausführungsform, dass die verschiedenen Elemente der besagten Nukleinsäure nicht notwendigerweise auf nur einem Vektor enthalten sein müssen, trägt dem Aspekt Rechnung, der die Vektorgruppe betrifft. Eine Vektorgruppe umfasst entsprechend mindestens zwei Vektoren. Ansonsten gilt betreffend die Vektoren bzw. die Vektorengruppe das hierin allgemein zu Vektoren Ausgeführte.

Die verwendeten Adenoviren sind durch die verschiedenen hierin offenbarten Nukleinsäuren bzw.

Genprodukte charakterisiert und können ansonsten all jene den Fachleuten auf dem Gebiet bekannten Elemente umfassen, wie dies auch bei Adenoviren vom Wildtyp der Fall ist (Shenk, T.: Adenoviridae: The virus and their replication. Fields Virology, 3. Auflage, Hrsg. Fields, B.N., Knipe, D.M., Howley, P.M. et al., Lippincott-Raven Publishers, Philadelphia, 1996, Kapitel 67).

Die Replikation von Adenoviren ist ein ausgesprochen komplexer Vorgang und greift im Regelfalle auf den humanen Transkriptionsfaktor E2F zurück Während einer viralen Infektion werden zunächst die "frühen Gene" E1, E2, E3 und E4 exprimiert. Die Gruppe der "späten Gene" ist für die Synthese der viralen Strukturproteine verantwortlich. Für die Aktivierung sowohl der frühen wie auch der späten Gene spielt die E1-Region bestehend aus zwei Transkriptionseinheiten E1A und E1B, welche für verschiedene E1A- und E1B-Proteine codieren, eine entscheidende Rolle, da sie die Transkription der E2, E3, E4-Gene induzieren (Nevins, J. R., Cell 26, 213-220, 1981). Zudem können die E1A-Proteine in ruhenden Zellen die DNA-Synthese induzieren und so deren Eintritt in die S-Phase einleiten (siehe Boulanger and Blair, 1991). Darüber hinaus interagieren sie mit den Tumorsuppressoren der Rb-Klasse (Whyte, P. et al., Nature 334, 124-127, 1988). Dabei wird der zelluläre Transkriptionsfaktor E2F freigesetzt. Die E2F-Faktoren können dann an die entsprechenden Promotorbereiche sowohl zellulärer wie auch viraler Gene binden (insbesonder an den adenoviralen E2 early Promotor) und die Transkription und somit die Replikation einleiten (Nevins, J. R., Science 258, 424-429, 1992).

Für das Einleiten bzw. die Durchführung der Replikation werden insbesondere die Genprodukte der E2-Region benötigt, da sie für drei essentielle Proteine kodieren. Die Transkription der E2-Proteine wird durch zwei Promotoren gesteuert, den "E2-Early E2F-abhängigen", hierin auch als E2-early Promotor oder früher E2-Promotor bezeichnet, und den "E2-late" Promoter (Swaminathan und Thimmapaya, The Molecular Repertoire of Adenoviruses III: Current Topics in Microbiology and Immunology,Vol 199, 177-194, Springer Verlag 1995). Zudem spielen die Produkte der E4-Region zusammen mit dem E1A- und E1B-55kDa-Protein eine wichtige Rolle für die Aktivität von E2F bzw. die Stabilität von p53. Zum Beispiel wird durch eine direkte Interaktion des von der E4-Region codierten E4orf6/7-Proteins mit dem Heterodimer bestehend aus E2F und DP der E2-Promoter noch stärker transaktiviert (Swaminathan und Thimmapaya, JBC 258, 736-746, 1996). Weiterhin wird p53 durch den Komplex bestehend aus E1B-55kDa und E4orf6 inaktiviert (Steegenga, W. T. et al., Oncogene 16, 349-357, 1998), um einen erfolgreichen lytischen Infektionszyklus durchlaufen zu können. Ferner besitzt das E1B-55kDa Protein eine weitere wichtige Funktion insoweit, als dass es in Wechselwirkung mit dem E4orf6 Protein den Export der viralen RNA aus dem Zellkern fördert, wohingegen die zelleigenen RNAs im Kern zurückgehalten werden (Bridge und Ketner, Virology 174, 345-353, 1990). Eine weitere wichtige Beobachtung ist die, dass der Proteinkomplex bestehend aus E1B-55kDa/E4orf6 in den sogenannten "viral inclusion bodies" lokalisiert ist. Es wird angenommen, dass diese Strukturen Orte der Replikation und Transkription darstellen (Ornelles und Shenk, J. Virology 65, 424-429, 1991).

Eine weitere für die Replikation und insbesondere für die Freisetzung von Adenoviren besonders wichtige Region ist die E3-Region. Die E3-Region enthält genauer die genetische Information für eine Vielzahl von relativ kleinen Proteinen, die für den adenoviralen Infektionszyklus in vitro, d.h. in der Zellkultur nicht essentiell sind. Sie spielen jedoch für das Überleben des Virus während einer akuten und/oder latenten Infektion in vivo eine bedeutende Rolle, da sie unter anderem immunregulatorische und apoptotische Funktion(en) besitzen (Marshall S. Horwitz, Virololgie, 279, 1-8, 2001; Russell, a.a.O.). Es konnte gezeigt werden, dass ein Protein mit einer Größe von ca. 11,6 kDa den Zelltod induziert. Das Protein wurde infolge seiner Funktion als ADP - für den englischen Begriff adenovirus death protein - bezeichnet (Tollefson, J. Virology, 70, 2296-2306, 1996). Das Protein wird vornehmlich in der späten Phase des Infektionszyklus gebildet. Ferner führt die Überexpression des Proteins zu einer besseren Lyse der infizierten Zellen (Doronin et al., J. Virology, 74, 6147-6155, 2000).

Weiterhin ist dem vorliegenden Erfinder bekannt, dass E1A-deletierte Viren, d.h. insbesondere solche Viren, die kein 12S E1A-Protein und auch kein 13S E1A-Protein exprimieren, bei höheren MOI's sehr effizient replizieren können (Nevins J. R., Cell 26, 213-220, 1981), die jedoch in der klinischen Anwendung nicht zu realisieren sind. Dieses Phänomen wird in der Literatur als "E1A-like activity" bezeichnet. Ferner war bekannt, dass von den von E1A insgesamt 5 codierten Proteinen zwei Proteine, nämlich das 12S- und das 13S-Protein, die Expression der anderen adenoviralen Gene steuern bzw. induzieren (Nevins, J. R., Cell 26, 213-220, 1981; Boulanger, P. und Blair, E.; Biochem. J. 275, 281-299, 1991). Dabei hat sich gezeigt, dass hauptsächlich die CR3-Region des 13S-Proteins die transaktivierende Funktion ausübt (Wong HK und Ziff EB., J Virol., 68, 4910-20, 1994). Adenoviren, die bestimmte Deletionen in der CR1-und/oder CR2-Region und/oder CR3-Region des 13S-Proteins aufweisen, sind weitestgehend replikationsdefekt, wirken aber noch bei einzelnen Zelllinien transaktivierend auf die viralen Gene bzw. Promotoren, insbesondere auf die E2 Region (Wong HK, Ziff EB., J Virol. 68, 4910-20, 1994; Mymryk, J. S. und Bayley, S. T., Virus Research 33, 89-97,1994).

Nach Infektion einer Zelle, typischerweise einer Tumorzelle, mit einem Wildtyp-Adenovirus wird YB-1 vermittelt durch E1A, E1B-55K und E4orf6 in den Kern induziert und co-lokalisiert mit E1B-55K im Kern in den viral inclusion bodies, was eine effektive Replikation des Virus im Zellkern sowohl in vitro als auch in vivo erlaubt. Dabei war bereits früher festgestellt worden, dass auch E4orf6 an E1B-55 K bindet (Weigel, S. und Dobbelstein , M. J. Virology, 74, 764-772, 2000; Keith N. Leppard, Seminars in Virology, 8, 301-307, 1998.) und somit den Transport bzw. die Verteilung von E1B-55K in den Kern vermittelt, was eine optimale Virusproduktion bzw adenovirale Replikation gewährleistet. Durch das Zusammenwirken von E1A, E1B-55K und YB-1 bzw. durch den Komplex aus E1B-55K/E4orf6 mit YB-1 und der Kolokalisation von YB-1 und E1B-55K im Kern in den sogenannten viral inclusion bodies ist eine erfindungsgemäße effiziente Replikation des Virus, und damit die Verwendung der hierin beschriebenen Viren zur Replikation in Zellen, die YB-1-Kern-positiv sind, bzw. zur Herstellung eines Medikamentes zur Behandlung von Erkrankungen, bei denen YB-1-Kern-positive Zellen beteiligt sind, möglich. Die dadurch vor diesem zellulären Hintergrund mögliche Replikation führt zu einer Lyse der Zelle, Freisetzung des Virus und Infektion und Lyse benachbarter Zellen, so dass im Falle der Infektion einer Tumorzelle bzw. eines Tumors letztlich eine Lyse des Tumors, d.h. eine Onkolyse, eintritt.

YB-1 gehört zu einer Gruppe hoch konservierter Faktoren, die an der invertierten CAAT-Sequenz, der sogenannten Y-Box, binden. Sie können sowohl auf der Ebene der Transkription als auch der Translation regulatorisch wirken (Wolffe, A. P. Trends in Cell Biology 8, 318-323, 1998). Es werden immer mehr Y-Box-abhängige Regulationswege bei der Aktivierung aber auch bei der Hemmung Wachstums- und Apoptose-assoziierter Gene aufgefunden (Swamynathan, S. K. et al.,FASEB J. 12, 515-522, 1998). So interagiert YB-1 direkt mit p53 (Okamoto, T. et al., Oncogene 19, 6194-6202, 2000), spielt eine wichtige Rolle bei der Fas-Genexpression (Lasham, A. et al., Gene 252, 1-13, 2000), MDR und MRP-Genexpression (Stein, U. et al., JBC 276, 28562-69, 2001; Bargou, R. C. et al., Nature Medicine 3, 447-450, 1997) und bei der Aktivierung von Topoisomerasen und Metalloproteinasen (Mertens. P. R. et al., JBC 272, 22905-22912, 1997; Shibao, K. et al., Int. J. Cancer 83, 732-737, 1999). Zudem ist YB-1 an der Regulation der mRNA-Stabilität (Chen, C-Y. et al., Genes & Development 14, 1236-1248, 2000) und an Reparaturvorgängen beteiligt (Ohga, T. et al., Cancer Res. 56, 4224-4228, 1996;).

Die nukleäre Lokalisation von YB-1 in Tumorzellen führt zu einer E1A-unabhängigen viralen Replikation, bei der insbesondere weder ein 12S E1A-Protein noch ein 13S E1A-Protein exprimiert vorliegt bzw. verwendet wird, (Holm, P. S. et al. JBC 277, 10427-10434, 2002) und im Falle der Überexpression des Proteins YB-1 zu einer multidrug resistance (Vielfachresistenz). Zudem ist bekannt, dass die adenoviralen Proteine wie z. B. E4orf6 und E1B-55K einen positiven Effekt auf die virale Replikation ausüben (Goodrum, F. D. und Ornelles, D. A, J. Virology 73, 7474-7488, 1999), wobei ein funktionelles E1A Protein für das Einschalten der anderen viralen Genprodukte (z. B. E4orf6, E3ADP und E1B-55K) verantwortlich ist (Nevins J. R., Cell 26, 213-220, 1981). Dies unterbleibt jedoch bei den im Stand der Technik bekannten E1A-minus Adenoviren, bei denen das 13S E1A-Protein nicht vorhanden ist. Die Kernlokalisation von YB-1 in multidrug resistenten Zellen, die YB-1 im Kern aufweisen, erlaubt die Replikation bzw. Partikelbildung derartiger E1A-minus Viren. Hierbei ist jedoch die Effizienz der viralen Replikation bzw. Partikelbildung im Vergleich zum Wildtyp Ad5 um ein Vielfaches geringer. Eine Kombination von YB-1, welches entweder bereits im Zellkern der Tumorzelle enthalten ist, oder durch äußere Faktoren (z.B. Applikation von Zytostatika oder Bestrahlung oder Hyperthermie) in den Zellkern induziert, d.h. veranlasst wird, im Zellkern vorhanden zu sein, insbesondere unabhängig vom Zellzyklus vorhanden zu sein, oder als Transgen durch einen Vektor eingeführt wird, mit einem System, bevorzugterweise mit einem adenoviralen System, welches die adenoviralen Gene einschaltet, aber nicht zur viralen Replikation fähig ist, stellt demgegenüber überraschenderweise ein System dar, welches eine sehr effektive virale Replikation bzw. Partikelbildung durch YB-1 vermittelt und damit eine Onkolyse erlaubt. Geeignete Zytostatike sind beispielsweise solche, die zu den folgenden Gruppen gehören: Anthracycline, wie beispieleweise Daunomycin und Adriamycin; Alkylanzien, wie beispeislweise Cyclophosphamid; Alkaloide, wie beispielsweise Etoposid; Vin-Alkaloide, wie beispielsweise Vincristin und Vinblastin; Antimetabolite wie beispielsweise 5-Fluorouracil und Methrothrexat; Platin-Derivate, wie beispielsweise cis-Platin; Topoisomerase-Inhibitoren, wie beispielsweise Camphothecin; und Taxane, wie beispielsweise Taxol. Die hierin offenbarten Adenoviren, insbesondere rekombinanten Adenoviren, welche nur in YB-1-Kern-positiven Zellen zur Replikation befähigt sind, sind in ihrer Fähigkeit, transaktivierend auf die viralen Gene E1B-55K, E4orf6, E4orf3 und E3ADP zu wirken, beschränkt, verglichen mit den diesbezüglichen transaktivierenden Fähigkeiten von Adenoviren vom Wildtyp, insbesondere vom Wildtyp Ad5. Der vorliegende Erfinder hat nun überraschenderweise festgestellt, dass diese beschränkte transaktivierende Fähigkeit dadurch aufgehoben werden kann, dass die entsprechenden Gene und insbesondere E1B-55K und E4orf6 in Verbindung mit der Kernlokalisation von YB-1 zur Expression gebracht werden. Wie in den Beispielen hierin gezeigt wird, erhöht sich die virale Replikation bzw. Partikelbildung unter diesen Umständen auf ein Niveau, welches nahezu vergleichbar ist mit dem Replikationsverhalten bzw. Partikelbildungsverhalten von Adenoviren vom Wildtyp.

Bei dem Medikament, im Rahmen dessen oder bei dessen Herstellung die hierin beschriebenen Adenoviren verwendet werden, ist vorgesehen, dass dieses in der Regel systemisch appliziert wird, gleichwohl es auch, wenn dieses lokal appliziert oder abgegeben wird. Die Applikation erfolgt mit der Absicht, dass insbesondere jene Zellen mit dem Adenovirus infiziert werden und insbesondere darin eine Replikation der Adenoviren erfolgt, bei denen eine Beteiligung, bevorzugter Weise kausal, an der Ausbildung eines Zustandes, typischerweise einer Erkrankung, vorliegt, zu deren Diagnose und/oder Prävention und/oder Behandlung das erfindungsgemäße Medikament verwendet wird.

Ein derartiges Medikament ist bevorzugter Weise für die Behandlung von Tumorerkrankungen vorgesehen. Dabei sind jene Tumorerkrankungen besonders bevorzugt, bei denen entweder YB-1 bereits im Zellkern infolge des der Tumorerkrankung zugrundeliegenden Mechanismus, insbesondere des zugrundeliegenden pathologischen Mechanismus, vorliegt, oder aber durch äußere Maßnahmen die Anwesenheit von YB-1 im Zellkern bedingt wird, wobei die Maßnahmen geeignet sind, YB-1 in den Zellkern zu transferieren, dort zu induzieren oder dort zu exprimieren. Der Begriff Tumor oder Tumorerkrankung soll hierbei sowohl maligne wie benigne Tumoren und entsprechende Erkrankungen bezeichnen. Dabei kann vorgesehen sein, dass das Medikament mindestens eine weitere pharmazeutisch wirksame Verbindung enthält. Die Art und der Umfang dieser weiteren pharmazeutisch aktiven Verbindungen wird dabei von der Art der Indikation abhängen, für die das Medikament eingesetzt wird. Im Falle der Verwendung des Medikamentes für die Behandlung und/oder die Prophylaxe von Tumorerkrankungen werden typischerweise Zytostatika, wie beispielsweise cis-Platin und Taxol, Daunoblastin, Daunorubicin, Adriamycin (Doxorubicin) und/oder Mitoxantron oder andere der hierin beschriebenen Zytostatika oder Gruppen von Zytostatika verwendet.

Das erfindungsgemäße Medikament kann dabei in verschiedenen Formulierungen vorliegen, bevorzugter Weise in einer flüssigen Form. Weiterhin wird das Medikament Hilfsstoffe wie Stabilisatoren, Puffer, Konservierungsstoffe und dergleichen enthalten, die dem Fachmann auf dem Gebiet der Galenik bekannt sind.

Der vorliegende Erfinder hat überraschenderweise festgestellt, dass die erfindungsgemäße Verwendung der hierin beschriebenen Viren mit besonders großer Erfolgsrate bei solchen Tumoren verwendet werden kann, bei denen YB-1 unabhängig vom Zellzyklus im Zellkern vorkommt. Normalerweise ist YB-1 im Cytoplasma, insbesondere auch im perinukleären Plasma, vorhanden. In der S-Phase des Zellzyklus findet sich YB-1 im Zellkern von sowohl normalen wie auch Tumorzellen. Dies ist jedoch nicht ausreichend, um eine virale Onkolyse unter Verwendung derartiger modifizierten Adenoviren zu bewerkstelligen. Die im Stand der Technik beschriebene vergleichsweise geringe Wirksamkeit von derartigen attenuierten Adenoviren beruht letztlich auf deren fehlerhaften Anwendung. Mit anderen Worten, es können derartige adenovirale Systeme, insbesondere auch mit einer größeren Wirksamkeit dort eingesetzt werden, wo die molekularbiologischen Voraussetzungen für eine virale Onkolyse unter Verwendung dieser, hierin beschriebenen attenuierten oder modifizierten Adenoviren gegeben sind. Im Falle der hierin erfindungsgemäß zu verwendend beschriebenen Adenoviren, wie beispielsweise AdΔ24, d1922-947, E1Ad/01/07, CB016, d1520 und die im europäischen Patent EP 0 931 830 beschriebenen rekombinanten Adenoviren, liegen diese Voraussetzungen bei solchen Tumorerkrankungen vor, deren Zellen eine vom Zellzyklus unabhängige Kernlokalisation von YB-1 aufweisen. Diese Form der Kernlokalisation kann dabei durch die Art des Tumors selbst bedingt sein, oder aber durch die hierin beschriebenen erfindungsgemäßen Agenzien oder Maßnahmen bewirkt werden. Die vorliegende Erfindung definiert somit eine neue Gruppe von Tumoren bzw. Tumorerkrankungen und damit auch von Patienten, die mit den erfindungsgemäßen Viren, besonders aber auch mit den im Stand der Technik bereits beschriebenen attenuierten oder modifizierten Adenoviren, noch wirksam behandelt werden können.

Eine weitere Gruppe von Patienten, die erfindungsgemäß unter Verwendung der hierin als erfindungsgemäß zu verwenden beschriebenen, im Stand der Technik als solches wenigstens zum Teil bekannten Adenoviren, oder unter Verwendung der hierin erstmalig beschriebenen Adenoviren behandelt werden können, insbesondere unter Verwendung solcher Adenoviren, die Mutationen bzw. Deletionen im E1A-Protein aufweisen, welche die Bindungen von Rb/E2f nicht stören oder aber in YB-1-Kern-negativen Zellen nicht replizieren oder eine stark verringerte Replikation, wie hierin definiert und/oder ein deletiertes Onkoprotein, insbesondere E1A, aufweisen bzw. zeigen, wie beispielsweise im Falle der Viren AdΔ24, d1922-947, E1Ad/01/07, CB106 und der im europäischen Patent EP 0931 830 beschriebenen Adenoviren, sind jene Patienten, bei denen durch Anlegen oder Realisieren bestimmter Bedingungen gewährleistet wird, dass YB-1 in den Kern wandert oder dort induziert oder dorthin transportiert wird. Die Verwendung derartiger Adenoviren bei dieser Patientengruppe beruht insoweit auf der Erkenntnis, dass die Induktion der viralen Replikation auf der Kernlokalisation von YB-1 mit anschließender Bindung von YB-1 an den E2-late-Promotor beruht. Infolge der hierin offenbarten Erkenntnisse können Adenoviren wie beispielsweise AdΔ24, d1922-947, E1Ad/01/07, CB106 und/oder die im europäischen Patent EP 0931 830 beschriebenen, auch in solchen Zellen replizieren, die YB-1 Kern-positiv sind und/oder in Zellen, in den YB-1 dereguliert im Sinne der vorliegenden Erfindung vorliegt. Insoweit können diese Adenoviren erfindungsgemäß für die Behandlung von Erkrankungen bzw. Patientengruppen verwendet werden, die Zellen mit diesen Eigenschaften aufweisen, insbesondere dann, wenn diese Zellen an der Ausbildung der jeweiligen zu behandelnden Erkrankung beteiligt sind. Dies begründet den Erfolg von AdΔ24, d1922-947, E1Ad/01/07, CB016 und der im Patent EP 0931 830 beschriebenen Adenoviren bei der erfindungsgemäßen Behandlung solcher Tumoren, die YB-1 zellzyklusunabhängig im Kern oder YB-1 dereguliert im Sinne der vorliegenden Offenbarung aufweisen. Eine weitere Gruppe von Patienten, die erfindungsgemäß unter Verwendung der hierin als erfindungsgemäß zu verwenden beschriebenen Viren bzw. unter Verwendung der hierin erstmalig beschriebenen Viren, insbesondere Adenoviren, behandelbar sind, sind somit solche, die YB-1 Kern-positiv sind und/oder YB-1 Kern-positiv sind als Ergebnis der nachfolgend beschriebenen Behandlungen und/oder solche Patienten, die eine der nachfolgenden Maßnahmen, bevorzugt im Sinne einer Behandlung, erfahren haben oder gleichzeitig mit der Gabe der entsprechenden Viren erfahren. Dabei ist es im Rahmen der vorliegenden Erfindung, dass YB-1 Kern-positive Patienten solche Patienten sind, die insbesondere in einer Anzahl der einen Tumor ausbildenden Zellen YB-1 unabhängig vom Zellzyklus im Kern aufweisen. Zu diesen Maßnahmen gehört die Verabreichung von Cytostatika, wie sie hierin insgesamt beschrieben sind und/oder im Rahmen einer Tumortherapie verwendet werden. Weiterhin gehört zu dieser Gruppe von Maßnahmen Bestrahlung, insbesondere eine Bestrahlung, wie sie im Rahmen einer Tumortherapie angewandt wird. Bestrahlung bedeutet dabei insbesondere die Bestrahlung mit energiereicher Strahlung, bevorzugterweise mit radioaktiver Strahlung, bevorzugtererweise wie sie im Rahmen einer Tumortherapie verwendet wird. Eine weitere Maßnahme stellt Hyperthermie bzw. das Anlegen von Hyperthermie dar, bevorzugterweise Hyperthermie, wie sie im Rahmen einer Tumortherapie verwendet wird. In einer ganz besonders bevorzugten Ausführungsform ist dabei vorgesehen, dass die Hyperthermie lokal angewandt wird. Schließlich ist eine weitere Maßnahme die Hormonbehandlung, insbesondere die Hormonbehandlung, wie sie bei der Tumorbehandlung zur Anwendung gelangt. Im Rahmen einer derartigen Hormonbehandlung werden Anti-Östrogene und/oder Anti-Androgene verwendet. Dabei werden Anti-Östrogene, wie beispielsweise Tamoxifen, insbesondere bei der Therapie von Brustkrebs verwendet und Anti-Androgene, wie beispielsweise Flutamid oder Cyproteronacetat, bei der Therapie von Prostatakrebs verwendet.

Dabei ist es im Rahmen der vorliegenden Erfindung, wenn einige der den Tumor aufbauenden Zellen YB-1 entweder inhärent oder nach Induktion bzw. aktivem Einführen in den Kern dort aufweisen oder aber hinsichtlich YB-1 dereguliert im Sinne der vorliegenden Offenbarung vorliegt. Bevorzugterweise sind etwa 5 % oder ein jeglicher Prozentsatz darüber, d. h. 6 %, 7 %, 8 % etc. der den Tumor aufbauenden Zellen derartige YB-1 Kern-positive Zellen oder Zellen, in denen YB-1 dereguliert vorliegt. Die Kernlokalisation von YB-1 kann durch Stress von außen bzw. lokal appliziertem Stress induziert werden. Diese Induzierung kann beispielsweise durch Bestrahlung, insbesondere UV-Bestrahlung, Anwendung von Zytostatika, wie sie unter anderem hierin ebenfalls offenbart sind, und Hyperthermie erfolgen. Im Zusammenhang mit Hyperthermie ist beachtlich, dass diese zwischenzeitlich sehr spezifisch, insbesondere örtlich spezifisch, realisiert werden kann und somit ebenfalls spezifisch einen Kerntransport von YB-1 in den Zellkern bedingen kann und infolgedessen die Voraussetzungen für eine Replikation des Adenovirus und damit einer Zell- und Tumorlyse, die bevorzugt lokal beschränkt erfolgt, gegeben sind (Stein U, Jurchott K, Walther W, Bergmann S, Schlag PM, Royer HD. J Biol Chem. 2001,276(30):28562-9; Hu Z, Jin S, Scotto KW. J Biol Chem. 2000 Jan 28; 275(4):2979-85; Ohga T, Uchiumi T, Makino Y, Koike K, Wada M, Kuwano M, Kohno K. J Biol Chem. 1998, 273(11):5997-6000).

Das erfindungsgemäße Medikament würde somit auch an solche Patienten und Patientengruppen verabreicht werden bzw. wäre für solche bestimmt, bei denen durch geeignete Vorbehandlungen oder gleichzeitige Behandlung ein Transport von YB-1, insbesondere in die entsprechenden Tumorzellen, bedingt werden würde.

Auf der Grundlage dieser technischen Lehre ist es für den Fachmann im Rahmen seiner Fähigkeiten, geeignete Modifikationen insbesondere von E1A vorzunehmen, die beispielsweise Deletionen oder Punktmutationen umfassen können, um so verschiedene Ausführungsformen der im Rahmen der erfindungsgemäßen Verwendung einsetzbaren Adenoviren zu erzeugen.

Wie bereits vorstehend ausgeführt, sind die erfindungsgemäß verwendeten Adenoviren in der Lage, in solchen Zellen bzw. zellulären Systemen zu replizieren, die YB-1 im Kern aufweisen. Für die Frage, inwieweit die erfindungsgemäß verwendeten Adenoviren zur Replikation und damit zur Tumorlyse in der Lage sind, ist der Status der Zellen hinsichtlich des Vorhandenseins oder Nichtvorhandenseins von Rb, d. h. des Retinoblastom-Tumorsupressor-Produktes, unabhängig. Weiterhin ist es im Rahmen der erfindungsgemäßen Verwendung der besagten Adenoviren nicht erforderlich, auf den p53-Status der infizierten, zu infizierenden oder zu behandelnden Zellen abzustellen, da bei Verwendung der hierin offenbarten adenoviralen Systeme im Zusammenhang mit YB-1-Kern-positiven Zellen, d.h. Zellen, die unabhängig vom Zellzyklus YB-1 im Kern aufweisen, dieser ebenso wie der Rb-Status auf das Replikationsgeschehen des Adenovirus keinen Einfluss für die Ausführung der hierin offenbarten technischen Lehre hat.

Das Onkogen bzw. Onkogenprotein E1A kann dabei entweder unter der Kontrolle der eigenen natürlichen adenoviralen Promotoren stehen und/oder aber über einen Tumor- oder Gewebe-spezifischen Promotor gesteuert werden. Geeignete nicht-adenovirale Promotoren können ausgewählt sein aus der Gruppe, die Cytomegalovirus-Promotor, RSV-(Rous sarcoma Virus) - Promotor, Adenovirus-basierender Promotor Va I und den nicht-viralen YB-1-Promotor (Makino Y. et al., Nucleic Acids Res. 1996, 15, 1873-1878) umfasst. Weitere Promotoren, die im Zusammenhang mit einem jeden Aspekt der hierin offenbarten Erfindung verwendet werden können, stellen der Telomerase-Promotor, der Alpha-Fetoprotein (AFP)-Promotor, der Caecinoembryonic Antigen Promotor (CEA) (Cao, G., Kuriyama, S., Gao, J., Mitoro, A., Cui, L., Nakatani, T., Zhang, X., Kikukawa, M., Pan, X., Fukui, H., Qi, Z. Int. J. Cancer, 78,242-247, 1998), der L-Plastin-Promotor (Chung, I., Schwartz, PE., Crystal, RC., Pizzorno, G, Leavitt, J., Deisseroth, AB.

Cancer Gene Therapy, 6, 99-106, 1999), Argenin-Vasopressin-Promotor (Coulson, JM, Staley, J., Woll, PJ. British J. Cancer, 80, 1935-1944, 1999), E2f-Promotor (Tsukada et al. Cancer Res., 62, 3428 - 3477), Uroplakin II Promotor (Zhang et al., Cancer Res., 62, 3743-3750, 2002) und der PSA-Promotor (Hallenbeck PL, Chang, YN, Hay, C, Golightly, D., Stewart, D., Lin, J., Phipps, S., Chiang, YL. Human Gene Therapy, 10, 1721-1733, 1999) dar. Ferner stellt der in der deutschen Patentanmeldung DE 101 50 984.7 beschriebenen YB-1 abhängigen E2-late-Promotor von Adenoviren einen Promotor dar, wie er im Rahmen der vorliegenden Erfindung verwendet werden kann.

Hinsichtlich des Telomerase-Promotors ist bekannt, dass dieser in humanen Zellen von zentraler Bedeutung ist. So wird die Telomeraseaktivität durch die transkriptionelle Kontrolle des Telomerase reverse Transkriptase-Gens (hTERT), welches die katalytische Untereinheit des Enzyms darstellt, reguliert. Die Expression der Telomerase ist in 85% der humanen Tumorzellen aktiv. Im Unterschied dazu ist sie in den meisten normalen Zellen inaktiv. Ausgenommen davon sind Keimzellen und embryonales Gewebe (Braunstein, I. et al., Cancer Research, 61, 5529-5536, 2001; Majumdar, A. S. et al., Gene Therapy 8, 568-578, 2001). Genaue Untersuchungen am hTERT-Promotor haben gezeigt, dass Fragmente des Promotors von 283 bp bzw. 82 bp vom Initiationscodon entfernt für eine spezifische Expression in Tumorzellen ausreichend sind (Braunstein I. et al.; Majumdar AS et al., aaO). Daher eignet sich dieser Promotor bzw. die spezifischen Fragmente, um eine spezifische Expression eines Gens und insbesondere eines Transgens, bevorzugterweise eines hierin offenbarten Transgens, nur in Tumorzellen zu erzielen. Der Promotor soll die Expression des modifizierten Onkogens, bevorzugt des E1A-Onkogenproteins, nur in Tumorzellen ermöglichen. Auch ist die Expression eines Transgens, insbesondere eines solchen, das ausgewählt ist aus der Gruppe, die E4orf6, E1B55kD, ADP und YB-1 umfasst, in einem adenoviralen Vektor unter einem dieser Promotoren in einer Ausführungsform vorgesehen. Es ist auch im Rahmen der vorliegenden Erfindung, dass der Leserahmen des transaktivierenden Onkogenproteins, insbesondere des E1A-Proteins im Leserahmen (engl. "in frame") mit einem oder mehreren der Genprodukte des adenoviralen Systems ist. Der Leserahmen des transalctivierenden E1A-Proteins kann jedoch auch unabhängig davon sein.

Die verschiedenen Transgene, so auch EIB55kD, E4orf6, ADP und dergleichen, insbesondere wenn es sich dabei um virale Gene handelt, können grundsätzlich aus einem jeden entsprechenden Virus, bevorzugterweise Adenovirus und bevorzugtererweise Adenovirus Ad5, cloniert werden. Im Stand der Technik sind darüber hinaus eine Vielzahl von Plasmiden beschrieben, die die entsprechenden Gene enthalten, und aus denen diese sodann entnommen und in die erfindungsgemäßen Adenoviren ebenso wie in die hierin gemäß der Erfindung zu verwendenden Viren eingefügt werden können. Ein Beispiel für ein Plasmid, das E1B55kD exprimiert, ist beispielsweise beschrieben von Dobbelstein, M. et al., EMBO Journal, 16, 4276-4284, 1997. Die codierende Region des E1B55K-Gens kann zusammen mit der 3'-nicht-codierenden Region (die 3'UTR-Region liegt bevorzugterweise etwa bei der Basenposition 3507-4107 im Adenovirus-Wildtypgenom) beispielsweise aus diesem Gen mittels Bam HI aus dem Plasmid pDCRE1B herausgeschnitten werden. Das entsprechende Fragment umfassend das E1B55kD-Gen sowie die 3'-nicht-codierende Region entspricht den Nukleotiden 2019 bis 4107 des Adenovirus Typ 5. Es ist jedoch auch im Rahmen der vorliegenden Erfindung, dass das E1B55kD-Gen mittels der Restriktionsenzyme Bam HI und BfrI bzw. XbaI aus diesem Plasmid geschnitten wird und anschließend in den Adenovirus eincloniert wird. Es ist auch im Rahmen der vorliegenden Erfindung, dass auch Anlaoge davon und insbesondere Analoge der 3'UTR-Region im Rahmen der vorliegenden Erfindung verwendet werden. Unter einem Analogon zu der 3' UTR-Region wird eine jegliche Sequenz verstanden, die die gleiche Wirkung entfaltet wie die 3' UTR-Region, insbesondere die gleiche Wirkung bei der Expression eines Gens, bevorzugterweise des E1B55cD-Gens. Derartige Analoga können durch die Fachleute auf dem Gebiet im Rahmen von Routineversuchen bestimmt werden, bspw. dadurch, dass die 3'- UTR-Region um ein oder mehrere Nukleotide verlängert und verkürzt wird und dann überpru'ft wird, ob das solchermassen erhaltene Analogon noch die gleiche Wirkung aufweist wie die 3'-UTR-Region, wie zuvor beschrieben. In einer Ausführungsform umfasst der Begriff der 3'-UTR-Region somit auch eine jegliches Analogon dazu.

Es ist im Rahmen der vorliegenden Erfindung, dass, sofern nichts Gegenteiliges angegeben ist, für den Fall, dass von den erfindungsgemäßen Viren gesprochen wird, dies sowohl die dafür codierende Nukleinsäuren, wie auch die adenoviralen Partikel, bevorzugterweise einschließlich der entsprechenden Nukleinsäure bezeichnet. Die entsprechende Nukleinsäure kann im Übrigen auch in einem anderen Vektor integriert vorliegen.

Hinsichtlich der Eigenschaft der Zellen, zu deren Lyse die hierin beschriebenen Adenoviren erfindungsgemäß verwendet werden, ist vorgesehen, dass diese in einer Ausführungsform eine Resistenz, bevorzugterweise eine Mehrfach- oder Vielfach-Resistenz zeigen. Resistenz, wie hierin verwendet, bezeichnet dabei bevorzugterweise eine Resistenz gegen die hierin beschriebenen Zytostatika. Diese Mehrfach-Resistenz geht bevorzugterweise mit der Expression, bevorzugter Weise einer Überexpression, des membranständigen Transportproteins P-Glycoprotein einher, welches als Marker für die Bestimmung entsprechender Zellen und damit auch von diesem aufweisenden Tumoren bzw. entsprechende Patientengruppen herangezogen werden kann. Der Begriff Resistenz, wie er hierin verwendet wird, umfasst dabei sowohl die auch als klassische Resistenz bezeichnete, durch das P-Glycoprotein vermittelte Resistenz, wie auch die auch als atypische Resistenz bezeichnete Resistenz, die durch MRP vermittelte oder andere, nicht-P-Glycoprotein vermittelte Resistenzen umfasst. Ein weiterer Marker, der mit der Expression von YB-1 korreliert, ist die Topoisomerase II alpha. Insoweit kann in einem Screenen-Verfahren, um zu bestimmen, ob ein Patient mit den Adenoviren erfindungsgemäß mit Aussicht auf Erfolg behandelt werden kann, an Stelle von der bzw. in Ergänzung zur Bestimmung von YB-1 im Kern die Expression von Topoisomerase II alpha herangezogen werden. Ein Marker, der grundsätzlich ähnlich wie das P-Glycoprotein verwendet werden kann, ist MRP. Ein weiterer Marker, zumindest in dem Umfang, als dass colorectrale Karzinomzellen oder Patienten mit einem Colorectalcarcinom betroffen sind, ist PCNA (engl. proliferating cell nuclear antigen (Hasan S. et al., Nature, 15, 387-391, 2001.), wie beispielsweise beschrieben von Shibao K. et al. (Shibao K et al., Int. Cancer, 83, 732-737, 1999). Schließlich ist, zumindest für den Bereich der Brustkrebs- und Osteosarcoma-Zellen die Expression von MDR (engl. multiple drug resistance) ein Marker im vorstehend beschriebenen Sinne (Oda Y et al., Clin. Cancer Res., 4, 2273-2277, 1998). Ein weiterer möglicher Marker, der erfindungsgemäß verwendet werden kann, ist p73 (Kamiya, M., Nakazatp, Y., J Neurooncology 59, 143-149 (2002); Stiewe et al., J. Biol. Chem., 278, 14230-14236, 2003).

Es ist somit ein besonderer Vorteil der vorliegenden Erfindung, dass auch jene Patienten unter Anwendung der erfindungsgemäßen Verwendung der Adenoviren, wie hierin beschrieben, therapiert werden können, die ansonsten im medizinisch-klinischen Sinne als "austherapiert" gelten und somit eine weitgehende Behandlung der Tumorerkrankung nach den Methoden des Standes der Technik mit Aussicht auf Erfolg nicht mehr möglich ist, insbesondere die Verwendung von Zytostatika sinnvoller Weise nicht mehr möglich ist bzw. nicht mehr erfolgreich durchgeführt werden kann im Sinne einer Beeinflussung bzw. Verringerung des Tumors. Der Begriff des Tumors bezeichnet hierin allgemein auch eine jegliche Tumor- oder Krebserkrankung, die entweder inhärent YB-1 im Zellkern enthält oder aber durch Realisieren exogener Maßnahmen, wie hierin offenbart, YB-1 im Zellkern, bevorzugterweise unabhängig vom Zellzyklus, aufweist.

Weiterhin können die hierin beschriebenen Viren zur Behandlung grundsätzlich von Tumoren verwendet werden.

Die Tumoren, die durch die hierin beschriebenen Viren insbesondere behandelt werden können, sind bevorzugterweise jene Tumoren, die aus der Gruppe ausgewählt sind, die Tumore des Nervensystems, okulare Tumoren, Tumore der Haut, Tumore der Weichteile, gastrointestinale Tumore, Tumore des respiratorischen Systems, Tumore des Skeletts, Tumore des endokrinen Systems, Tumore des weiblichen Geschlechtssystem, Tumore der Mammadrüse, Tumore des männlichen Geschlechtssystems, Tumore des harnableitenden Systems, Tumore des haematopoetischen Systems sowie vermischte und embryonale Tumore umfasst. Dabei ist es im Rahmen der vorliegenden Erfindung, dass es sich bei diesen Tumoren insbesondere um resistente Tumoren handelt, wie insbesondere hierin definiert.

Die Gruppe der Tumore des Nervensystems umfasst dabei bevorzugterweise:

1. Tumore der Schädelhöhle sowie des Gehirns (Intracranium), bevorzugtererweise Astrozytom, Oligodendrogliom, Menigiom, Neuroblastom, Ganglioneurom, Ependymom, Schwannom, Neurofibrom, Hemangioblastom,Lipom, Craniopharyngiom, Teratom und Chordom;

2. Tumore des Rückenmarkes und des Wirbelkanals, bevorzugtererweise Glioblastom, Meningiom, Neuroblastom, Neurofibrom, Osteosarkom, Chondrosarkom, Hemangiosarkom, Fibrosarkom und Multiple Myelome; und

3. Tumore der peripheren Nerven, bevorzugtererweise Schwannom, Neurofibrom, Neurofibrosarkom und Perineurale Fibroblastome.

Die Gruppe der okularen Tumore umfasst dabei bevorzugterweise:

1. Tumore der Augenlider und der Liddrüsen, bevorzugtererweise Adenom, Adenokarzinom, Papillom, Histiozytom, Mastzelltumor, Basalzelltumor, Melanom, Plattenepithelkarzinom, Fibrom und Fibrosarkom;

2. Tumore der Konjunktiven und der Nickhaut, bevorzugtererweise Plattenepithelkarzinom, Hemangiom, Hemangiosarkom, Adenom, Adenokarzinom, Fibrosarkom, Melanom und Papillom; und

3. Tumore der Orbita, des Nervus opticus und des Augapfels, bevorzugtererweise Retinoblastom, Osteosarkom, Mastzelltumor, Meningiom, Retikulozelltumor, Gliom, Schwannom, Chondrom, Adenokarzinom, Plattenepithelkarzinom, Plasmazelltumor, Lymphom, Rhabdomyosarkom und Melanom.

Die Gruppe der Tumore der Haut umfasst dabei bevorzugterweise:

Tumore des Histiocytom, Lipom, Fiborsarlcom, Fibrom, Mastzell Tumor, Malignes Melanom, Papillom, Basalzelltumor, Keratoakanthom, Hemangioperizytom, Tumore der Haarfollikel, Tumore der Schweißdrüsen, Tumore der Talgdrüsen, Haemangiom, Haemangiosarkom, Lipom, Liposarkom, Malignes fibröses Histiozytom, Plasmazytom und Lymphangiom.

Die Gruppe der Tumore der Weichteile umfasst dabei bevorzugterweise:

Tumore des alveoläres Weichteilsarkom, epitheloidzelliges Sarkom, Chondrosarkom der Weichteile, Osteosarkom der Weichteile, Ewing-Sarlcom der Weichteile, primitive neuroektodermale Tumore (PNET), Fibrosarkom, Fibrom, Leiomyosarkom, Leiomyom, Liposarkom, malignes fibröses Histiozytom, malignes Hemangioperizytom, Hemangiom, Hemangiosarkom, malignes Mesenchymom, maligner peripherer Nervenscheidentumor (MPNST, malignes Schannom), malignes melanozytisches Schwannom, Rhabdomyosarkom, Synovialsarkom, Lymphangiom und Lymphangiosarkom.

Die Gruppe des Gastrointestinale Tumors umfasst dabei bevorzugterweise:

1. Tumore der Mundhöhle und Zunge, bevorzugtererweise Plattenepithelkarzinom, Fibrosarkom, Merkelzelltumor, Induktives Fibroameloblastom, Fibrom, Fibrosarkom, Virale Papillomatose, Idiopathische Papillomatose, Nasopharyngeale Polypen, Leiomyosarkom, Myoblastom und Mastzelltumor;

2. Tumore der Speicheldrüsen, bevorzugtererweise Adenokarzinom;

3. Tumore des Oesophagus, bevorzugtererweise Plattenepithelkarzinom, Leiomyosarkom, Fibrosarkom, Osteosarkom, Barrettkarzinom und paraoesophageale Tumore;

4. Tumore der exocrines Pankreas, bevorzugtererweise Adenokarzinom; und

5. Tumore des Magens, bevorzugtererweise Adenokarzinom, Leiomyom, Leiomyosarkom und Fibrosarkom.

Die Gruppe der Tumore des respiratorischen Systems umfasst dabei bevorzugterweise:

1. Tumore der Nase und Nasenhöhle, des Kehlkopfes und der Trachea, bevorzugtererweise Plattenepithelkarzinom, Fibrosarkom, Fibrom, Lymphosarkom, Lymphom, Hemangiom, Hemangiosarkom, Melanom, Mastzelltumor, Osteosarkom, Chondrosarkom, Oncozytom (Rhabdomyom), Adenokarzinome und Myoblastom; und

2. Tumore der Lunge, bevorzugtererweise Plattenepithelkarzinom, Fibrosarkom, Fibrom, Lymphosarkom, Lymphom, Hemangiom, Hemangiosarkom, Melanom, Mastzelltumor, Osteosarkom, Chondrosarkom, Oncozytom (Rhabdomyom), Adenokarzinome, Myoblastom, kleinzelliges Karzinom, nicht-kleinzelliges Karzinom, bronchiales Adenokarznom, bronchoalveoläres Adenokarzinom und alveoläres Adenokarzinom.

Die Gruppe der Tumore des Skeletts umfasst dabei bevorzugterweise:

Osteosarkom, Chondrosarkom, Parosteales Osteosarkom, Hemangiosarkom, Synovialzellsarkom, Hemangiosarköm, Fibrosarkom, Malignes Mesenchymom, Riesenzelltumor, Osteom und multilobulares Osteom.

Die Gruppe der Tumore des endokrinen Systems umfasst dabei bevorzugterweise:

1. Tumore der Schilddrüse/Nebenschilddrüse, bevorzugtererweise Adenome und Adenokarzinome;

2. Tumore der Nebenniere, bevorzugtererweise Adenom, Adenokarzinom und Pheochromozytom (Nebennierenmarktumor);

3. Tumore der Hypothalamus/Hypophyse, bevorzugtererweise Adenom und Adenokarzinom;

4. Tumore des endokrines Pankreas, bevorzugtererweise Insulinom (Betazelltumor, APUDom) und Zollinger-Ellison Syndrom (Gastrin sezernierender Tumor der Deltazellen des Pankreas); und

5. sowie multiple endokrine Neoplasien (MEN) und Chemodectom.

Die Gruppe der Tumore des weiblichen Geschlechtssystems umfasst dabei bevorzugterweise:

1. Tumore der Ovarien, bevorzugterweise Adenom, Adenokarzinom, Zystadenom, und undifferenzierte Karzinome;

2. Tumore der Uterus, bevorzugtererweise Leiomyom, Leiomyosarkom, Adenom, Adenokarzinom, Fibrom, Fibrosarkom und Lipom;

3. Tumore der Zervix, bevorzugtererweise Adenokarzinom, Adenom, Leiomyosarkom und Leiomyom;

4. Tumore der Vagina und Vulva, bevorzugtererweise Leiomyom, Leiomyosarkom, Fibroleiomyom, Fibrom, Fibrosarkom, Polypen und Plattenepithelkarzinom.

Die Gruppe der Tumore der Mammadrüse umfasst dabei bevorzugterweise:

Fibroadenom, Adenom, Adenokarzinom, mesenchymale Tumore, Karzinome, Karzinosarkome.

Die Gruppe der Tumore des männlichen Geschlechtssystems umfasst dabei bevorzugterweise:

1. Tumore der Hoden, bevorzugtererweise Seminom, Interstitialzelltumor und S ertolizelltumor;

2. Tumore der Prostata, bevorzugtererweise Adenokarzinome, undifferenzierte Karzinome, Plattenepithelkarzinome, Leiomyosarkome und Transitionalzellkarzinom; und

3. Tumore des Penis und der externen Genitalien, bevorzugtererweise Mastzelltumor und Plattenepithelkarzinom.

Die Gruppe der Tumore des harnableitenden Systems umfasst dabei bevorzugterweise:

1. Tumore der Niere, bevorzugtererweise Adenokarzinom, Transitionalzellkarzinom (epitheliale Tumore), Fibrosarkom, Chondrosarkom (mesenchymale Tumore), Wilm's Tumor, Nephroblastom und embryonales Nephrom (embryonal pluripotente Blastome);

2. Tumore der Urether, bevorzugtererweise Leiomyom, Leiomyosarkom, Fibropapillom, Transitionalzellkarzinom;

3. Tumore der Harnblase, bevorzugtererweise Transitionalzellkarzinom, Plattenepithelkarzinom, Adenokarzinom, Botryoid (Embryonales Rhabdomyosarkom), Fibrom, Fibrosarkom, Leiomyom, Leiomyosarkom, Papillom und Hemangiosarko; und

4. Tumore der Urethra, bevorzugtererweise Transitionalzellkarzinom, Plattenepithelkarzinom und Leiomyosarlcom.

Die Gruppe der Tumore des hämatopoetischen Systems umfasst dabei bevorzugterweise:

1. Lymphome, Lymphatische Leukämie, Nichtlymphatische Leukämie, Myeloproliferative Leukämie, Hodgkin's Lymphom, Non-Hodgkin's Lymphom.

Die Gruppe der vermischten und embryonalen Tumore umfasst dabei bevorzugterweise:

Hemangiosarkome, Thymom und Mesotheliom.

Tumoren sind ausgewählt aus der Gruppe, die Brustkrebs, Ovarialkarzinom, Prostatakarzinom, Osteosarkom, Glioblastom, Melanom, kleinzelliges Lungenkarzinom und Kolorektalkarzinom umfasst. Weitere Tumoren sind jene, die resistent, wie hierin beschrieben, sind, bevorzugt Gene, die mehrfach resistent sind, insbesondere auch solche Tumoren der vorstehend beschriebenen Gruppe.

Beschrieben wird auch ein Verfahren zum Screenen von Patienten, die mit einem modifizierten Adenovirus, d.h. einem Adenovirus, wie er erfindungsgemäß verwendet wird, wie beispielsweise AdΔ24, d1922-947, E1Ad/01/07, CB016 oder die im europäischen Patent EP 0931 830 beschriebenen Viren, behandelbar sind, wobei das Verfahren die folgenden Schritte umfasst:

- Untersuchen einer Probe des Tumorgewebes und
- Festellen, ob YB-1 im Kern Zellzyklus-unabhängig lokalisiert ist.

Anstelle von oder in Ergänzung zu YB-1 kann auch das Vorhandensein der vorstehend beschriebenen Marker festgestellt werden.

In dem Falle, dass das Tumorgewebe oder ein Teil davon YB-1 im Zellkern, insbesondere Zellzyklus-unabhängig, lokalisiert aufweist, können die hierin offenbarten Adenoviren erfindungsgemäß verwendet werden.

Es ist vorgesehen, dass die Untersuchung des Tumorgewebes unter Verwendung eines Mittels erfolgt, das ausgewählt ist aus der Gruppe, die Antikörper gegen YB-1, Aptamere gegen YB-1, Spiegelmere gegen YB-1 sowie Anticaline gegen YB-1 umfasst. Grundsätzlich die gleichen Mittel können für die entsprechenden Marker hergestellt bzw. verwendet werden. Die Herstellung von Antikörpern, insbesondere monoklonalen Antikörpern, ist den Fachleuten auf dem Gebiet bekannt. Ein weiteres Mittel zum spezifischen Nachweis von YB-1 oder den Markern stellen Peptide dar, die mit hoher Affinität an ihre Zielstrukturen, im vorliegenden Falle YB-1 oder die besagten Marker, binden. Im Stand der Technik sind Verfahren bekannt, wie beispielsweise phage-display, um derartige Peptide zu erzeugen. Dabei wird typischerweise von einer Peptid-Bibliothek ausgegangen, wobei die einzelnen Peptide eine Länge von etwa 8 bis 20 Aminosäuren aufweisen und die Größe der Bibliothek etwa $10^2$ bis $10^{18}$, bevorzugterweise $10^8$ bis $10^{15}$ verschiedene Peptide beträgt. Eine spezielle Form von an Zielmolekülen bindenden Polypeptiden stellen die sogenannten Anticaline dar, wie sie beispielsweise in der deutschen Patentanmeldung DE 197 42 706 beschrieben sind.

Ein weiteres Mittel zum spezifischen Binden an YB-1 oder die entsprechenden, hierin offenbarten Marker und damit zum Nachweis einer Zellzyklus-unabhängigen Lokalisation von YB-1 im Zellkern sind die sogenannten Aptamere, d. h. D-Nukleinsäure, die auf RNA- oder DNA-Basis entweder als Einzelstrang oder als Doppelstrang vorliegen und spezifisch an ein Zielmolekül binden. Die Herstellung von Aptameren ist beispielsweise beschrieben im europäischen Patent EP 0 533 838. Eine Sonderform der Aptamere stellen die sogenannten Aptazyme dar, die beispielsweise beschrieben sind von Piganeau, N. et al. (2000), Angew. Chem. Int. Ed., 39, Nr. 29, Seiten 4369 - 4373. Dabei handelt es sich um eine spezielle Ausführungsform von Aptameren insoweit, als dass sie neben dem Aptameranteil noch einen Ribozymanteil aufweisen und nach Bindung oder Freisetzung des an den Aptamerteil bindenden Zielmoleküls der Ribozymanteil katalytisch aktiv wird und ein Nukleinsäuresubstrat spaltet, was mit der Erzeugung eines Signals einhergeht.

Eine weitere Form der Aptamere stellen sogenannte Spiegelmere dar, d. h. zielmolekülbindende Nukleinsäuren, die aus L-Nulcleinsäuren hergestellt sind. Das Verfahren zur Herstellung dieser Spiegelmere ist beispielsweise beschrieben in WO 98/08856.

Die Probe des Tumorgewebes kann dabei durch Punktion oder durch einen chirurgischen Eingriff erhalten werden. Die Feststellung, ob im Kern YB-1 Zellzyklus-unabhängig lokalisiert ist, erfolgt dabei häufig unter Verwendung mikroskopischer Techniken und/oder mittels Immunhistoanalyse, typischerweise unter Verwendung von Antikörpern oder einem der weiteren vorstehenden Mitteln. Weitere Verfahren zum Nachweis, dass YB-1 im Kern und insbesondere dort Zellzyklus-unabhängig lokalisiert ist, sind dem Fachmann bekannt. Beispielsweise kann bei dem Durchmustern von gegen YB-1 gefärbten Gewebeschnitten die Lokalisation von YB-1 leicht erkannt werden. Dabei ergibt sich bereits infolge der Häufigkeit des Auftretens von YB-1 im Kern, dass es sich um eine Zellzyklus-unabhängige Lokalisation im Kern handelt. Eine weitere Möglichkeit zum Zellzyklus-unabhängigen Nachweis von YB-1 im Kern besteht in der Durchführung einer Färbung gegen YB-1 und Feststellen, ob YB-1 im Kern lokalisiert ist, und Durchführung der Bestimmung des Zellstadiums der Zellen. Dies bzw. die Detektion von YB-1 kann aber auch unter Verwendung der vorstehend genannten, gegen YB-1 gerichteten Mittel erfolgen. Der Nachweis der Mittel erfolgt dabei durch Verfahrensweisen, die den Fachleuten auf dem Gebiet bekannt sind. Dadurch, dass die besagten Mittel spezifisch gegen YB-1 gerichtet sind und insoweit nicht an andere Strukturen innerhalb der zu untersuchenden Probe, insbesondere der Zellen, binden, kann durch eine geeignete Markierung der Mittel deren Lokalisierung und infolge der spezifischen Bindung an YB-1 auch die Lokalisierung von YB-1 entsprechend nachgewiesen und festgestellt werden. Verfahren zum Markieren der Mittel sind den Fachleuten auf dem Gebiet bekannt.

Es ist im Rahmen der vorliegenden Erfindung, dass die hierin beschriebenen Viren, seien es die erfindungsgemäßen Viren oder die gemäß der Erfindung zu verwendenden Viren, auch bei Erkrankungen, bevorzugterweise Tumorerkrankungen und bevorzugtererweise Tumorerkrankungen, bei denen zumindet ein Teil der Tumorzellen eine Mehrfach-Resistenz, insbesondere eine multidrug resistance aufweist, verwendet werden können, bei denen YB-1 dereguliert vorliegt. Dies gilt auch für einen jeden anderen Aspekt, wie er hierin im Zusammenhang mit Zellen und Tumoren beschrieben ist, soweit sie sich auf Zellen und Erkrankungen bezieht, bei denen sichYB-1 im Zellkern befindet, bevorzugterweise unabhängig vom Zellzyklus im Kern befindet.

Die vorliegende Erfindung soll im folgenden anhand der Figuren und Beispiele weiter veranschaulicht werden, wobei sich daraus neue Merkmale, Ausführungsformen und Vorteile der Erfindung ergeben. Dabei zeigt

Fig. 1 den strukturellen Aufbau der darin als AdE1/E3-minus Adenovirus bezeichneten adenoviralen Vektoren, die E1/E3-deletierte Adenoviren sind, Wildtyp-Adenovirus und Adenovirus d1520;

Fig. 2 die Bindungsdomänen des E1A-Proteins hinsichtlich der Bindung von p300, p107 und p105;

Fig. 3 U2OS-Zellen, welche nicht YB-1 im Kern aufweisen, nach Infektion mit dem darin als E1/E3-minus Ad5 bezeichneten E1/E3-deletierten Adenoviren Ad5 und d1520;

Fig. 4 257RDB-Zellen, welche YB-1 im Kern aufweisen, nach Infektion mit dem darin als E1/E3-minus Ad5 bezeichneten E1/E3-deletierten Adenoviren Ad5 und Adenovirus d1520;

Fig. 5 257RDB-Zellen und U2OS-Zellen, nach Infektion mit dem Adenovirus dl1119/1131;

Fig. 6 das Ergebnis einer EMSA-Analyse, womit belegt wird, dass YB-1 in den vielfachresistenten Zellen bzw. Zelllinien 257RDB, 181 RDB, MCF-7Ad- im Zellkern vorhanden ist, wohingegen YB-1 in U2OS und HeLa-Zellen nicht im Kern vorhanden ist;

Fig. 7 den strukturellen Aufbau des E1A-Proteins vom Wildtyp-Adenovirus, von Adenovirus d1520 und Adenovirus dl1119/1131;

Fig. 8 ein Balkendiagramm, welches die Replikationseffizienz von Adenoviren bei Anwesenheit zusätzlich exprimierter viraler Proteine in Absolutzahlen zeigt; und

Fig. 9 ein Balkendiagramm, welches die Steigerung der Replikationseffizienz von Adenoviren bei Anwesenheit zusätzlich exprimierter viraler Proteine zeigt;

Fig. 10 mit U2OS-Zellen bewachsene Näpfen nach Kristallviolett-Färbung und Infektion mit dl1520 mit 10 bzw. 30 pfu/Zelle bzw. Kontrolle (K) ohne Gabe von Daunorubicin bzw. mit Gabe von 40 ng pro ml Daunorubicin;

Fig. 11 mit HeLa-Zellen bewachsene Näpfen nach Kristallviolett-Färbung und Infektion mit d1520 mit 10 bzw. 30 pfu/Zelle bzw. Kontrolle (K) ohne Gabe von Daunorubicin bzw. mit Gabe von 40 ng pro ml Daunorubicin;

Fig. 12 ein Diagramm des Tumorvolumens über die Zeit von Tumoren verschiedenen Ursprungs (RDB257 und HeLa) nach Behandlung mit PBS bzw. d1520;

Fig. 13 Aufnahmen von euthanasierten Mäusen, die einen Tumor auf der Grundlage von RDB257-Zellen entwickelten, nach Behandlung mit PBS bzw. mit 5 x $10^8$ pfu d1520;

Fig. 14 das Ergebnis einer Southern Blot-Analyse eines Zellextraktes (von den sucutan gewachsenen Tumoren) von RDB257-Zellen und HeLa-Zellen nach Infektion mit dl520;

Fig. 15 ein Balkendiagramm, welches die Replikationseffizienz bzw. die Partikelbildung von d1520 und Wildtyp-Adenovirus in YB-1 Kern-positiven Tumorzellen (257RDB und 181RDB) und YB-1 Kern-negativen Tumorzellen (HeLa, U2OS) zeigt;

Fig. 16 den strukturellen Aufbau des Adenovirus vom Wildtyp und des adenoviralen Vektors AdXvir03;

Fig. 17 den strukturellen Aufbau des adenoviralen Vektors AdXVir03/01;

Fig. 18AB mit 181RDB-Zellen (Fig. 18A) und 272RDB-Zellen (Fig. 18B) bewachsene Näpfe nach Kristallviolett-Färbung und Infektion mit Ad312 (20 pfu/Zelle), Xvir03 (5 pfu/Zelle) und Kontrolle (nicht infiziert), wobei die Krisstallviolettfärbung fünf Tage nach Infektion erfolgte;

Fig. 19 das Ergebnis einer Southern Blot-Analyse des Replikationsverhaltens von Adenovirus dl 520 in U373 Zellen mit und ohne Behandlung der Zellen mit Irinotecan;

Fig. 20 das Ergebnis einer Southern Blot-Analyse des Replikationsverhaltens von Adenovirus dl 520 in U373 Zellen mit und ohne Behandlung der Zellen mit Trichostatin A;

Fig. 21 das Ergebnis einer FACS-Analyse von mit Trichostatin behandelten U 373 Zellen betreffend die Expression des Coxsackievirus-Adenovirus-Rezeptors (CAR), ausgedrückt als prozentualer Anteil der CAR-positiven Zellen; und

Fig. 22 vier verschiedene Panele von Zellrasen zur Darstellung der Wirkung von replizierendem Adenovirus dl520 und Irinotecan und Trichostatin in verschiedenen Kombinationen;

Fig. 23 eine schematische Darstelluing des ORF von E1B 55K mit dem 3'UTR-Fragment und der Restriktinosschnittstelle Bfr I an Position 3532; und

Fig. 24 die Sequenz der E1B55k-3'UTR Region entsprechend Sequenzposition 3507 bis 4174 von Wildtyp Ad 5; und

**Beispiel 1: Strukturen von E1A-Modifikationen, wie sie von den erfindungsgemäß verwendeten Adenoviren aufgewiesen werden können**

Fig. 1 zeigt den strukturellen Aufbau der adenoviralen Vektoren AdE1/E3-minus, d.h. des E1/E3-deletieren Adenovirus, Wildtyp-Adenovirus und Adenovirus dl520.

Das Adenovirus AdE1/E3-minus weist keine funktionalen für E1A und keine für E1B und keine für E3 codierenden Bereiche auf und dient im Rahmen der durchgeführten Experimente als Toxizitätskontrolle

Das Wildtyp-E1A-Gen kodiert für insgesamt 5 Proteine, die durch alternatives splicing der RNA von E1A hervorgehen. Dabei werden unter anderem zwei unterschiedliche Proteine, nämlich ein 289 Aminosäuren großes Protein und ein 243 Aminosäuren großes Protein hergestellt. D1520 kodiert nicht für das 289 Aminosäure große Protein, da es eine Deletion im CR3-Bereich des E1A-Gens aufweist, das zu einem Fehlen des 13S-Genproduktes führt. Der erfindungsgemäß verwendbare Adenovirus dl520 wird unter Fachleuten als 12S-E1A Virus bezeichnet. Der im Stand der Technik bekannte Adenovirus dl347 (Wong und Ziff, J. Virol., 68, 4910-4920, 1994) ist ebenfalls ein 12S-E1A Virus, der erfindungsgemäß verwendet werden kann.

Innerhalb des 289 Aminosäuren großen Proteins, welches von 13S-E1A mRNA kodiert wird, gibt es 3 Bereiche, die bei den unterschiedlichen adenoviralen Subtypen konserviert vorliegen. Diese werden als CR1, CR2 und CR3 bezeichnet. Während CR1 und CR2 bei beiden E1A-Proteinen (E1A 12S und E1A 13S) vorkommt, d. h. sowohl bei dem 289 Aminosäure langen wie auch bei dem 243 Aminosäure langen Protein, ist der CR3-Bereich nur bei dem größeren der beiden vorstehend genannten Proteine zu finden.

Der CR3-Bereich wird für die Aktivierung der viralen Gene, insbesondere von E1B, E2, E3 und E4 benötigt. Viren, die nur das kleinere, 243 Aminosäuren lange Protein aufweisen, transaktivieren nur sehr schwach die viralen Gene und führten keine adenovirale Replikation in solchen Zellen durch, die YB-1 nicht im Kern aufweisen. Da YB-1 nur in Tumorzellen im Kern vorliegt bzw. nachweisbar ist, eignet sich dieser Vektor, um eine tumorspezifische Replikation zu induzieren.

Durch die Deletion von CR3 in dl520 ist dieser Adenovirus nicht in der Lage, zelluläres YB-1 in den Zellkern zu translokalisieren, hierin auch als Translozieren bezeichnet, und somit auch nicht in der Lage, in YB-1-Kern-negativen Zellen zu replizieren und stellt damit einen der erfindungsgemäß zu verwendenden Viren dar, wobei dieser Virus die erfindungsgemäß erforderliche Transaktivierung aufweist.

**Beispiel 2: Wirkmechanismus von Adenoviren in Abhängigkeit des Rb-Status von Zellen**

In Fig. 2 sind die Bildungsdomänen des E1A-Proteins hinsichtlich der Bindung von p300, p107 und p105 dargestellt. P300 ist dabei ebenso wie p107 ein zelluläres Bindungsprotein. Die Bindung des Retinoblastoma-Proteins (pRb), ein Tumorsuppressor-Protein, erfolgt über CR1 und CR2. Studien haben gezeigt, dass pRb und p107/p300 in Verbindung mit dem zellulären Transkriptionsfaktor E2F eine transkriptionelle Regulation ausüben. Das Wildtyp E1A-Protein unterbricht die Bindung von E2F an Rb. Das solchermaßen freigesetzte E2F bindet an den E2 early Promotor und induziert dadurch die adenovirale Replikation.

Es ist im Stand der Technik bekannt, dass bestimmte Deletionen im E1A-Onkoprotein dazu führen, dass rekombinante adenovirale Vektoren wie die nachstehend genannten vornehmlich in Rb-negativen Zellen zur Replikation befähigt ist und erfindungsgemäß verwendet werden können. Zum Beispiel weist der adenovirale Vektor dl922-947 eine Deletion in der CR2-Region auf (Aminoäurepositionen 122-129) und der Vektor CB016 Deletionen in den Bereichen CR1 (Aminoäurepositionen 27-80) und CR2 (Aminoäurepositionen 122-129). Der Vektor E1A*dl*01/07 weist eine Deletion im CR2-Bereich auf (Aminoäurepositionen 111-123). Durch eine zusätzliche Deletion am N-Terminus (Aminoäurepositionen 4-25), erfolgt zudem keine Bindung an das Protein p300. Der adenovirale Vektor AdΔ24 weist eine Deletion in der CR2-Region auf (Aminoäurepositionen 120-127). Der im Patent EP 0 931 830 beschriebene adenovirale Vektor weist Deletionen in dem CR1 und CR2-Bereich auf.

Der Bindungsmechanismus von E2F/RB und die durch E1A vermittelte Freisetzung von E2F ist grundlegend verschieden von dem der vorliegenden Erfindung zugrunde liegenden Mechanismus. Nicht die Freisetzung von E2F vom Rb-Protein ist, wie im Stand der Technik angenommen, ein wichtiger, um nicht zu sagen der entscheidende Vorgang der adenoviralen Replikation, sondern die Kernlokalisation des humanen Transkriptionsfaktors YB-1. Dieser Transkriptionsfaktor kommt in normalen Zellen über den größten Teil des Zellzyklus lediglich im Zytoplasma vor. Nach Infektion mit einem Adenovirus wird dieser unter bestimmten Bedingungen in den Kern induziert oder liegt bei bestimmten zellulären Systemen wie bestimmten Tumorerkrankungen, wie z.B. aber nicht darauf beschränkt, Brustkrebs, Ovarialkarzinom, Prostatakarzinom, Osteosarkom, Glioblastom, Melanom, kleinzelliges Lungenkarzinom und Kolorektalkarzinom, bereits im Kern vor.

**Beispiel 3: Infektion von U2OS-Zellen**

Pro Schale wurden 100.000 U2OS-Zellen ausplattiert. Am nächsten Tag wurden die Zellen wie in Fig. 3 dargestellt mit den verschiedene Adenoviren infiziert. Die Infektion erfolgte in 500 μL serumfreie DMEM-Medium für 1 h bei 37° C. Anschließend wurde das Infektionsmedium entfernt und durch 2 ml Vollmedium (10 % FKS/DMEM) ersetzt. Nach 3 Tagen erfolgte die Auswertung mit Hilfe einer Kristallviolettfärbung.

Wie aus Fig. 3 ersichtlich zeigen die U2OS Zellen, welche YB-1 nicht im Kern aufweisen, nach Infektion mit zwei verschiedenen Adenoviren, nämlich dem als E1/E3-minus bezeichneten E1/E3-deletierten Adenovirus und Adenovirus d1520, der erfindungsgemäß verwendet werden kann, keine Lyse, wie durch Kristallviolettfärbung der Zellen dargestellt. Dabei wird zunächst das Medium entfernt. Anschließend werden die Zellen mit Kristallviolett überschichtet (50 % ETOH, 3 % Formaldehyd, 5 % Essigsäure, 1 % Kristallviolett) und für 5-10 min bei Raumtemperatur inkubiert. Danach werden die Platten mit 6 Näpfen mit Wasser gründlich gewaschen und bei Raumtemperatur getrocknet.

Dies bestätigt die der vorliegenden Erfindung zugrundeliegende Erkenntnis, dass das Vorhandensein von YB-1 erforderlich ist, um die erfindungsgemäß zu verwendenden Viren zu einer Lyse von infizierten Zellen zu veranlassen.

**Beispiel 4: Infektion von 257RDB-Zellen**

Pro Schale wurden 100.000 257RDB-Zellen ausplattiert. Am nächsten Tag wurden die Zellen wie in Fig. 4 dargestellt mit den verschiedene Adenoviren infiziert. Die Infektion erfolgte in 500 μL serumfreiem DMEM-Medium für 1 h bei 37° C. Anschließend wurde das Infektionsmedium entfernt und durch 2 ml Vollmedium (10 % FKS/DMEM) ersetzt. Nach 3 Tagen erfolgte die Auswertung mit Hilfe einer Kristallviolettfärbung.

Das Ergebnis dieses Versuchs ist in Fig. 4 dargestellt. Das Adenovirus als E1/E3-minus Ad5 bezeichnete E1/E3-deletierte Adenovirus zeigt keine Lyse bei niedrigen MOIs (pfu/Zelle) bei Infektion von 257RDB-Zellen, die YB-1 im Kern aufweisen. Im Gegensatz dazu zeigt d1520, welcher wie in Beispiel 3 belegt, in YB-1-Kern-negativen Zellen nicht repliziert und gleichzeitig mit E1A für ein im Sinne der vorliegenden Erfindung transaktivierendes Onkogenprotein codiert, eine praktisch vollständige Lyse bei einer MOI (engl. multiplicity of infection) von 40 pfu pro Zelle und eine noch überwiegende Lyse bei einer MOI von 10 pfu pro Zelle. Daraus ergibt sich, dass dl 520 und vergleichbare Viren, wie hierin beispielsweise mit dl1119/1131 oder AdXvir 03 beschrieben, eine gegenüber einem E1-deletierten oder einem E1/E3-deletierten Adenoviurs um etwa eine Größenordnung (etwa zehnfach) verringerte MOI erforderlich machen, was deren Eignung zur klinischen Anwendung begründet.

Wie in Fig. 7 dargestellt zeichnet sich das E1A-Protein von dl520 dadurch aus, dass der Bereich CR3 davon deletiert ist, was zu der für die erfindungsgemäße Verwendung des Adenovirus erforderlichen Transaktivierung und Replikation in YB-1-Kern-positiven Zellen führt.

**Beispiel:5: Infektion von 257RDB und U2OS-Zellen mit dl1119/1131**

Wie in Fig. 5 dargestellt kommt es bei Infektion von YB-1-Kern-negativen U2OS-Zellen mit dem Adenovirus dl1119/1131, das eine Deletion der Aminosäuren 4-138 des E1A-Proteins bzw. der dafür codierenden Nuldeinsäure und ein Stop-Codon nach Aminosäure 218 aufweist, wodurch das exprimierte verkürzte E1A-Protein die CR3-Region des vollständigen E1A-Proteins enthält, bei einer MOI von 20 pfu pro Zelle zu keiner Lyse. Als Negativkontrolle wurde ein nicht-infizierter Zellrasen herangezogen.

Im Gegensatz dazu zeigt sich unter dem Einfluss von Adenovirus dl1119/1131 in einem zellulären System wie 257RDB, welches YB-1 im Kern aufweist, d. h. YB-1-Kern-positiv ist, bereits bei einer MOI von 20 pfu pro Zelle eine praktisch vollständige Lyse des Zellrasens. Insoweit findet sich auch mit diesem Beispiel ein Beleg für die Aussage, dass ein modifiziertes E1A-Onkogenprotein, welches, wie in Fig. 7 dargestellt, beispielsweise lediglich den CR3-Bereich umfasst und dem der Bereich CR1 sowie CR2 fehlt, die für die erfindungsgemäße Verwendung von Adenoviren erforderliche Transaktivierung in YB-1-Kern-positiven Zellen zeigt, mit der Folge einer viralen Replikation. Der Adenovirus dl1119/1131 stellt somit einen weiteren, erfindungsgemäß verwendbaren Adenovirus dar. Dabei ist es im Rahmen der vorliegenden Erfindung, dass auch solche Viren verwendet werden können, die hinsichtlich des CR3-Bereiches wie dl1119/1131 ausgebildet sind, jedoch im Unterschied dazu den Bereich CR1 und/oder CR2 aufweisen.

**Beispiel 6: Nachweis von nuklärem YB-1 bei vielfachresistenten Zellen**

Dem Experiment liegt die Überlegung zugrunde, das nukläres YB-1 als Transkriptionsfaktor an die Y-Box (CAAT-Sequenz) innerhalb des mdr1-Promoters (engl. multiple drug resistance promoter) binden sollte. Um dies nachzuweisen, wurde eine sogenannte EMSA-Analyse (electrophoretic mobility shift assay) durchgeführt. Dabei wird Kernprotein isoliert und anschließend werden 1-10 $\mu$g Protein mit einem kurzen DNA-Fragment (Oligo) zusammen bei 37° C inkubiert. Um nukläres YB-1 zu bestimmen, wurde folgendes Oligonukleotid benutzt: *mdr1* promoter im Unterschied zu U20S (Position -86 bis -67): TGAGGCTGATTGGCTGGGCA (die Y-box ist unterstrichen).

Dieses DNA-Fragment wird zuvor mit einer Kinase am 5'-Ende mit $^{32}$P radiaktiv markiert. Anschließend erfolgt die Auftrennung in einem nativen Polyacrylamidgel. Falls das Protein YB-1 an einer Sequenz am Oligonucleotid bindet, ist dies zu erkennen, da ungebundenes Oligonukleotid im Gel schneller wandert als das gebundene Oligonucleotid (Holm, P. S. et al., JBC 277, 10427-10434, 2002; Bargou, R. C. et al., Nature Medicine 3, 447-450, 1997).

Wie in Fig. 6 dargestellt, konnte im Rahmen einer EMSA-Analyse gezeigt werden, dass YB-1 in den vielfachresistenten Zellen 257RDB, 181RDB und MCF-7Ad-Zellen im Kern im Gegensatz zu den Zelllinien U2OS und HeLa-Zellen vorhanden ist.

Die in Beispiel 4 und 5 gezeigten Ergebnisse belegen, dass die Adenoviren dl520 und dl1119/1131 in YB-1-Kern-positiven Zellen wie z. B. 257RDB im Unterschied zu U205 replizieren und eine Zellyse induzieren. Dies belegt somit die Aussage der erfindungsgemäßen Verwendung der Adenoviren. Weiterhin belegen die Ergebnisse, dass bereits eine schwache Transaktivierung der viralen Gene in YB-1- Kern-positiven Zellen im Vergleich zum Wildtyp-Adenovirus durch die modifizierten oder deletierten E1A-Genprodukte bei Anwesenheit von YB-1 im Zellkern erfolgreich mit einer Replikation und Lyse von derartigen Zellen einhergeht, einschließlich beispielsweise von vielfachresistenten Zellen, und somit die hierin beschriebenen Adenoviren bei der Lyse derartiger Tumoren verwendet werden können.

**Beispiel 7: Steigerung der Replikationsefrizienz von E1-minus Adenoviren**

In diesem Beispiel wird die Substitution der frühen viralen Gene E1B-55K und E4orf6 durch Transfektion mit dem Plasmid pE4orf6 und Infektion mit-dem E1/E3-deletierten Adenovirus Ad-55K gezeigt. Ad-55k ist ein E1/E3 deletiertes Virus, wobei E1B-SSk, in die E1 kloniert wurde und unter CMV-Kontrolle steht (Dobbelstein, M. et al., EMBO Journal, 16, 4276-4284, 1997) Diese Substitution wird mit Blick darauf erforderlich, dass AdYB-1, d. h. ein Adenovirus, der YB-1 exprimiert, diese frühen Gene nicht exprimiert und der vorliegende Erfinder erkannt hat, dass eine Substitution dieser frühen Gene in einem Replikationssystem, bei dem YB-1 im Kern vorhanden ist, in der Lage ist, die Replikationseffizienz bzw. die Partikelbildungseffizienz in einem Umfang vergleichbar derjenigen von Wildtyp-Adenoviren vom Typ Ad5 zu erhöhen.

Dabei wurde wie folgt vorgegangen:

Transfektion von je $10^5$ U2OS-Zellen mit dem Plasmid pE4orf6 mit Hilfe von Lipofectamin. Das Plasmid pE4orf6 trägt die für das frühe virale Gen E4orf6 codierende DNA-Sequenz unter CMV-Kontrolle.

24 h nach der Transfektion mit dem Plasmid pE4orf6 wurden die Zellen mit dem YB-1 exprimierenden E1/E3-deletierten Adenovirus AdYB-1 (50 pfu/Zelle) und dem E1/E3-deletierten E1B-55K Adenovirus Ad-55K (50 pfu/Zelle) infiziert. Ad-55K ist ein E1/E3-deletiertes Virus, welches als Transgen das virale Gen E1B-55K unter CMV-Kontrolle trägt.

Anschließend wurden die Zellen vom Medium (2ml) 5 Tage nach der Infektion (= post infectionem) entfernt. Die Freisetzung der viralen Partikel aus den isolierten Zellen erfolgte durch dreimaliges alternierendes Einfrieren und Auftauen (engl. thaw/freeze). Anschließend wurde ein Plaque Assay auf 293-Zellen zur Bestimmung der gebildeten infektiösen Partikel (plaque forming units pro ml (pfu/ml)) durchgeführt. Das Ergebnis ist in den Figs. 8 und 9 dargestellt. Dabei zeigt Figur 8 das Ergebnis des Plaque Assays, dargestellt in absoluten Zahlen. Die deutlichste Differenz zur Infektion mit AdYB-1 alleine zeigt hierbei die Kombination aus Transfektion mit dem Plasmid pE4orf6 und Co-Infelction mit den beiden Viren AdYB-1 und Ad-55K. Fig. 9 zeigt das Ergebnis von Fig. 8, wobei hier die Steigerung der Replikationseffizienz als Vielfaches der für AdYB-1 ermittelten Replikation dargestellt ist. Die mit Plasmid pE4orf6 transfizierten und anschließend mit AdYB-1 und E1B-55K (Ad-55K) infizierten Zellen produzierten bis zu 25 mal mehr pfu/ml.

Aufgrund dieser Ergebnisse kann gefolgert werden, dass die Substitution von E1B-55K und E4orf6 die Anzahl der gebildeten Viren (pfu/ml) nach Infektion mit dem E1/E3-deletierten Adenovirus AdYB-1 um einen Faktor von bis zu 25 erhöht. Dabei sind die additiven Effekte von E1B-55K und E4orf6 auf die Produktion von plaque forming units (pfu) signifikant größer als die Effekte eines der beiden Genprodukte alleine.

Kontrollversuche mit einem Plasmid, welches EGFP exprimierte, zeigten deutlich, dass in dem gewählten experimentellen Ansatz nur etwas 10 % der Zellen erfolgreich mit dem Plasmid pE4orf6 transfiziert werden konnten. Die Anzahl der in den Zellen gebildeten Partikel, die sowohl E1B-55K als auch E4orf6 exprimierten, ist mit der des humanen Adenovirustyp 5 (Wildtyp) vergleichbar. Dies bestätigt die der vorliegenden Erfindung zugrundeliegende Erkenntnis, dass die Expression von E4orf6 und E1B-55K in Verbindung mit der Kernlokalisation von YB-1. in der Lage ist, eine adenovirale Replikation bzw. Partikelbildung, insbesondere von E1A-deletiertes Adenoviren zu bewerkstelligen, die vergleichbar derjenigen von Wildtyp Ad5 ist.

**Beispiel 8: Verstärkung der Replikation von in YB1-Kern-negativen Zellen nicht replizierenden Adenoviren in YB-1-Kern-positiven Zellen nach Gabe von** Zytostatika

Im Stand der Technik ist bekannt, dass durch die Zugabe von verschiedenen Zytostatika die Kernlokalisation des humane Transkriptionsfaktor YB-1 induziert wird. Wie vom vorliegenden Erfinder gefunden, steuert kernlokalisiertes YB-1 die adenovirale Replikation mittels Aktivierung des adenoviralen E2-late Promoters. Die Kombination beider Effekte kann dazu genutzt werden, um eine spezifische Tumorlyse herbeizuführen.

Bei der Durchführung der onkolytischen Assays wurde wie folgt vorgegangen. 200.000 Zellen (HeLa bzw. U2OS) wurden je Napf in 6-Napf-Platten ausplattiert. Am nächsten Tag wurden 40 ng/ml (Endkonzentration) Daunorubicin zugegeben. Nach 3 Stunden Inkubationszeit wurden die Zellen mit 10 bzw. 30 pfu dl520/Zelle infiziert. Anschließend wurden die Zellen in Zytostatikafreiem Medium incubiert. Nach 3-5 Tagen wurden die Zellen mit Kristallviolett angefärbt.

Wie aus Fig. 10 und 11 ersichtlich, induziert die Zugabe von Daunorubicin die Replikation von dl520 durch die Kernlokalisation von YB-1. Somit erzielt dl520 in Verbindung mit dem Zytostatikum Daunorubicin einen größeren tumorlytischen Effekt als Daunorubicin alleine.

**Beispiel 9: In vivo Tumorlyse durch dl520**

Die in dieser in vivo Studie verwendeten Zellen HeLa (YB-1 Kern-negativ) und 257RDB (YB-1 Kern-positiv) werden unter sterilen Zellkulturbedingungen expandiert. Kurz vor der Injektion der Zellen in die Mäuse (Stamm CDINuNu), um einen Tumor subcutan zu setzen, werden diese durch Trypsinierung geerntet, in DMEM-Medium (10 % FKS) aufgenommen, gezählt und einmal mit PBS gespült. Anschließend werden die Zellen zentrifugiert, das PBS abgesaugt und in frischem PBS, der gewünschten Zellzahl entsprechen, portioniert. Die subcutan injizierte Zellzahl betrug in dieser Studie je $5x10^6$ Zellen von beiden Zelllinien. Die Injektion erfolgt subcutan in eine Flanke der Tiere, wobei die HELA-Zellen in die rechte Seite und die 257RDB-Zellen in die linke Flankenseite zur besseren Unterscheidung injiziert wurden. Das Wachstum der Tumoren wurde zweimal wöchentlich kontrolliert und dabei die Länge und die Breite der Tumoren mit einer Schiebleere gemessen. Daraus wurde das Tumorvolumen über folgende mathematische Formel berechnet:

$$3/4\pi * a/2 * (b/2)^2 \quad a = \text{Länge}, b = \text{Breite}$$

Wenn der Tumor ein Volumen von 200 bis 520 $mm^3$ erreicht hat, wird das Virus bzw. PBS als Negativkontrolle intratumoral appliziert. Die zu injizierenden Volumina waren gleich und betrugen jeweils 50 μl. Dies wird an 3 aufeinanderfolgenden Tagen wiederholt. Die Gesamtdosis an appliziertem Virus betrug $5x10^8$ pfu. Danach wird das Tumorwachstum weiterhin zweimal pro Woche dokumentiert und das Volumen berechnet. Am Ende der Studie werden die Mäuse euthanasiert und die Tumoren für weitere Analysen entnommen.

Die Ergebnisse sind in den Figuren 12 und 13 dargestellt.

Fig. 12 zeigt ein Diagramm, welches das Volumen des Tumors in Abhängigkeit von der Zeit und den verschiedenen Behandlungsschemata darstellt. Im Falle der durch RDB257 ausgebildeten Tumoren erfolgt bei Injektion von PBS ein signifikantes Wachstum des Tumors von ca. 438 $mm^3$ bis 1466 $mm^3$. Unter dem Einfluss des erfindungsgemäß verwendeten Vektors dl520 konnte das Tumorwachstum signifikant verringert werden. Ausgehend von einer durchschnittlichen Tumorgröße von 344 $mm^3$ erhöhte sich die Tumorgröße lediglich um 21 % auf insgesamt 543 $mm^3$.

Als Kontrolle wurde im vorliegenden Beispiel der Tumor aus HeLa-Zellen verwendet, der sich bei Gabe von PBS hinsichtlich seines Wachstums ähnlich verhielt, wie der auf RDB257 zurückgehende Tumor bei Gabe von PBS. Mit dl520 behandelte HeLa-Zellen basierte Tumoren zeigten einen noch wesentlicheren Anstieg des Tumorwachstums ausgehend 311 $mm^3$ bis 1954 $mm^3$.

Fig. 13 zeigt Aufnahmen von euthanasierten Nacktmäusen, denen unter Anwendung von RDB257 ein Tumor gesetzt wurde. Es ist deutlich zu erkennen, dass nach der erfindungsgemäßen Applikation des Adenovirus dl520 eine signifikante Rückbildung des Tumors eintrat. Im vorliegenden Falle wurde sogar eine Verringerung des Tumorvolumens erzielt (Tag 1 nach Applikation des Virus dl520: 515 $mm^3$; Tag 30 nach Applikation des Virus dl520: 350 $mm^3$.

**Beispiel 10: Southern Blot von Tumor-DNA**

Die DNA wird aus einem Tumorstück isoliert, welches aus der Mitte des in Beispiel 9 gesetzten Tumors entnommen worden ist. Für die Isolierung wird das Dneasy Tissue Kit von der Firma Qiagen benutzt. Die Durchführung der DNA-Isolierung erfolgte nach Herstellerangaben. Dabei wird die DNA aus den Zellen durch eine alkalische Lyse freigesetzt. Anschließend wird isolierte DNA über eine Säule gereinigt. Anschließend wird die Konzentration der isolierten DNA photometrisch bei 260 nm gemessen. Die Analyse erfolgte anhand von 2 μg der DNA-Proben, die mit dem Restriktionsenzym Kpn I 10 Units verdaut wurden. Dann erfolgt eine elektrophoretisch Auftrennung der Proben in einem 0,8%igen Agarosegel. Anschließend wurde die DNA auf eine Nylonmembran geblottet (Durchführung nach dem System von Schleicher & Schuell). Die auf der Membran geblottete DNA wird gegen eine spezifische 1501 bp große DNA-Sonde hybridisiert Die 1501 bp große DNA-Sonde bindet spezifisch an das 3369 bpgroßes Kpn I-Fragment innerhalb der E2A-codierenden Ad5-Sequenz. Die Sonde wurde zuvor per PCR (Primer:5'- GTC GGA GAT CAG ATC CGC GT, 5'- GAT CCT CGT CGT CTT CGC TT) hergestellt und mit $^{32}P$ radioaktiv markiert. Anschließend wird die Membran gewaschen und auf eine Filmfolie exponiert.

Das Ergebnis des Southern-Blots der Tumor-DNA ist in Fig. 14 dargestellt. Die Analyse belegt, dass nur dl520 in den resistenten Zellen RDB257 in vitro repliziert, wie in den Spuren 3, 4 und 5 dargestellt. Spur 1 zeigt als Positivkontrolle Ad-5d, Spur 6, 7 und 8 DNA von HeLa-Zellen, die mit dl520 infiziert wurden. Nachdem HeLa-Zellen keine YB-1-Kernpositivität zeigen, erfolgte darin keine Replikation des Virus dl520, so dass entsprechend die E2A-Sequenz nicht nachgewiesen werden konnte.

Ein weiteres Ergebnis mit dl520 ist in der Fig. 15 dargestellt. Anhand eines Plaque-assays wurde die Partikelbildung (pfulml) nach Infektion mit dl520 und Wildtyp-Adenovirus untersucht. Dabei werden verschiedene YB-1 Kern-positive (257RDB und 181RDB) Tumorzellen und YB-1 kernnegative Tumorzellen mit dl520 und Wildtyp-Adenovirus infiziert.

Dabei wurde wie folgt vorgegangen:

Jeweils 100.000-200.000 Zellen werden in sog. Platten mit 6 Näpfen (engl. 6 well plates) in L 15-Medium (resistente Zellen) bzw. DMEM (nicht-resistente Zellen) jeweils mit 10 % FKS ausplattiert. Nach 24 h erfolgt die Infektion mit dl520 und WT-Adenovirus (10 pfu/Zelle) 3 Tage nach der Infektion (post infectionem) erfolgt die Freisetzung der viralen Partikel aus der Zellsuspension (3 ml) durch dreimaliges alternierendes Einfrieren und Auftauen (engl. thaw/freeze). Anschließend wurde ein Plaque Assay auf 293-Zellen zur Bestimmung der gebildeten infektiösen Partikel (plaque forming units pro ml (pfu/ml)) durchgeführt. Das Ergebnis ist in der Fig. 15 dargestellt. Dabei zeigt das Ergebnis des Plaque Assays, dass dl520 in den YB-1 Kern-positiven Zellen (257RDB und 181RDB) vergleichbar dem Wildtyp-Adenovirus repliziert. Insoweit wird bei der erfindungsgemäßen Verwendung der hierin beschriebenen Adenoviren eine Replikationseffizienz vergleichbar derjenigen von Wildtyp-Adenoviren erreicht.

**Beispiel 11: Struktureller Aufbau des adenoviralen Vektors Xvir03**

Fig.16 zeigt den strukturellen Aufbau des adenoviralen Vektors Xvir03. Das Adenovirus Xvir03 ist ein sogenanntes E1/E3-deletiertes Adenovirus. Das heisst, dass keine für die adenovirale Replikation funktionellen E1A-, E1B- (E1B55k und E1B19K Proteine) und E3- Proteine hergestellt werden. Die Deletion der E1 Region erstreckt sich von 342 - 3528; die Deletion der E3 Region von Basenposition 27865 - 30995. Wie hierin verwendet, bezeichnet der Begriff "E1-deletiertes Virus ein solches Virus, bei dem E1 funktional nicht mehr aktiv ist. Dies kann durch eine Inaktivierung bei ansonsten weitestgehend intakter Nukleinsäure- bzw. Aminosäuresequenz erfolgen, kann jedoch auch eine unterschiedlich große Deletion des für E1-Region kodierenden Proteine bedeuten. Durch das Fehlen ders E1A und E1B Proteines bzw. der dafür codierenden Nukleinsäuren wird die E4-Region, z.B. E4orf6, nicht oder nur sehr schwach (ca. 1-5% im Vergleich zum Wildtyp-Adenovirus) exprimiert. In der E1-Region werden die viralen Gene E1B55k und E4orf6 vermittelt durch den in den Xvir 03 eingeführten, heterologen CMV-Promoter (Firma Clontech: Plasmid pShuttle) exprimiert. Anstelle des CMV-Promotors können auch jene hierien beschriebenen Promotoren verwendet werden, wie im Zusammenhang mit der Expression von E1A offenbart. Der offene Leserahmen beider Gene ist über eine sogenante IRES-Sequenz (engl.: internal ribosomal entry site) miteinander verbunden (Pelletier, J. and Sonenberg, N. Nature, 1988, 334, 320-325). Dieses Element (Firma Novagen: pCITE) erlaubt die Expression von 2 Proteinen aus einer mRNA.

**Bei der Herstellung des Vektors wurde wie folgt vorgegangen: System Adeno-X der Firma Clontech**

Das Plasmid E1B55k-pShuttle ging durch Umklonierung des offenen Leserahmen von E1B55k aus pCGNE1B von M. Dobelstein (Universität Marburg) mit XbaI und BfrI bzw. nur Bam HI, wobei in diesem Fall dann die Enden geglättet und ins geglättet pshuttle einkloniert werden, in den pShuttle-Vektor von Clontech hervor. Anschließend wurde E1B55k in pShuttle mit ApaI linearisiert, die Enden geglättet und mit NheI geschnitten.

In einem zweiten Vektor, dem pcDNA3.1(+) (Invitrogen), wurden nacheinander das IRES-Element als PCR-Produkt mit dem pCITE-4a(+) der Firma Novagen als Template über TA-Klonierung in die EcoRV-Schnittstelle und das E4orf6 aus dem Plasmid pCMV-E4orf6 (M. Dobelstein, Uni Marburg) über BamHI hineinkloniert = IRES-E4orf6-pcDNA3.1(+). IRES-E4orf6 in pcDNA3.1 (+) wurde linearisert mit NotI, die Enden geglättet und anschließend wurde das Fragment IRES-E4ORF6 mit NheI herausgeschnitten. Das Fragment IRES-E4orfF6 wurde mit dem geöffneten Vektor E1B55k-pShuttle (blunt, NheI) verknüpft. Die Kassette wurde anschließend aus dem E1B55k-IRES-E4orf6-pShuttle zusammen mit dem CMV-Promotor und dem *bovine growth hormone* (BGH)-PolyA in das ΔE1, ΔE3 Adeno-X-Plasmid (Clontech) mit I-Ceu I und PI-SceI kloniert und als AdcmvE1B/IRES/E4orf6 bezeichnet. Danach erfolgte die Adenovirusherstellung nach Herstellerangaben (Clontech). Das mit PacI linearisierte Adenoplasmid mit dem Expressionselement CMV-E1B55k-IRES-E4orf6-BGH polyA wurde mit in HEK293 Zellen transfiziert und nach 11 Tagen *post transfectionem* die sich ablösenden Zellen zusammen mit dem Medium abgenommen, um durch wiederholte Einfrier-Auftau-Zyklen die entstandenen Adenoviren freizusetzten.

Es ist im Rahmen der vorliegenden Erfindung und für den Fachmann im Lichte der vorliegenden Offenbarung durchführbar, dass für die Herstellung der erfindungsgemäßen, bevorzugterweise rekombinanten Adenoviren, insbesondere solche, welche die Kassetten E4orf6-IRES-E1B55k bzw. YB-1-IRES-E1A12S einzeln und/oder zusammen enthalten, auch andere Systeme verwendet werden können, z. B. das System AdEasy der Firma QBIOGENE und Microbix. Zudem können die einzelnen Transgene innerhalb der Kassetten untereinander ausgetauscht werden. Es ist somit im Rahmen der vorliegenden Erfindung, dass auch solche Adenoviren hergestellt und erfindungsgemäß verwendet werden können, bei denen die Kassetten den folgenden Aufbau aufweisen: E1B55k-IRES-E4orf6 bzw. E1A12S-IRES-YB1.

Im Rahmen der vorliegenden Erfindung wurde ein sogenanntes E1/E3-deletiertes rekombinantes Adenovirus, welches die Kassetten E4orf6-IRES-E1B55k enthält, verwendet. Es ist jedoch im Rahmen einer Ausführungsform, dass das Virus nur eine E1-Deletion aufweist, d. h. dass die E3-Region intakt bleibt. Dabei kann wahlweise die E4-Region teilweise und/oder vollständig deletiert vorliegen.

Bei der Herstellung des Vektors mit Hilfe anderer Systeme wurde wie folgt vorgegangen. Herstellung des Adenovirus Ad-Xvir 3'UTR mit intakter E3-Region mit dem Vektorsystem nach Graham (Firma Microbix)

**Klonierung des Vektors CMV-E40RF6-IRES-E1B55k 3'UTR-polyA in pDelta ElspIA**

Für das Plasmid E1B55k 3'UTR-pShuttle (Clontech) wurde der offene Leserahmen mit der 3'-UTR durch Amplifikation aus der DNA des Adenovirus Typ 5 gewonnen (E1B55k Vorwärtsprimer = 5'-ATGGAGCGA.AGAAACCC-3' und E1B55k 3'UTR Rückwärtsprimer = 5'- CACGTCCTGGAAAAAATACAC-3') und in die geglättete NheI-Schnittstelle, die mit T-Enden versehen wurde (TA -Klonierung), in das pShuttle-Plasmid der Firma Clontech kloniert. Dadurch wurde das Transgen am 5' Ende mit einem hCMV-Promotor versehen und am 3' Ende mit dem Bovine Growth Hormone Polyadenylierungssignal. Es ist jedoch auch im Rahmen der vorliegenden Erfindung, das E1B55k aus dem Plasmid pCGNE1B von Dobbelstein (Dobbelstein, M. et al., EMBO Journal, 16, 4276-4284, 1997) via Bam HI und Glätten bzw. TA-Klonierung zu verwenden. In den Figs. 23 und 24 ist unter anderem das einklonierte E1B55k-3'UTR näher beschrieben

**Klonierung des Vektors E4ORF6-IRES-pcDNA3.1(+)**

Die Amplifikate E4orf6 mit der Adenovirus Typ 5 DNA als Template (E4orf6 Vorwärtsprimer 5'-CTTCAGGATCCATGACTACGTCCGGCG-3' und E4orf6 Rückwärtsprimer 5'-GAAGTGAATTCCTACATGGGGGTAGAGTCATAATCGT-3') bzw. aus dem Plasmid pCMVE4-34kD mit Bam HI herausgeschnitten (Dobbelstein et al., EMBO, 16, 4276-4284,1997) und das IRES-Element mit dem pCITE-4a(+) der Firma Novagen als Template (IRES Vorwärtsprimer = 5'-TCCGGTTATTTTCCACCATATTGC-3' und IRES Rückwärtsprimer = 5'-TTATCATCGTGTTTTTCAAAGG-3') wurden nacheinander in die Multiple Klonierungsstelle des pcDNA3.1(+)-Vektor hineinkloniert. Hierzu wurden für das E4orf6 Transgen Primer verwendet, die eine *Bam*HI Schnittstelle am 5'-Ende und eine *Eco*RI-Schnittstelle am 3'-Ende des offenen Leserahmens generierten. Das Amplifikat wurde mit den entsprechenden Restriktionsenzymen verdaut und damit die Enden für die gerichtete Klonierung in den mit entprechend *Bam*HI und EcoRI geöffneten Vektor kompatibel gemacht. Anschließend wurde das Plasmid E4orf6 in pcDNA3.1 (+) mit *Eco*RV linearisiert, T-Enden angehängt und das Amplifikat für das IRES-Element hineinkloniert. Nach Überprüfung der korrekten Orientierung des IRES-Elements wurde der Vektor für die weitere Klonierung benutzt.

Die Verknüpfung der beiden Transgene mit dem IRES-Element entstand durch Umklonierung der Kassette E4ORF6-IRES in das vorweg generierte Plasmid CMV-E1B55k 3'UTR-polyA - pShuttle (Clontech), das mit *Not*I linearisiert, geglättet und anschließend mit *Xba*I geschnitten wurde. E4ORF6-IRES in pcDNA3.1(+) wurde mit *Not*I linearisiert, die Enden geglättet und weiter mit *Nhe*I verdaut. Durch die Ligation des E4ORF6-IRES-Inserts mit dem CMV-E1B55k 3'UTR-polyA-pShuttle (Clontech) entstand XVIR-3'UTR in pShuttle (Clontech).

Herstellung des verwendeten adenoviralen Shuttlevektor

Da der für das nun benutzte adenovirale Herstellungssystem der Firma Microbix der Shuttlevektor p∆E1sp1A weder einen CMV-Promotor noch ein Bovine Growth Hormone Polyadenylierungssignal enthält, wurden diese Elemente in p∆E1sp1A hineinkloniert. Hierzu wurde p∆E1sp1A mit *Cla*I linearisiert, geglättet und mit *Eco*RI geschnitten. Die Elemente CMV-MCS(multiple cloning site)-Poly-A wurden aus pShuttle (Clontech) mit MfeI linearisiert, die Enden geglättet und weiter mit EcoRI geschnitten. Anschließend wurde die Kassette (Xvir-3'UTR pShuttle von Clontech) mit PmeI in den ebenfalls mit PmeI geschnittenen und dann dephosphorylierten CMV-MCS-Poly-A p∆E1sp1A Vektor umkloniert. Das Klonierungsprodukt Xvir-3'UTR-p∆E1sp1A wurde für die Virusherstellung verwendet.

Virusherstellung

Xvir-3'UTR-pAElsp1A und pBHGE3 (von Microbix, enthält die E3-Region, die dem wildtyp des Adenovirus Typ 5 entspricht) wurden in HEK 293 Zellen kotransfiziert, wodurch infolge Rekombination homologer Sequenzbereiche beider Vektoren das Virus Ad-Xvir-3'UTR E3 entstand.

Herstellung des Adenovirus Ad-Xvir 3'UTR-AdEASY E3 mit dem AdEASY-System (Firma Qbiogene)

Herstellung des verwendeten adenoviralen Shuttlevektors

Da für das hier verwendete System der vorliegende Vektor pShuttle-AdEASY weder einen CMV-Promotor noch das Bovine Growth Hormone Polyadenylierungssignal enthielt, wurden diese Elemente in pShuttle-AdEASY hineinkloniert. Hierzu wurde das Plasmid mit *Eco*RI verdaut, die Enden durch Auffüllen mit T4-Polymerase und dNTPs geglättet, das Backbone dephosphoryliert und die beiden entstandenen Schnittprodukte religiert. Durch diese Vorgehensweise wurde die Restriktionserkennungssequenzen für *Eco*RI vernichtet. Das daraus hervorgegangene Plasmid wurde pShuttle(-*Eco*RI)-AdEASY genannt.

Anschließend wurde die Kassette CMV-MCS-polyA aus dem pShuttle von Clontech mit *Mfe*I und *Eco*RI geschnitten, die Enden geglättet und in den Vektor pShuttle (-*Eco*RI)-AdEASY kloniert, der dafür mit *Xba*I linearisiert, geglättet und dephosphoryliert wurde. Daraus entstand das Plasmid CMV-MCS-PolyA-pShuttle-AdEASY. In dieses Plasmid wurde die Kassette E4Orf6-IRES-E1B55k-3'UTR mit *Mlu*I und *Eco*RI hineinkloniert. Dadurch entstand das Plasmid Xvir-3'UTR in pShuttle AdEASY. Dieses wurde mit *Bst*1 107I und *Mro*I linearisiert und zusammen mit dem Rescue-Plasmid pAdEASY in BJ5183 (EC) Bakterien mittels Elektroporation eingebracht. Durch homologe Rekombination entstand das adenovirale Plasmid Ad-Xvir-3'UTR-pAdEASY, das nach Transfektion in HEK293 Zellen zur Virusproduktion führte.

Einbringung der wt E3 Region in das pAdEASY

Da die E3 Region in dem Plasmid pAdEASY weitgehend deletiert ist, wurde für die Rekonstruktion die E3E4-Region aus dem Plasmid pAdEASY mit *Spe*I und *Pac*I in das Plasmid CMV-MCS-PolyA pShuttle (AdEASY) kloniert und damit das Plasmid E3E4-pShuttle-AdEASY generiert.

Durch Restriktion mit *Nde*I und Religation wurde von zwei *Nde*I Schnittstellen eine deletiert und damit auch die Multiple Klonierungsstelle aus dem Plasmid. Durch diese Prozedur entstand das Plasmid E3E4-pShuttle (-*Nde*I)-AdEASY.

Anschließend wurde das 4007 bp große wtE3-Region Fragment aus Wildtyp Adenovirus Typ 5 mit *SpeI* und *NdeI* ausgeschnitten und in das *Spe*I und *Nde*I aufgeschnittene E3E4-pShuttle (-*Nde*I)-AdEASY kloniert. Der so entstandene Vektor wurde wtE3E4-pShuttle (-*Nde*I)-AdEASY benannt.

Anschließend wurde die wtE3 E4-Region aus dem E3E4-pShuttle (-*Nde*I)-AdEASY mit *Spe*I und *Pac*I geschnitten und in das mit *Spe*I und *Pac*I geschnittene pAdEASY reinkloniert, wodurch in dem Plasmid pAdEASY die E3-Region wieder hergestellt wurde (pAdEASY-E3). Durch die Transformation von BJ5183 (EC) Bakterien mit den Plasmiden Xvir-3'UTR in pShuttle AdEASY und pAdEASY-E3 entstand durch homologe Rekombination Xvir-3'UTR-pAdEASY-E3.

E4-Manipulation für die genannten Systeme

Um für mögliche therapeutische Transgene Platz zu schaffen und um eine unerwünschte homologe Rekombination zu unterbinden, kann die E4-Region im Plasmid E3E4-pShuttle (-*Nde*I)-AdEASY spezifisch deletiert werden. Dabei wird die E4orf6-Region durch Herausschneiden mit PstI und Religation um ca. 0,6 kB, bevorzugterweise 629 oder 634 bp verkürzt. Dies kann wie beschrieben in Figur 17 Xvir03/01 der Fall sein. Entsprechende Deletionen sind in anderen Systemen zur Herstellung rekombinanter Adenoviren durch den Fachmann durchführbar.

Klonierung des RGD-Motivs in Ad-Xvir 3'UTR-AdEASY E3 im speziellen (gilt aber auch für alle anderen Systemen)

Für die verbesserte Infektiösität wurde in Anlehnung an Dmitriev et al. 1998 (An Adenovirus Vector with Genetically Modified Fibers Demonstrates Expanded Tropism via Utilization of a Coxsackievirus and Adenovirus Receptor-Independent Cell Entry Mechanism) der HI Loop des Fiber-knob Domäne modifiziert: Die entsprechende Region wurde mit Primern RGD-Hpa fw (5'-GAGgttaacCTAAGCACTGCCAAG-3'), RGD-EcoRV rev (5'-CATAGAGTATGCAGATATCGTTAGTGTTACAGGTTTAGTTTTG-3') sowie RGD-EcoRV fw (5'-GTAACACTAACGATATCTGCATACTCTATGTCATTTTCATGG-3') und RGD-Bfr rev (5'-CAGCGACATGAActtaagTGAGCTGC-3') amplifiziert und dabei eine EcoRV-Schnittstelle generiert. In diese Schnittstelle wurden gepaarte Oligonukleotide kloniert, die für ein Arg-Gly-Asp (RGD)- Peptid kodieren: RGD-Oligo 1 (5'-CACACTAAACGGTACACAGGAAACAGGAGACACAACTTGTGACTGCCGCGGAGACT GTTTCTGCCC-3') und RGD-Oligo 2 (5'-GGGCAGAAACAG TCTCCGCGGCAGTCA CAAGTTGTGTCTCCTGTTTCCTGTGTACCGTTTAGTGTG-3'). Somit befindet sich das RGD-Motiv im HI Loop der Fiberknob-Domäne.

Der vorstehend beschriebene Vektor eignet sich grundsätzlich wie die anderen hierin beschriebenen erfindungsgemäß zu verwendenden Viren. Insbesondere ist der vorstehend beschriebene Vektor dazu geeignet, in YB-1-Kern-positiven Zellen sowie Zellen, in denen YB-1 dereguliert, d.h. im Vergleich zu Normal-Zellen bzw. Nicht-Tumorzellen überexprimiert vorliegt, zu replizieren und insoweit eine Lyse herbeizuführen. Die Anwendung dieses Vektors erstreckt sich dabei auch insbesondere auf jene Krankheiten und Patientengruppen oder Patientenkollektive, die für die anderen hierin beschriebenen erfindungsgemäß zu verwendenden Adenoviren und erfindungsgemäßen Adenoviren offenbart sind.

**Beispiel 12: Struktureller Aufbau des adenoviralen Vektors Xvir03/01**

Wie aus Fig. 17 ersichtlich, ist XVIR03/01 eine Weiterentwicklung von XVIR03. Dabei können in der E3 Region therapeutische Gene wie beispielsweise die hierin beschriebenen Gene und Transgene kloniert werden. Zudem wurde eine Deletion in der E4-Region eingeführt, um eine homologe Rekombination mit dem E4orf6 aus der Expressionskassette von Xvir03 zu vermeiden. Dies führt auch dazu, dass bei diesem Konstrukt größere Transgene kloniert werden können. Die deletierte E3-Region enthält für das Einsetzen einer Kassette nutzbare SacI, NdeI und NheI- Schnittstellen, in die beispielsweise die therapeutischen Transgene einkloniert werden können. Es kann jedoch auch die E3-Region intakt verbleiben und die therapeutischen Gene in die E4-Region kloniert werden. Somit wird unter anderem die Expression des adenoviral death protein ADP sichergestellt.

**Vorbereitung eines Plasmids zur Klonierung therapeutischer Gene in die E3-Region sowie zur Deletion in der E4-Region:**

Das pAdenoX-Plasmid von Clontech verfügt über eine SfuI -Schnittstelle hinter der 3' ITR-Region, die im wildtyp Adenovirus fehlt. Die E3-E4-Region wurde mit SpeI (Position 23644) und SfuI aus pAdenoX (Clontech) in pcDNA3.1(+) (Invitrogen) übernommen = pcDNA3.1-E3Δ27865-30995-E4. Der Großteil des E4ORF6, nämlich 33241-33875 wurde mittels PstI entfernt = pcDNA3.1-E3Δ27865-30995,E4Δ33241-33875. Für die Weiterentwicklung von XVIR03 wurde der deletierte E3/E4-Bereich aus pcDNA3.1-E3Δ27865-30995, E4Δ33241-33875 mittels SfuI und SpeI in das Plasmid pAdenoX kloniert = pAdenoX E3Δ27865-30995, E4Δ33241-33875.

Die Expressionskassette wurde anschließend wie für Xvir03 beschrieben aus dem E1B55k-IRES-E4orf6-pShuttle zusammen mit dem CMV-Promotor und dem *bovine Growth hormone* (BGH)-PolyA in das pAdenoX E3Δ27865-30995.E4Δ33241-33875 mit I-Ceu I und PI-SceI kloniert und als AdcmvE1B/IRES/E4orf6-ΔE4 bezeichnet. Danach erfolgte die Adenovirusherstellung nach Herstellerangaben (Clontech).

Es ist im Rahmen der vorliegenden Erfindung und für den Fachmann im Lichte der vorliegenden Offenbarung offensichtlich und praktikabel, dass für die Herstellung der erfindungsgemäßen, bevorzugterweise rekombinanten Adenoviren auch andere Systeme verwendet werden können, z. B. das System der Firma QBIOGENE und der Firma Microbix.

Der vorstehend beschriebene Vektor eignet sich grundsätzlich wie die anderen hierin beschriebenen erfindungsgemäß zu verwendenden Viren. Insbesondere ist der vorstehend beschriebene Vektor dazu geeignet, bei YB-1-Kern-positiven Zellen sowie Zellen, in denen YB-1 dereguliert, d.h. im Vergleich zu Normal-Zellen bzw. Nicht-Tumorzellen überexprimiert vorliegt zu replizieren und insoweit eine Lyse herbeizuführen. Die Anwendung dieses Vektors erstreckt sich dabei auch insbesondere auf jene Krankheiten und Patientengruppen oder Patientenkollektive, die für die anderen hierin beschriebenen erfindungsgemäß zu verwendenden Adenoviren und erfindungsgemäßen Adenoviren offenbart sind.

**Beispiel 13: Onkolytische Wirkung von Xvir 03 in 257 RDB- und 181 RDB-Zellen**

Pro Napf einer Platte mit sechs Näpfen (engl. 6 well plate) wurden jeweils 100.000 Zellen (257RDB und 181RDB) ausplattiert. Am nächsten Tag wurden die Zellen wie in Fig. 18 dargestellt mit Ad312 (20pfu/Zelle) und Xvir03 (5pfu/Zelle) infiziert. Die Infektion erfolgte in 500 μL serumfreie DMEM-Medium für 1 h bei 37° C. Anschließend wurde das Infektionsmedium entfernt und durch 2 ml Vollmedium (10 % FKS/DMEM) ersetzt. Nach 5 Tagen erfolgte die Auswertung mit Hilfe einer Kristallviolettfärbung. Das Ergebnis ist in Figs. 18A und 18B dargestellt.

Wie aus Fig. 18A und 18B ersichtlich, zeigen die vielfachresistenten Zellen, welche YB-1 im Kern aufweisen, nach Infektion mit Ad312 und Xvir03 nur bei Xvir03 eine Lyse, wie durch Kristallviolettfärbung der Zellen dargestellt. Dabei wird zunächst das Medium entfernt. Anschließend werden die Zellen mit Kristallviolett überschichtet (50 % ETOH, 3 % Formaldehyd, 5 % Essigsäure, 1 % Kristallviolett) und für 5-10 min bei Raumtemperatur inkubiert. Danach werden die Platten mit sechs Näpfen mit Wasser gründlich gewaschen und bei Raumtemperatur getrocknet.

Es ist dem vorliegenden Erfinder bekannt, dass E1A-deletierte Viren (z. B. Ad312), die jedoch keine transaktivierenden Adenoviren im Sinne der vorliegenden Erfindung sind, bei höheren MOI's sehr effizient replizieren können (Nevins J. R., Cell 26, 213-220, 1981), die jedoch in der klinischen Anwendung nicht zu realisieren sind. Dieses Phänomen wird in der Literatur als "E1A-like activity" bezeichnet. Der hier eingesetzte Adenovirus Ad312 ist ein E1A-deletiertes Virus. Bei der eingesetzten Menge (20 pfu/zelle), die noch oberhalb des klinisch wünschenswerten Titers liegt, werden die frühen adenoviralen Gene wie z. B. E1B55k und E4orf6 nicht oder nur sehr gering exprimiert (Nevins J. R., Cell 26, 213-220, 1981). Wie bereits beschrieben spielen diese Gene bzw. die Proteine eine bedeutende Rolle bei der viralen Replikation. Im Gegesatz dazu werden diese Gene bzw. Proteine beim Adenovirus Xvir03 exprimiert (Fig 16). Anhand der Fig. 18A und 18B wird ersichtlich, dass die Expression der Gene E1B55k und E4orf6 zur einer effizienten viralen Replikation und Zellyse führen bei gleichzeitig geringem erforderlichen Infektionstiter (augedrückt als pfu/Zelle). Dies bestätigt die der vorliegenden Erfindung zugrundeliegende Erkenntnis, dass die Expression von E4orf6 und E1B-55K (und das Nicht-Vorhandensen von E1A) in Verbindung mit der Kernlokalisation von YB-1 in der Lage ist, eine sehr effiziente adenovirale Replikation zu induzieren. Die dazu erforderliche Menge von nur 1 bis 5 pfu/Zelle lässt jetzt eine klinischen Anwendung zu.

Dies bestätigt die der vorliegenden Erfindung zugrundeliegende Erkenntnis, dass das Vorhandensein von YB-1 im Kern, insbesondere Zellzyklus-unabhängig, erforderlich ist, um die erfindungsgemäß zu verwendenden Viren zu einer Lyse von infizierten Zellen zu veranlassen.

**Beispiel 14: Replikation von Adenovirus in Zellen nach Gabe von Irinotecan**

Um die Wirkung von Irinotectan auf die adenovirale Replikation zu bestimmen, wurden $10^6$-Tumorzellen U373 in 10 $cm^2$ Petrischalen ausplattiert. In einem ersten Ansatz erfolgte nach 24 h die Zugabe von 5μM Irinotecan. Nach wiederum 24 h wurden die Zellen mit 10 pfu/Zelle dl520 infiziert. Nach 3 Tagen Inkubation ohne Irinotecan wurde die DNA gemäß der in Beispiel 10 beschriebenen Vorgehensweise isoliert.

In einem parallelen Ansatz wurden die solchermaßen angesetzten U373-Zellen nicht mit Irinotecan präinkubiert. Nach 48-stündiger Kultivierung der Zellen ohne Irinotecan wurden diese mit 10 pfu/Zelle dl520 infiziert und anschließend ohne Irinotecan für weitere 3 Tage inkubiert. Die DNA wurde wie oben beschreiben isoliert.

Anschließend wurden 2μg DNA mit dem Restriktionsenzym Kpn I verdaut und eine Southern Blot Analyse durchgeführt. Als Sonde diente ein mittels PCR hergestellter Abschnitt des adenoviralen Genoms (Position:22734-24235).

Das Ergebnis ist in Fig. 19 dargestellt. Fig. 19. zeigt, dass nach Inkubation mit Irinotecan die adenovirale Replikation in U 373 Zellen nach Behandlung mit Irinotecan (Bahn 2) gegenüber der unbehandelten Kontrolle, bei der keine Inkubation mit Irinotecan erfolgte (Bahn 1), stark erhöht ist. Die bedeutet, dass unter dem Einfluss von Irinotecan die adenovirale Replikation erhöht ist.

**Beispiel 15: Replikation von Adenovirus in Zellen nach Gabe von Trichostatin A**

Um die Wirkung von Trichostatin A auf die adenovirale Replikation zu bestimmen, wurden $10^6$-Tumorzellen U373 in 10 $cm^2$ Petrischalen ausplattiert. Nach 24 h erfolgte die Zugabe von 0, 0,25, 0,5 und 0,75 μM Trichostatin A. Nach wiederum 24 h wurden die Zellen mit 10 pfu/Zelle dl520 infiziert.

Nach 3 Tagen Inkubation im Medium ohne Trichostatin wurde die DNA isoliert. Anschließend wurden 2μg DNA mit dem Restricktionsenzym Kpn I verdaut und eine Southern Blot Analyse durchgeführt. Als Sonde diente ein mittels PCR hergestellter Abschnitt des adenoviralen Genoms (Position:22734-24235).

Das Ergebnis ist in Fig. 20 dargestellt. Fig. 20 zeigt, dass nach Inkubation mit steigenden Konzentrationen an Trichostatin A die adenovirale Replikation in U 373 Zellen (Bahnen 2,3 und 4) gegenüber der unbehandelten Kontrolle, bei der keine Inkubation mit Trichostatin A erfolgte (Bahn 1), stark erhöht ist. Die bedeutet, dass unter dem Einfluss von Trichostatin A die adenovirale Replikation erhöht ist.

### Beispiel 16: Beeinflussung der Expression des Coxsackievirus-Adenovirus-Rezeptors (CAR) auf U373 Zellen in Reaktion auf die Gabe von Trichostatin A

200.000 Zellen U373 wurden in 6-Loch-Platten ausplattiert. Nach 24 h wurden die Zellen mit 1 μM Trichostatin für 24h kultiviert. Nach wiederum 24h wurden die Zellen isoliert. Anschließend erfolgte die Analyse der CAR-Expression nach Standardprotokoll mittels Facs-Analyse und dem primären Antikörper anti-CAR Klon RmcB der Firma Upstate und dem sekundären Antikörper Kaninchen-anti-Maus FITC (Firma DAKO).

Das Ergebnis ist in Fig. 21 dargestellt. Ohne Trichostatin-Behandlung waren 11.3 % der Zellen CAR-positiv, wohingegen nach Inkubation der Zellen mit 1μM Trichostatin 56, 2 %der Zellen CAR-positiv. Die Zahlen sind % der insgesamt im Test verwendeten Zellen.

Aus Fig. 21 kann somit entnommen werden, dass unter dem Einfluss des Histon-Deacylase-Inhibitors Trichostatin A der für das Binden von Adenoviren wichtige Faktor CAR unter dem Einfluss von Trichostatin A im größeren Maße exprimiert wird bzw. verfügbar ist, was die Effizienz der Transfektion der solchermaßen behandelten Zellen erhöht.

### Beispiel 17: Onkolyse von U373 Zellen durch Adenovirus nach Kombinationsbehandlung der Zellen mit Irinotecan und Trichostatin A.

Es wurden 200.000 U373-Zellen in 6-Loch Platten ausplattiert. Nach 24 h wurden entweder nur 2μM Irinotecan oder nur 1 μM Trichostatin A oder 1 μM Irinotecan + 0.5 μM Trichostatin zum Medium zugegeben. Nach 24 h Inkubation wurden die Zellen mit 10, 20 und 30 pfu/Zelle dl520 infiziert. Nach 3-5 Tagen erfolgte die Auswertung mit Hilfe der Kristallviolettfärbung. Die Versuche wurden in doppelter Ausführung durchgeführt.

Das Ergebnis ist in Fig. 22 dargestellt. Die sechs in Panel 1 dargestellten Platten zeigen einen vollständigen Zellrasen, der durch die Inkubation mit einer Kombination von Irinotecan und Trichostatin A nicht beeinträchtigt wird, wie durch die Kristallviolettfärbung dargestellt. Die beiden nächsten Doppel-Löcher von Panel 1 zeigen den Zellrasen nach Infektion mit 10 bzw. 20 pfu/Zelle dl520. Auch bei diesen Bedingungen kommt es nicht zu einer Lyse der Zellen, was auf das Ausbleiben einer Replikation von dl520 zunickzuführen ist. Damit wird gezeigt, dass weder dl520 bei 10 noch bei 20 pfu/Zelle noch 1 μM Irinotecan + 0.5 μM Trichostatin A alleine eine Zelllyse induzieren können.

Die weiteren in Fig. 22 dargestellten 6-Loch-Platten 2, 3 und 4, hierin auch als Panele 2, 3 und 4 bezeichnet, wurden grundsätzlich nach folgenden Schema behandelt. Die einzelnen Löcher wurden wie eingangs beschrieben mit U 373 Zellen beimpft und die Zellen darin kultiviert. Die Näpfe wurden, jeweils doppelt mit 10, 20 oder 30 pfu/Zelle dl 520 beimpft, wobei die Unterschiede der insgesamt drei 6-Loch-Platten in der Art der verwendeten Cytostatika bestand. Bei Panel 2 wurden 2μM Irinotecan, bei Panel 3 1 μM Trichostatin A und bei Panel 4 1 μM Irinotecan und 0,5 μM Trichostatin A zu den einzelnen Löchern zugesetzt.

In der 6-Loch Platte 2 (Panel 2) mit 2 μM Irinotecan werden die Zellen mit 30 pfu/Zelle dl520 lysiert. In der 6-Loch Platte 3 (Panel 3) mit 1 μM Trichostatin A werden die Zellen bei 20 und 30 pfu/Zelle d1520 lysiert. In der 6-Loch Platte 4 (Panel 4) mit 1 μM Irenotecan + 0.5 μM Trichostatin A werden die Zellen dagegen bereits bei 10 pfu/Zelle dl520 lysiert.

Die in den Figs. 19 bis 23 in ihrem Ergebnis dargestellten Versuche zeigen, dass die Kombination bestehend aus Irinotecan + Trichostatin A + dl520 eine effektivere Zellyse der Tumorzellen induziert als jede Substanz für sich alleine. Dies wird dadurch erreicht, dass Trichostatin A einerseits die CAR-Expression erhöht und somit die Infizierbarkeit der Zellen merklich verbessert. Andererseits wird durch Irinotecan YB-1 in den Zellkern transloziert und induziert somit eine verbesserte adenovirale Replikation. Zudem dient das zelluläre YB-1 nach Infektion mit dl520 der adenoviralen Replikation und steht für DNA-Reparaturvorgänge nicht mehr zur Verfügung. Je nach Betrachtungsweise führt dies zur verbesserten Wirksamkeit einerseits von dl520 und einer verbesserten Wirksamkeit der Cytostatika andererseits.

## Patentansprüche

1. Verwendung eines Adenovirus zur Herstellung eines Medikamentes, **dadurch gekennzeichnet, dass** der Adenovirus replikationsdefizient ist in Zellen, die YB-1 nicht im Kern aufweisen, der Adenovirus in Zellen repliziert, die YB-1 im Kern aufweisen, und der Adenovirus für ein virales Onkogenprotein E1A codiert, das zumindest ein adenovirales Gen transaktiviert, wobei das adenovirale Gen ausgewählt ist aus der Gruppe, die E1B55kDa, E4orf6, E4orf3 und E3ADP besteht, und das Medikament eine Kombination von mindestens zwei Mitteln umfasst, wobei ein jedes Mittel einzeln und unabhängig aus der Gruppe ausgewählt ist, die aus Cytostatika besteht, und wobei mindestens eines der Mittel den Transport von YB-1 in den Zellkern vermittelt.

2. Verwendung nach Anspruch 1, **dadurch gekennzeichnet, dass** das virale Onkoprotein E1A nicht die nukleäre Lokalisation von YB-1 induziert.

3. Verwendung nach einem der Ansprüche 1 bis 2 **dadurch gekennzeichnet, dass** das Medikament für Patienten ist, deren Zellen Rb-positiv oder Rb-negativ sind.

4. Verwendung nach einem der Ansprüche 1 bis 3, **dadurch gekennzeichnet, dass** das Medikament für die Behandlung von Tumoren ist.

5. Verwendung nach Anspruch 4, **dadurch gekennzeichnet, dass** die Zellen, insbesondere die den Tumor oder Teile davon ausbildenden Zellen eine Resistenz, insbesondere Mehrfachresistenz gegen pharmakologische Wirkstoffe, bevorzugter Weise Antitumormittel und bevorzugtererweise Zytostatika, aufweisen.

6. Verwendung nach Anspruch 5, **dadurch gekennzeichnet, dass** die Zellen eine Expression, bevorzugterweise eine Überexpression des membranständigen Transportproteins P-Glykoprotein zeigen.

7. Verwendung nach einem der Ansprüche 1 bis 6, **dadurch gekennzeichnet, dass** die Zellen p53-positiv oder p53-negativ sind.

8. Verwendung nach einem der Ansprüche 2 bis 7, **dadurch gekennzeichnet, dass** das Onkogenprotein gegenüber dem Wildtyp-Onkogenprotein E1A eine oder mehrere Mutationen oder Deletionen aufweist, wobei die Deletion bevorzugt eine solche ist, die ausgewählt ist aus der Gruppe, die Deletionen des Bereichs CR3 und Deletionen des N-Terminus und Deletionen des C-Terminus umfasst.

9. Verwendung nach Anspruch 8, **dadurch gekennzeichnet, dass** das EIA-Onkogenprotein an Rb binden kann.

10. Verwendung nach einem der Ansprüche 2 bis 7, **dadurch gekennzeichnet, dass** das Onkogenprotein gegenüber dem Wildtyp-Onkogenprotein eine oder mehrere Mutationen oder Deletionen aufweist, wobei die Deletion bevorzugt eine solche in der CR1-Region und/oder der CR2-Region ist.

11. Verwendung nach Anspruch 10, **dadurch gekennzeichnet, dass** das Onkogenprotein E1A nicht an Rb zu binden in der Lage ist.

12. Verwendung nach einem der Ansprüche 1 bis 11, **dadurch gekennzeichnet, dass** das virale Onkogenprotein unter der Kontrolle eines Gewebes- und/oder Tumor-spezifischen Promotors steht.

13. Verwendung nach einem der Ansprüche 1 bis 12, **dadurch gekennzeichnet, dass** der Adenovirus für YB-1 codiert.

14. Verwendung nach Anspruch 13, **dadurch gekennzeichnet, dass** YB-1 unter der Kontrolle eines Gewebe- und/oder Tumor-spezifischen Promotors steht.

15. Verwendung nach einem der Ansprüche 1 bis 14, **dadurch gekennzeichnet, dass** der Adenovirus für mindestens ein Protein codiert, das ausgewählt ist aus der Gruppe, die aus E4orf6, E4orf3, E1B55k und adenoviralem E3ADP-Protein besteht.

16. Verwendung nach einem der Ansprüche 1 bis 15 **dadurch gekennzeichnet, dass** die Zellen YB-1 im Kern aufweisen, insbesondere die den Tumor oder einen Teil davon ausbildenden Zellen YB-1 im Kern aufweisen.

17. Verwendung nach einem der Ansprüche 1 bis 16, **dadurch gekennzeichnet, dass** der Tumor YB-1 im Kern nach Induktion des Tranports von YB-1 in den Kern enthält.

18. Verwendung nach Anspruch 17, **dadurch gekennzeichnet, dass** der Transport von YB-1 in den Kern ausgelöst wird durch zumindest eine Maßnahme, die ausgewählt ist aus der Gruppe, die aus Bestrahlung, Gabe von Zytostatika und Hyperthermie besteht.

19. Verwendung nach einem der Ansprüche 1 bis 18, **dadurch gekennzeichnet, dass** der Virus, insbesondere der Adenovirus, ausgewählt ist aus der Gruppe, die aus AdΔ24, dl922-947, E1Ad/01/07, dl1119/1131, CB 016, dl520, und Viren, denen ein exprimiertes virales Onkogen fehlt, das zur Bindung eines funktionellen Rb-Tumorsuppressor-Genprodukts fähig ist, besteht.

20. Verwendung nach einem der Ansprüche 1 bis 19, **dadurch gekennzeichnet, dass** der Adenovirus E1B 19 kDa-defzient ist.

21. Verwendung einer Nukleinsäure codierend für einen Adenovirus wie in einem der Ansprüche 1 bis 20 definiert zur Herstellung eines Medikamentes, insbesondere zur Herstellung eines Medikamentes für die Behandlung von Tumoren, wobei das Medikament eine Kombination von mindestens zwei Mitteln umfasst, wobei ein jedes Mittel einzeln und unabhängig aus der Gruppe ausgewählt ist, die aus Cytostatika besteht, und wobei mindestens eines der Mittel den Transport von YB-1 in den Zellkern vermittelt.

22. Verwendung nach Anspruch 21, **dadurch gekenn-**

**zeichnet, dass** die Zellen, insbesondere die den Tumor oder Teile davon ausbildenden Zellen, eine Resistenz, insbesondere eine Mehrfachresistenz gegen pharmakologische Wirkstoffe, bevorzugterweise Antitumormittel, und bevorzugtererweise Zytostatika, aufweisen.

**23.** Verwendung nach Anspruch 21, **dadurch gekennzeichnet, dass** der Adenovirus so ausgebildet ist, dass die Replikation durch YB-1 über die Aktivierung des E2-Late-Promotors gesteuert wird, bevorzugterweise überwiegend über die Aktivierung des E2-Late-Promotors.

**24.** Verwendung eines mit YB-1 wechselwirkenden Mittels zur Charakterisierung von Zellen, Zellen eines Tumorgewebes oder Patienten, um zu bestimmen, ob diese(r) mit einem Adenovirus wie in einem der Ansprüche 1 bis 20 definiert kontaktiert und/oder behandelt werden sollen, wobei das mit YB-1 wechselwirkende Mittel ausgewählt ist aus der Gruppe, die aus Antikörpern, Aptameren, und Spiegelmeren besteht.

**25.** Verwendung eines Adenovirus nach einem der Ansprüche 1 bis 20, wobei der Virus eine für ein Transgen codierende Nukleinsäure umfasst.

**26.** Verwendung eines Adenovirus nach einem der Ansprüche 1 bis 20, wobei der Virus das Translations- und/oder das Transkriptionsprodukt eines Transgens umfasst.

**27.** Verwendung einer Nukleinsäure nach Anspruch 21, wobei die Nukleinsäure ein Transgen oder eine für ein Transgen codierende Nukleinsäure umfasst.

**28.** Verwendung nach einem der Ansprüche 25 bis 27, wobei das Transgen ausgewählt ist aus der Gruppe, die aus Prodruggenen, Zytokinen, Apoptose-induzierenden Genen, Tumorsuppressorgenen, Genen für Metalloproteinasen-Inhibitoren und Genen für Angiogene-Inhibitoren besteht.

**29.** Verwendung nach einem der Ansprüche 25 bis 27, wobei das Transgen ausgewählt ist aus der Gruppe, die aus Nukleinsäuren für siRNA, für Aptamere, für Antisense-Moleküle und für Ribozyme besteht, wobei die siRNA, die Aptamere, die Antisense-Moleküle und/oder die Ribozyme gegen ein Zielmolekül gerichtet ist.

**30.** Verwendung nach Anspruch 29, wobei das Zielmolekül ausgewählt ist aus der Gruppe, die aus Resistenz-relevanten Faktoren, Anti-Apoptose-Faktoren, Onkogenen, Angiogenese-Faktoren, DNA-Synthese-Enzymen, DNA-Reparaturenzymen, Wachstumsfaktoren, Rezeptoren für Wachstumsfaktoren, Transkriptionsfaktoren, Metalloproteinasen, insbesondere Matrix-Metalloproteinkinasen, und Plasminogenaktivator vom Urokinase-Typ besteht.

**31.** Verwendung nach einem der Ansprüche 1 bis 30, **dadurch gekennzeichnet, dass** mindestens zwei der Mittel an unterschiedlichen Zielmolekülen angreifen.

**32.** Verwendung nach Anspruch 31, **dadurch gekennzeichnet, dass** mindestens zwei der Mittel über einen unterschiedlichen Wirkmechanismus aktiv sind.

**33.** Verwendung nach einem der Ansprüche 1 bis 32, **dadurch gekennzeichnet, dass** mindestens ein Mittel die Infizierbarkeit einer Zelle erhöht, in der das Virus repliziert.

**34.** Verwendung nach einem der Ansprüche 1 bis 33, **dadurch gekennzeichnet, dass** das mindestens eine weitere Mittel die Verfügbarkeit einer Komponente der Zelle beeinflusst, bevorzugterweise die Verfügbarkeit der Komponente erhöht, wobei die Komponente die Aufnahme des Virus vermittelt.

**35.** Verwendung nach einem der Ansprüche 1 bis 34, **dadurch gekennzeichnet, dass** das mindestens eine den Transport von YB-1 in den Zellkern vermittelnde Mittel den Transport von YB-1 in den Zellkern erhöht

**36.** Verwendung nach einem der Ansprüche 1 bis 35, **dadurch gekennzeichnet, dass** mindestens ein Mittel ein Histon-Deacylase-Inhibitor ist.

**37.** Verwendung nach Anspruch 36, **dadurch gekennzeichnet, dass** der Histon-Deacylase-Inhibitor aus der Gruppe ausgewählt ist, die aus Trichostatin A, FR 901228, MS-27-275, NVP-LAQ824, PXD101 Apicidin und Scriptaid besteht.

**38.** Verwendung nach einem der Ansprüche 1 bis 36, **dadurch gekennzeichnet, dass** mindestens ein Mittel aus der Gruppe ausgewählt ist, die aus Trichostatin A, FR 901228, MS-27-275, NVP-LAQ824, PXD101 Apicidin und Scriptaid besteht.

**39.** Verwendung nach einem der Ansprüche 1 bis 38, **dadurch gekennzeichnet, dass** mindestens ein Mittel ein Topoisomerase-Inhibitor ist.

**40.** Verwendung nach Anspruch 39, **dadurch gekennzeichnet, dass** der Topoisomerase-Inhibitor aus der Gruppe ausgewählt ist, die aus Camptothecin, Irinotecan, Topotecan, DX-895If, SN-38, 9-aminocamptothecin, 9-nitrocamptothecin, Daunorubicn und Etoposid besteht.

**41.** Verwendung nach einem der vorhergehenden Ansprüche, **dadurch gekennzeichnet, dass** die Kombination aus Trichostatin A und Irinotecan besteht.

**42.** Verwendung nach einem der vorangehenden Ansprüche, **dadurch gekennzeichnet, dass** der Virus, insbesondere der Virus nach einem der vorangehenden Ansprüche, getrennt von den mindestens zwei Mitteln ist.

**43.** Verwendung nach Anspruch 42, **dadurch gekennzeichnet, dass** mindestens eine Einheitsdose des Virus von mindestens einer Einheitsdose von einem oder den mindestens zwei Mitteln getrennt ist.

**44.** Kit umfassend einen Virus wie in einem der vorangehenden Ansprüche definiert, und mindestens zwei Mittel, wobei ein jedes Mittel einzeln und unabhängig aus der Gruppe ausgewählt ist, die aus Cytostatika besteht, und wobei mindestens eines der Mittel den Transport von YB-1 in den Zellkern vermittelt.

**Claims**

**1.** Use of an adenovirus for the manufacture of a medicament, **characterised in that** the adenovirus is replication-deficient in cells which lack YB-1 in the nucleus, the adenovirus replicates in cells which have YB-1 in the nucleus, and the adenovirus encodes a viral oncogene protein E1A which transactivates at least one adenoviral gene, wherein the adenoviral gene is selected from the group consisting of E1B55kDa, E4orf6, E4orf3 and E3ADP, and wherein the medicament comprises a combination of at least two agents, wherein each agent is individually and independently selected from the group consisting of cytostatics, and wherein at least one of the agents mediates the transport of YB-1 into the nucleus.

**2.** Use according to claim 1, **characterised in that** the viral oncogene protein E1A does not induce nucleus localisation of YB-1.

**3.** Use according to any one of claims 1 to 2, **characterised in that** the medicament is for patients whose cells are Rb positive or Rb negative.

**4.** Use according to any one of claims 1 to 3, **characterised in that** the medicament is for the treatment of tumors.

**5.** Use according to claim 4, **characterised in that** the cells, preferably the cells forming the tumor or parts thereof, are resistant, preferably multiple resistant against pharmacological agents, preferably anti-tumor agents and more preferably cytostatics.

**6.** Use according to claim 5, **characterised in that** the cells express, preferably over-express, the membrane-anchored transport protein P glycoprotein.

**7.** Use according to any one of claims 1 to 6, **characterised in that** the cells are p53-positive or p53-negative.

**8.** Use according to any one of claims 2 to 7, **characterised in that** the oncogene protein exhibits one or several mutations or deletions compared to the wild type oncogene protein E1A, wherein the deletion is preferably one selected from the group comprising deletions of the CR3 region and deletions of the N-terminus and deletions of the C-terminus.

**9.** Use according to claim 8, **characterized in that** E1A oncogene protein is capable of binding to Rb.

**10.** Use according to any one of claims 2 to 7, **characterised in that** the oncogene protein comprises one or several mutations or deletions compared to the wild type oncogene protein, wherein the deletion is preferably a deletion in the CR1 region and/or CR2 region.

**11.** Use according to claim 10, **characterised in that** the oncogene protein E1A is incapable of binding to Rb.

**12.** Use according to any one of claims 1 to 11, **characterised in that** the viral oncogene protein is under the control of a tissue- and/or tumor-specific promoter.

**13.** Use according to any one of claims 1 to 12, **characterised in that** the the adenovirus codes for YB-1.

**14.** Use according to claim 13, **characterised in that** YB- 1 is under the control of a tissue-specific and/or tumor-specific promoter.

**15.** Use according to any one of claims 1 to 14, **characterised in that** the adenovirus codes for at least one protein, wherein the protein is selected from the group consisting of E4orf6, E4orf3, EIB55k and adenoviral E3ADP protein.

**16.** Use according to any one of claims 1 to 15, **characterised in that** the cells have YB-1 in the nucleus, preferably the cells forming the tumor or part thereof have YB-1 in the nucleus.

**17.** Use according to any one of claims 1 to 16, **characterised in that** the tumor comprises YB-1 in the nucleus after induction of the transport of YB-1 into the nucleus.

**18.** Use according to claim 17, **characterised in that** the transport of YB-1 into the nucleus is triggered by at least one measure selected from the group consisting of irradiation, administration of cytostatics and hyperthermia.

**19.** Use according to any one of claims 1 to 18, **characterised in that** the virus, preferably the adenovirus, is selected from the group consisting of AdΔ24, dl922-947, E1Ad/01/07, dl1119/1131, CB 016, dl520 and viruses lacking an expressed viral oncogene which is capable of binding a functional Rb tumor suppressor gene product.

**20.** Use according to any one of claims 1 to 19, **characterized in that** the adenovirus is E1B 19 kDa-deficient.

**21.** Use of a nucleic acid coding for an adenovirus as defined in any one of claims 1 to 20, for the manufacture of a medicament, preferably for the manufacture of a medicament for the treatment of tumors, wherein the medicament comprises a combination of at least two agents, wherein each agent is individually and independently selected from the group consisting of cytostatics, and wherein at least one of the agents mediates the transport of YB-1 into the nucleus.

**22.** Use according to claim 21, **characterised in that** the cells, preferably the cells forming the tumor or parts thereof, have a resistance, preferably a multiple resistance against pharmacologically active agents, preferably anti-tumor agents and more preferably cytostatics.

**23.** Use according to claim 21, **characterised in that** the adenovirus is designed such that the replication is controlled by YB-1 through the activation of the E2-late promoter, preferably predominantly through the activation of the E2-late promoter.

**24.** Use of a compound interacting with YB-1 for the characterisation of cells, cells of a tumor tissue or patients, in order to determine whether these shall be contacted with and/or treated with an adenovirus as defined in any one of claims 1 to 20, wherein the compound is selected from the group consisting of antibodies, aptamers and spiegelmers.

**25.** Use of an adenovirus according to any one of claims 1 to 20, wherein the virus comprises a nucleic acid coding for a transgene.

**26.** Use of an adenovirus according to any one of claims 1 to 20, wherein the virus comprises the translation and/or transcription product of a transgene.

**27.** Use according to claim 21, wherein the nucleic acid comprises a transgene or a nucleic acid coding for a transgene.

**28.** Use according to any one of claims 25 to 27, wherein the transgene is selected from the group consisting of prodrug genes, cytokines, apoptosis-inducing genes, tumor suppressor genes, genes for metalloproteinase inhibitors and genes for angiogenesis inhibitors.

**29.** Use according to any one of claims 25 to 27, wherein the transgene is selected from the group consisting of nucleic acids for siRNA, for aptamers, for antisense molecules and for ribozymes, wherein the siRNA, the aptamer, the antisense molecule and/or the ribozyme is targeting a target molecule.

**30.** Use according to claim 29, wherein the target molecule is selected from the group consisting of resistance relevant factors, anti-apoptosis factors, oncogenes, angiogenesis factors, DNA synthesis enzymes, DNA repair enzymes, growth factors, receptors for growth factors, transcription factors, metalloproteinases, preferably matrix metalloprotein kinases, and plasminogen activator of the urokinase type.

**31.** Use according to any one of claims 1 to 30, **characterized in that** at least two of the agents target different target molecules.

**32.** Use according to claim 31, **characterized in that** at least two of the agents act through different modes of action.

**33.** Use according to any one of claims 1 to 32, **characterized in that** at least one agent increases the capacity of a cell to be infected, wherein the cell is a cell in which the virus replicates.

**34.** Use according to any one of claims 1 to 33, **characterized in that** the at least one further agent influences the availability of a component of the cell, preferably increases the availability of the component, wherein the component mediates uptake of the virus.

**35.** Use according to any one of claims 1 to 34, **characterized in that** the at least one agent mediating the transport of YB-1 into the nucleus increases the transport of YB-1 into the nucleus.

**36.** Use according to any one of claims 1 to 35, **characterized in that** at least one agent is a histone deacylase inhibitor.

**37.** Use according to claim 36, **characterized in that** the histone deacylase inhibitor is selected from the group consisting of Trichostatin A, FR 901228, MS-27-275, NVP-LAQ824, PXD101 Apicidin and Scriptaid.

**38.** Use according to any one of claims 1 to 36, **characterized in that** at least one agent is selected from the group consisting of Trichostatin A, FR 901228, MS-27-275, NVP-LAQ824, PXD101 Apicidin and Scriptaid.

**39.** Use according to any one of claims 1 to 38, **characterized in that** at least one agent is a topoisomerase inhibitor.

**40.** Use according to claim 39, **characterized in that** the topoisomerase inhibitor is selected from the group consisting of Camptothecin, Irinotecan, Topotecan, DX-895If, SN-38, 9-aminocamptothecin, 9-nitrocamptothecin, Daunorubicn and Etoposid.

**41.** Use according to any one of the preceding claims, **characterised in that** the combination consists of Trichostatin A and Irinotecan.

**42.** Use according to any one of the preceding claims, **characterised in that** the virus, preferably the virus according to any one of the preceding claims, is separated from the at least two agents.

**43.** Use according to claim 42, **characterized in that** at least one unit dose of the virus is separated from at least one unit dose of one of or of the at least two agents.

**44.** Kit comprising a virus as defined in any one of the preceding claims, and at least two agents, wherein each of the agents is individually and independently selected from the group consisting of cytostatics, and wherein at least one of the agents mediates the transport of YB-1 into the nucleus.

## Revendications

**1.** Utilisation d'un adénovirus pour la préparation d'un médicament, **caractérisée en ce que** l'adénovirus est déficient en réplication dans des cellules qui ne présentent pas YB-1 dans le noyau, l'adénovirus se réplique dans des cellules qui présentent YB-1 dans le noyau et l'adénovirus code pour une protéine oncogène virale E1A, qui transactive au moins un gène adénoviral, le gène adénoviral étant choisi dans le groupe consistant en E1B55kDa, E4orf6, E4orf3 et E3ADP et le médicament comprenant une combinaison d'au moins deux agents, dans laquelle chaque agent est choisi, individuellement et indépendamment, dans le groupe consistant en les cytostatiques, et dans laquelle au moins un des agents favorise le transport d'YB-1 dans le noyau cellulaire.

**2.** Utilisation selon la revendication 1, **caractérisée en ce que** l'oncoprotéine virale E1A n'induit pas la localisation nucléaire d'YB-1.

**3.** Utilisation selon l'une quelconque des revendications 1 à 2, **caractérisée en ce que** le médicament est destiné aux patients dont les cellules sont Rb-positives ou Rb-négatives.

**4.** Utilisation selon l'une quelconque des revendications 1 à 3, **caractérisée en ce que** le médicament est destiné au traitement de tumeurs.

**5.** Utilisation selon la revendication 4, **caractérisée en ce que** les cellules, en particulier les cellules formant la tumeur ou des parties de celle-ci, présentent une résistance, en particulier une résistance multiple aux substances actives pharmacologiques, de préférence aux agents antitumoraux et plus préférablement aux cytostatiques.

**6.** Utilisation selon la revendication 5, **caractérisée en ce que** les cellules présentent une expression, de préférence une surexpression de la protéine membranaire de transport glycoprotéine P.

**7.** Utilisation selon l'une quelconque des revendications 1 à 6, **caractérisée en ce que** les cellules sont p53-positives ou p53-négatives.

**8.** Utilisation selon l'une quelconque des revendications 2 à 7, **caractérisée en ce que** la protéine oncogène présente, par rapport à la protéine oncogène E1A sauvage, une ou plusieurs mutations ou délétions, la délétion étant de préférence une délétion qui est choisie dans le groupe comprenant les délétions de la région CR3 et les délétions N-terminal et les délétions C-terminal.

**9.** Utilisation selon la revendication 8, **caractérisée en ce que** la protéine oncogène E1A peut se lier à Rb.

**10.** Utilisation selon l'une quelconque des revendications 2 à 7, **caractérisée en ce que** la protéine oncogène présente, par rapport à la protéine oncogène sauvage, une ou plusieurs mutations ou délétions, la délétion étant de préférence une délétion dans la région CR1 et/ou dans la région CR2.

**11.** Utilisation selon la revendication 10, **caractérisée en ce que** la protéine oncogène E1A n'est pas en mesure de se lier à Rb.

**12.** Utilisation selon l'une quelconque des revendications 1 à 11, **caractérisée en ce que** la protéine oncogène virale est sous le contrôle d'un promoteur spécifique d'un tissu et/ou d'une tumeur.

**13.** Utilisation selon l'une quelconque des revendications 1 à 12, **caractérisée en ce que** l'adénovirus code pour YB-1.

**14.** Utilisation selon la revendication 13, **caractérisée en ce qu'**YB-1 est sous le contrôle d'un promoteur spécifique d'un tissu et/ou d'une tumeur.

**15.** Utilisation selon l'une quelconque des revendications 1 à 14, **caractérisée en ce que** l'adénovirus code pour au moins une protéine choisie dans le groupe consistant en E4orf6, E4orf3, E1B55k et la protéine E3ADP adénovirale.

**16.** Utilisation selon l'une quelconque des revendications 1 à 15, **caractérisée en ce que** les cellules présentent YB-1 dans le noyau, en particulier les cellules formant la tumeur ou une partie de celle-ci présentent YB-1 dans le noyau.

**17.** Utilisation selon l'une quelconque des revendications 1 à 16, **caractérisée en ce que** la tumeur contient YB-1 dans le noyau après l'induction du transport d'YB-1 dans le noyau.

**18.** Utilisation selon la revendication 17, **caractérisée en ce que** le transport d'YB-1 est déclenché dans le noyau par au moins une mesure choisie dans le groupe consistant en une irradiation, l'administration de cytostatiques et une hyperthermie.

**19.** Utilisation selon l'une quelconque des revendications 1 à 18, **caractérisée en ce que** le virus, en particulier l'adénovirus, est choisi dans le groupe consistant en AdΔ24, dl922-947, E1Ad/01/07, dl1119/1131, CB 016, dl520 et les virus auxquels il manque un oncogène viral exprimé, et qui est capable de se lier à un produit génique fonctionnel, suppresseur de tumeur Rb.

**20.** Utilisation selon l'une quelconque des revendications 1 à 19, **caractérisée en ce que** l'adénovirus est déficient en E1B 19 kDa.

**21.** Utilisation d'un acide nucléique codant pour un adénovirus tel que défini dans l'une quelconque des revendications 1 à 20 pour la préparation d'un médicament, en particulier pour la préparation d'un médicament pour le traitement de tumeurs, le médicament comprenant une combinaison d'au moins deux agents, dans laquelle chaque agent est choisi, individuellement et indépendamment, dans le groupe consistant en les cytostatiques et dans laquelle au moins un des agents favorise le transport d'YB-1 dans le noyau cellulaire.

**22.** Utilisation selon la revendication 21, **caractérisée en ce que** les cellules, en particulier les cellules formant la tumeur ou des parties de celle-ci, présentent une résistance, en particulier une résistance multiple, aux substances actives pharmacologiques, de préférence aux agents antitumoraux et plus préférablement aux cytostatiques.

**23.** Utilisation selon la revendication 21, **caractérisée en ce que** l'adénovirus est formé de manière telle que la réplication d'YB-1 est dirigée par l'activation du promoteur tardif E2, de préférence principalement par l'activation du promoteur tardif E2.

**24.** Utilisation d'un agent interagissant avec YB-1 pour la caractérisation de cellules, de cellules d'un tissu tumoral ou de patients, en vue de déterminer si ceux-ci/celles-ci doivent être mis(es) en contact et/ou traité(e)s avec un adénovirus tel que défini dans l'une quelconque des revendications 1 à 20, l'agent interagissant avec YB-1 étant choisi dans le groupe consistant en les anticorps, les aptamères et les Spiegelmer.

**25.** Utilisation d'un adénovirus selon l'une quelconque des revendications 1 à 20, dans laquelle le virus comprend un acide nucléique codant pour un transgène.

**26.** Utilisation d'un adénovirus selon l'une quelconque des revendications 1 à 20, dans laquelle le virus comprend le produit de traduction et/ou de transcription d'un transgène.

**27.** Utilisation d'un acide nucléique selon la revendication 21, dans laquelle l'acide nucléique comprend un transgène ou un acide nucléique codant pour un transgène.

**28.** Utilisation selon l'une quelconque des revendications 25 à 27, le transgène étant choisi dans le groupe consistant en les gènes de prodogue, les cytokines, les gènes inducteurs de l'apoptose, les gènes suppresseurs de tumeur, les gènes d'inhibiteurs de métalloprotéinases et les gènes d'inhibiteurs d'angiogenèse.

**29.** Utilisation selon l'une quelconque des revendications 25 à 27, dans laquelle le transgène est choisi dans le groupe consistant en les acides nucléiques pour l'ARNsi, pour les aptamères, pour les molécules antisens et pour les ribozymes, l'ARNsi, les aptamères, les molécules antisens et/ou les ribozymes étant dirigés contre une molécule cible.

**30.** Utilisation selon la revendication 29, dans laqelle la molécule cible est choisie dans le groupe consistant en les facteurs de résistance, les facteurs anti-apoptose, les oncogènes, les facteurs d'angiogenèse, les enzymes de synthèse d'ADN, les enzymes de réparation d'ADN, les facteurs de croissance, les récepteurs des facteurs de croissance, les facteurs de transcription, les métalloprotéinases, en particulier les métalloprotéine-kinases matricielles et l'activateur du plasminogène de type urokinase.

**31.** Utilisation selon l'une quelconque des revendications 1 à 30, **caractérisée en ce qu'**au moins deux des agents attaquent des molécules cibles différentes.

**32.** Utilisation selon la revendication 31, **caractérisée en ce qu'**au moins deux des agents sont actifs via un mécanisme d'action différent.

**33.** Utilisation selon l'une quelconque des revendications 1 à 32, **caractérisée en ce qu'**au moins un agent augmente la sensibilité à l'infection d'une cellule dans laquelle le virus se réplique.

**34.** Utilisation selon l'une quelconque des revendications 1 à 33, **caractérisée en ce qu'**au moins un autre agent influence la disponibilité d'un constituant cellulaire, de préférence augmente la disponibilité des composants, ces derniers favorisant l'absorption du virus.

**35.** Utilisation selon l'une quelconque des revendications 1 à 34, **caractérisée en ce que** moins un agent favorisant le transport d'YB-1 dans le noyau cellulaire augmente le transport d'YB-1 dans le noyau cellulaire.

**36.** Utilisation selon l'une quelconque des revendications 1 à 35, **caractérisée en ce qu'**au moins un agent est un inhibiteur de l'histone-désacylase.

**37.** Utilisation selon la revendication 36, **caractérisée en ce que** l'inhibiteur de l'histone-désacylase est choisi dans le groupe consistant en la trichostatine A, FR 901228, MS-27-275, NVP-LAQ824, PXD101, l'apicidine et le scriptaïde.

**38.** Utilisation selon l'une quelconque des revendications 1 à 36, **caractérisée en ce qu'**au moins un agent est choisi dans le groupe consistant en la trichostatine A, FR 901228, MS-27-275, NVP-LAQ824, PXD101, l'apicidine et le scriptaïde.

**39.** Utilisation selon l'une quelconque des revendications 1 à 38, **caractérisée en ce qu'**au moins un agent est un inhibiteur de la topoisomérase.

**40.** Utilisation selon la revendication 39, **caractérisée en ce que** l'inhibiteur de la topoisomérase est choisi dans le groupe constitué par la camptothécine, l'irinotécan, le topotécan, DX-895If, SN-38, la 9-aminocamptothécine, la 9-nitrocamptothécine, la daunorubicine et l'étoposide.

**41.** Utilisation selon l'une quelconque des revendications précédentes, **caractérisée en ce que** la combinaison est constituée de trichostatine A et d'irinotécan.

**42.** Utilisation selon l'une quelconque des revendications précédentes, **caractérisée en ce que** le virus, en particulier le virus selon l'une quelconque des revendications précédentes, est séparé desdits au moins deux agents.

**43.** Utilisation selon la revendication 42, **caractérisée en ce qu'**au moins une dose unitaire du virus est séparée d'au moins une dose unitaire d'un ou desdits au moins deux agents.

**44.** Kit comprenant un virus tel que défini dans l'une quelconque des revendications précédentes et au moins deux agents, dans lequel chaque agent est choisi, individuellement et indépendamment, dans le groupe consistant en les cytostatiques, et dans lequel au moins un des agents favorise le transport d'YB-1 dans le noyau cellulaire.

Fig. 1

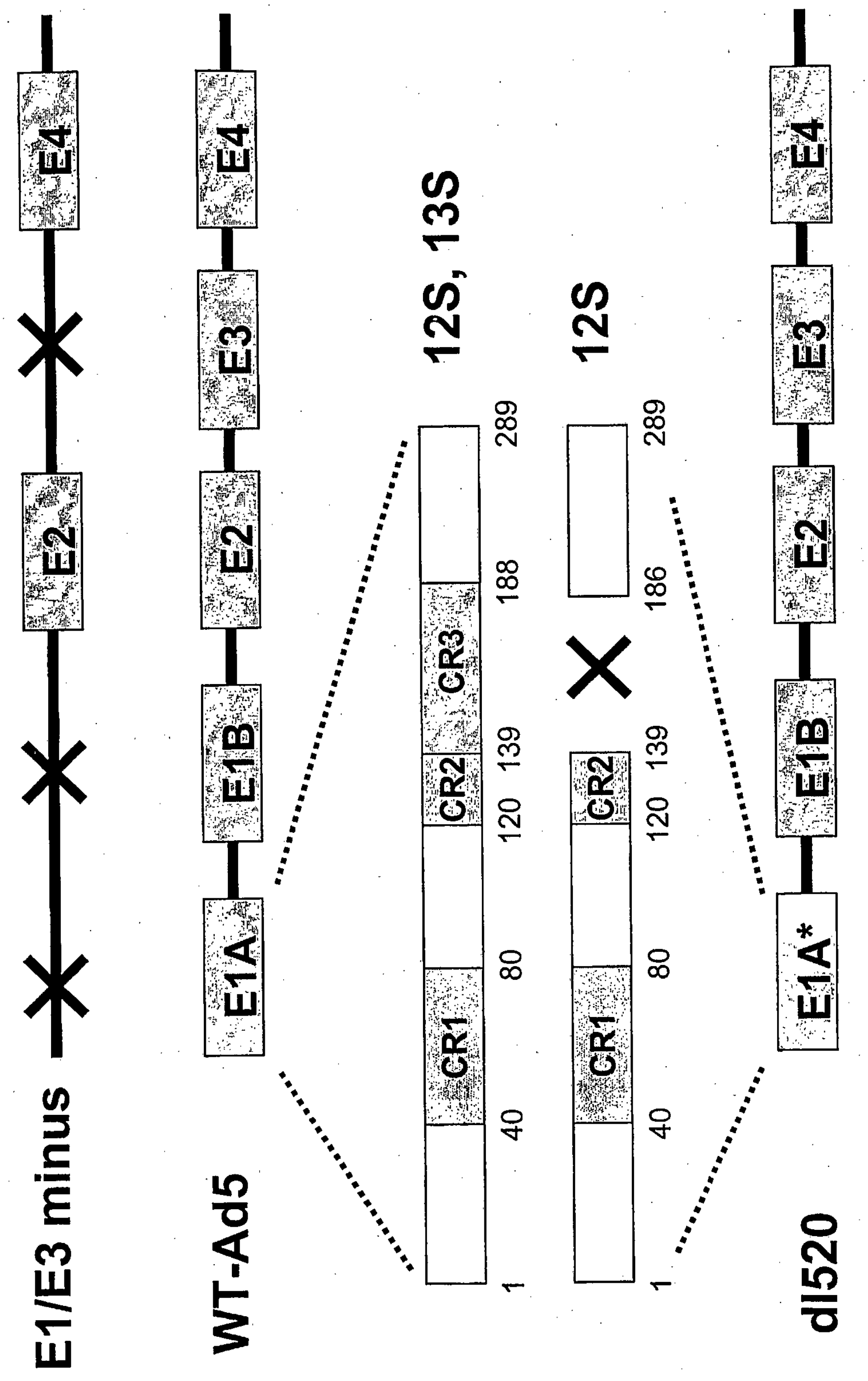
E1/E3 minus
E2
E4
WT-Ad5
E1A
E1B
E2
E3
E4
CR1
CR2
CR3
1
40
80
120
139
188
289
12S, 13S
CR1
CR2
1
40
80
120
139
186
289
12S
dl520
E1A*
E1B
E2
E3
E4

Fig. 2

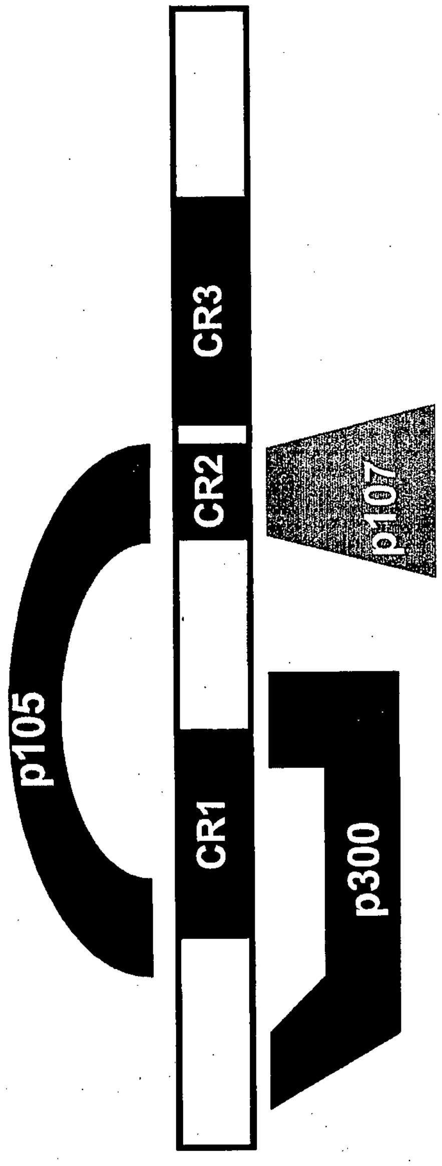


WT-E1A

Fig. 3

E1/E3-minus Ad5

40 pfu/Zelle 10 pfu/Zelle Nicht infiziert

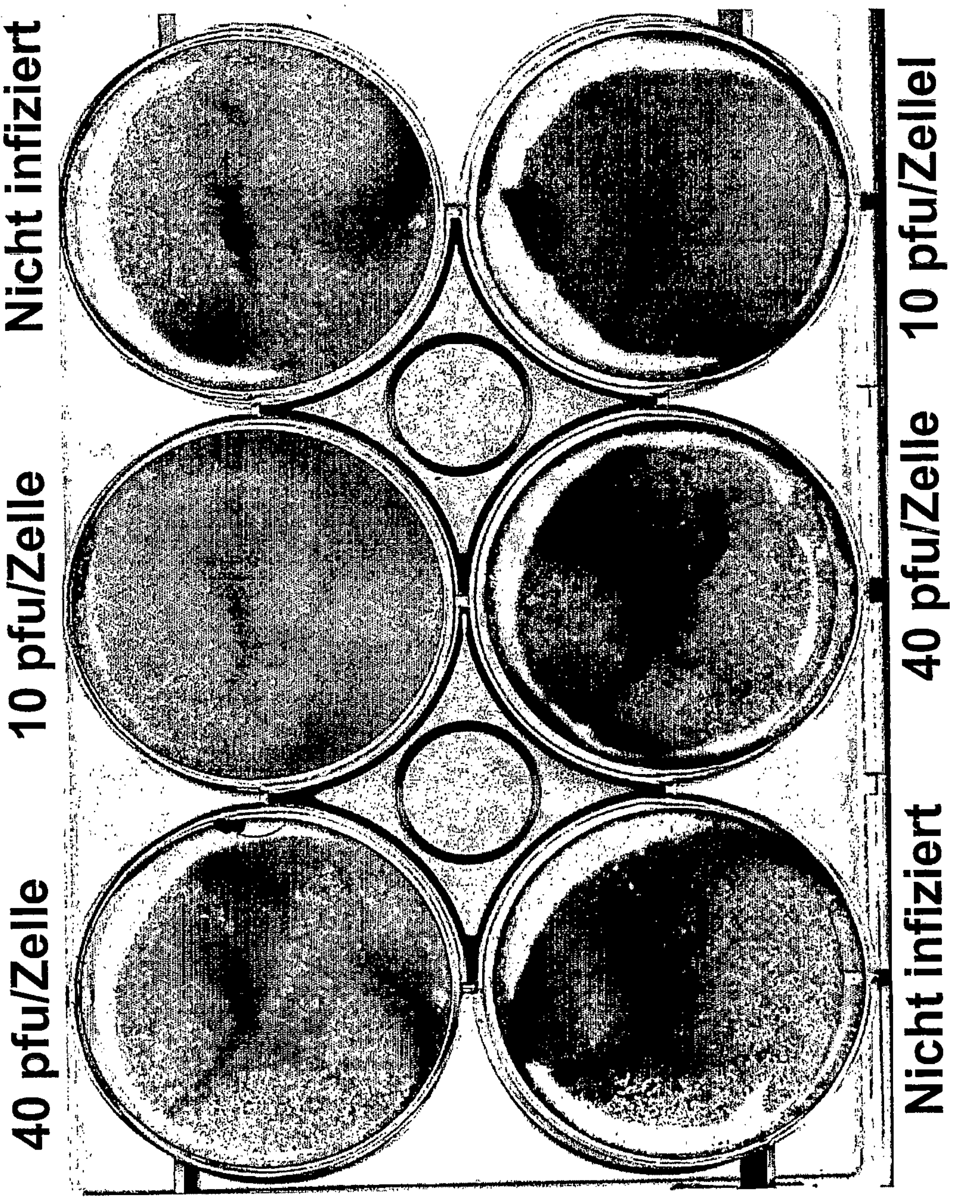

Nicht infiziert 40 pfu/Zelle 10 pfu/Zellel

dl520

Fig. 4

<u>E1/E3-minus Ad5</u>

40 pfu/Zelle 10 pfu/Zelle Nicht infiziert

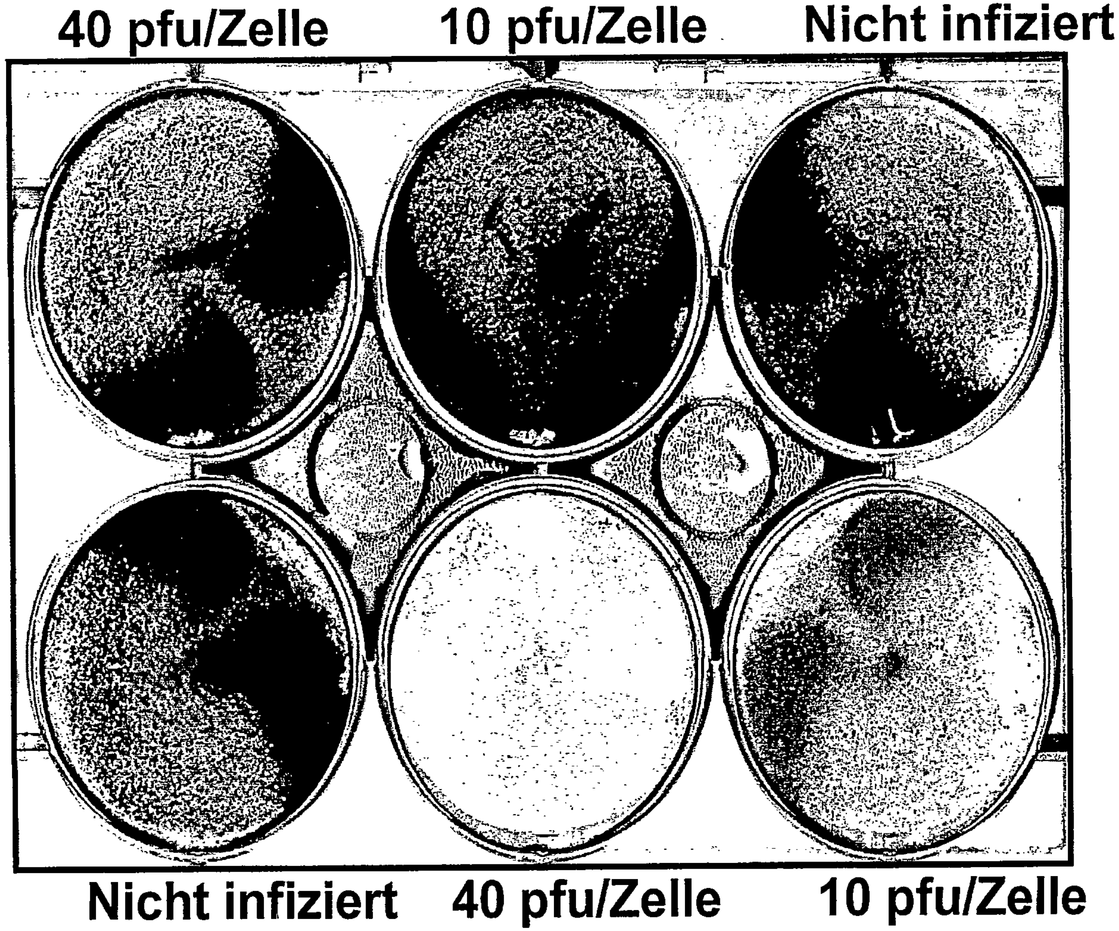

Nicht infiziert 40 pfu/Zelle 10 pfu/Zelle

<u>dl520</u>

Fig. 5

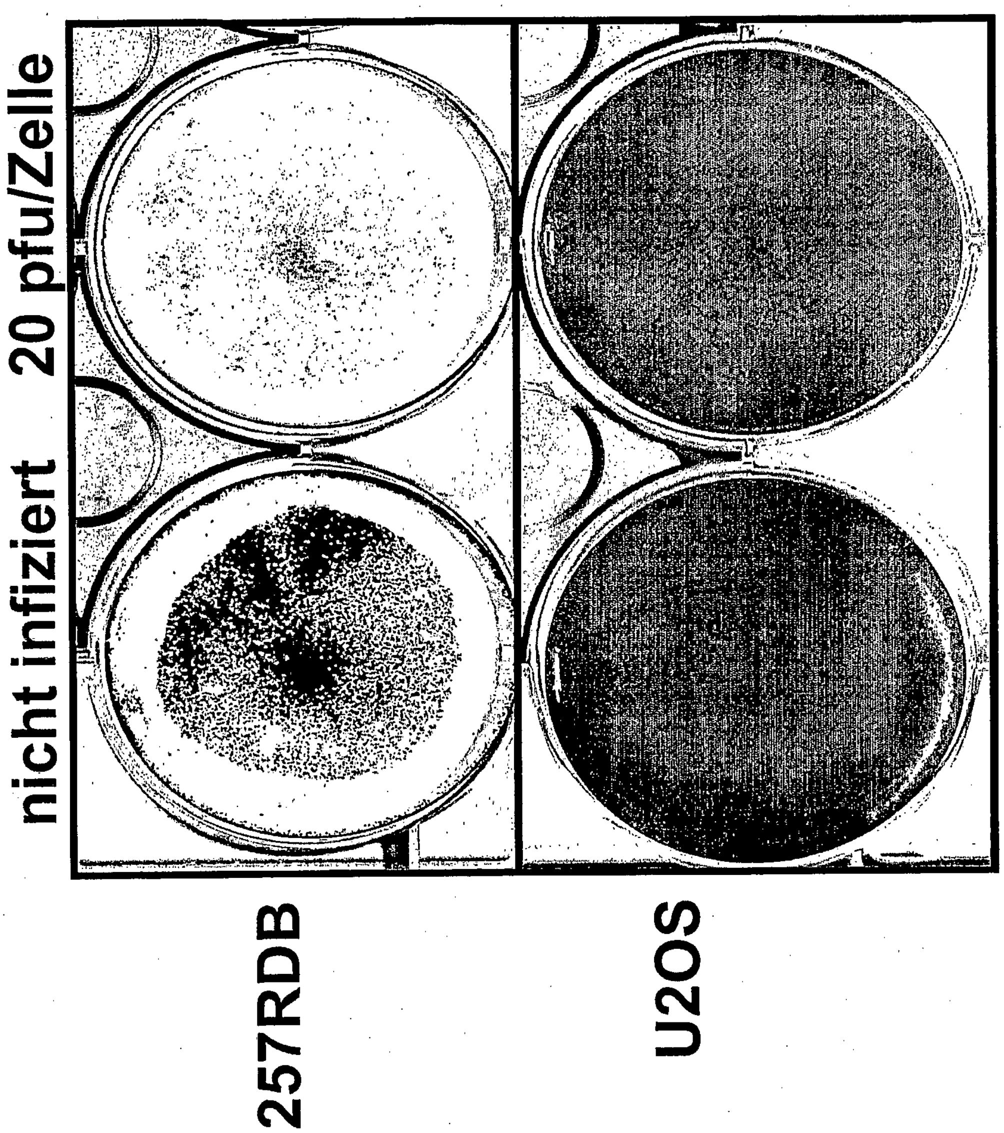

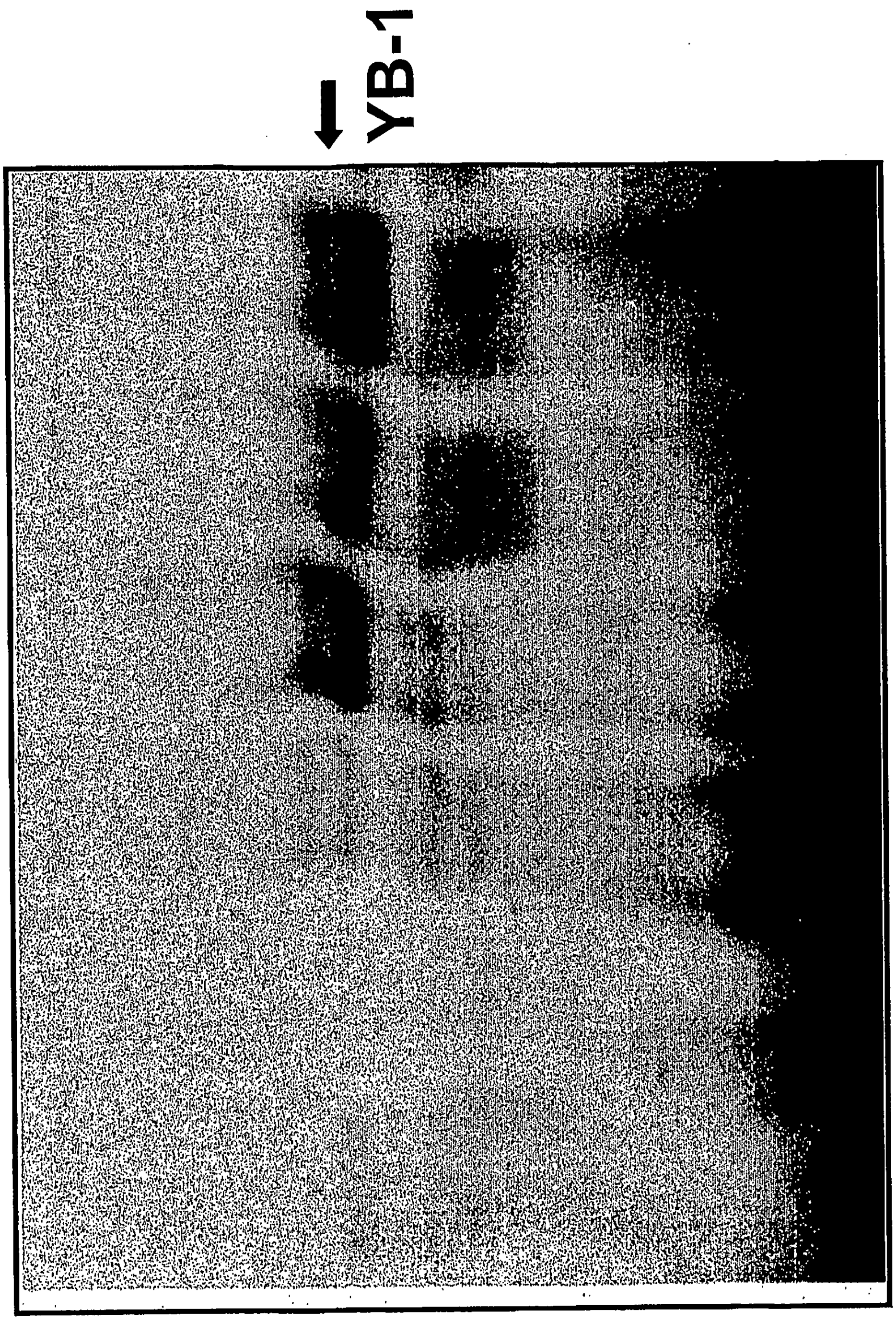


Fig. 6

Fig. 7

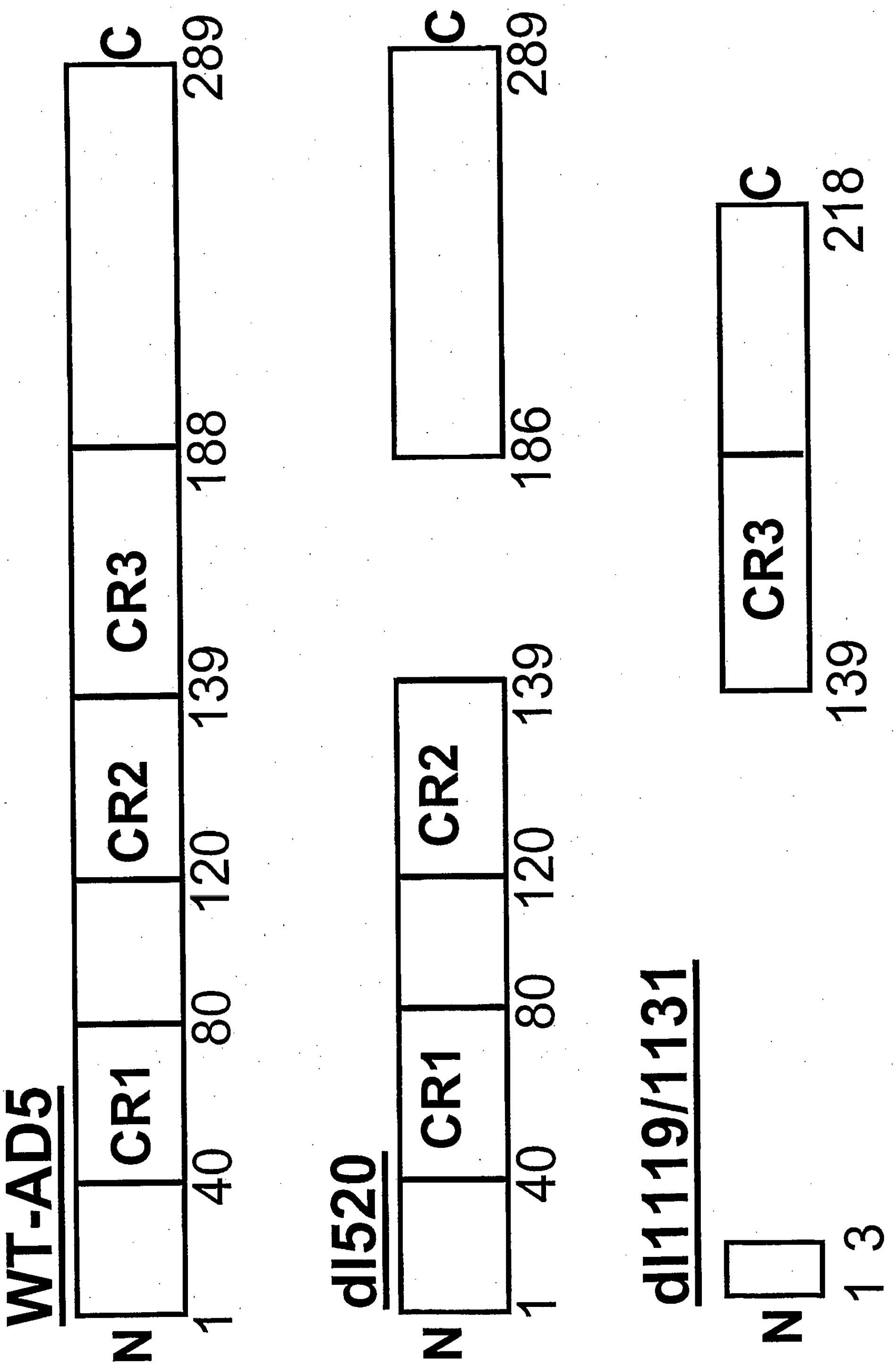
WT-AD5
N
CR1
CR2
CR3
C
1
40
80
120
139
188
289
dl520
N
CR1
CR2
C
1
40
80
120
139
186
289
dl1119/1131
N
CR3
C
1 3
139
218

Fig. 8

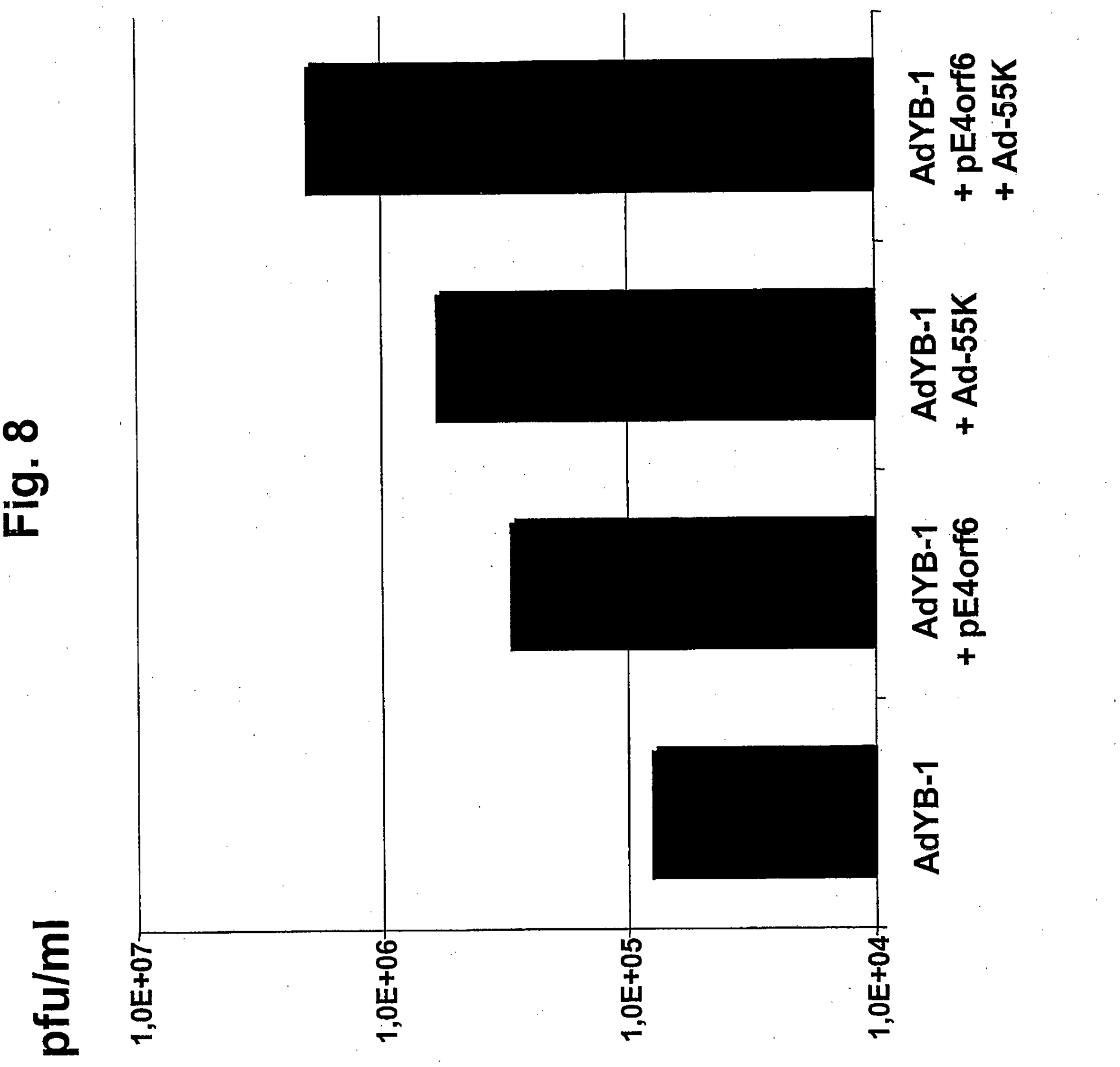
pfu/ml
1,0E+07
1,0E+06
1,0E+05
1,0E+04
AdYB-1
AdYB-1 + pE4orf6
AdYB-1 + Ad-55K
AdYB-1 + pE4orf6 + Ad-55K

Fig. 9

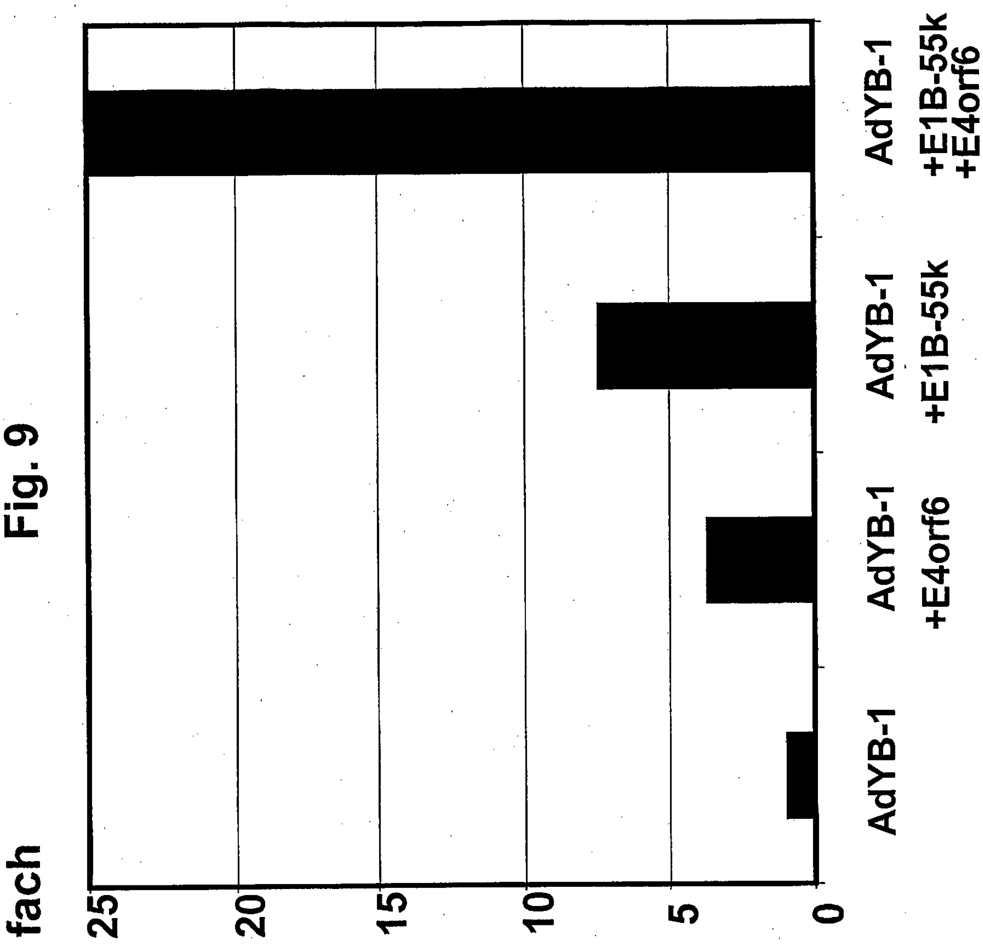
fach
0
5
10
15
20
25
AdYB-1
AdYB-1 +E4orf6
AdYB-1 +E1B-55k
AdYB-1 +E1B-55k +E4orf6

Fig. 10

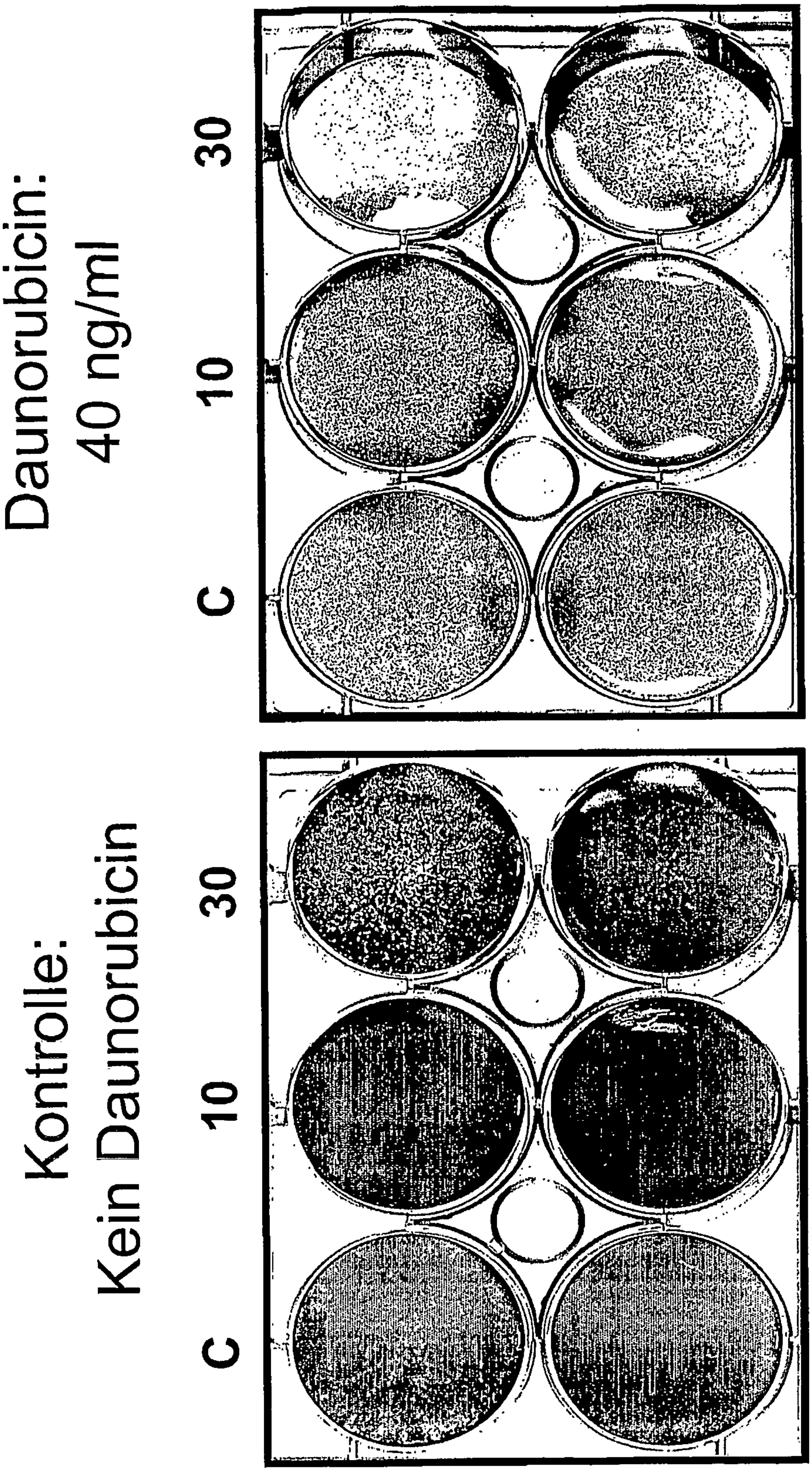
Daunorubicin:
40 ng/ml
C
10
30
Kontrolle:
Kein Daunorubicin
C
10
30

Fig. 11

Kontrolle:
Kein Daunorubicin

K 10 30

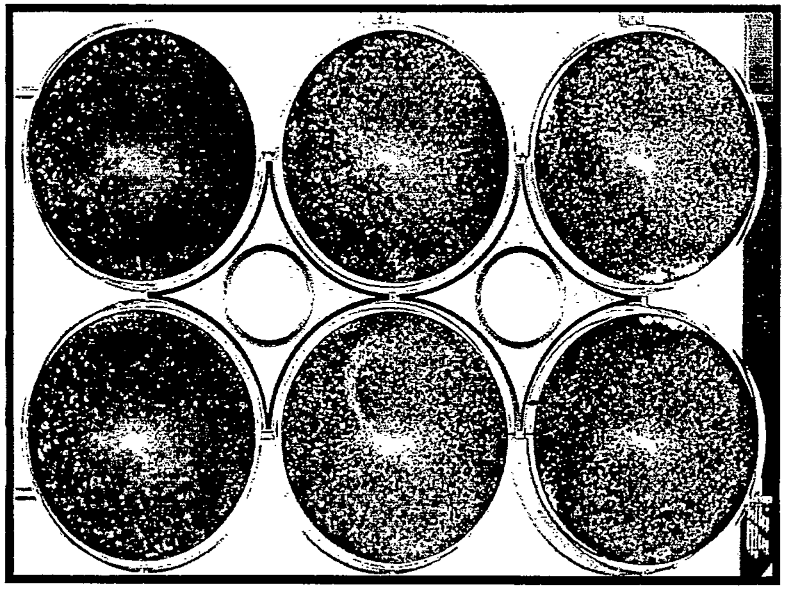

Daunorubicin:
40 ng/ml

K 10 30

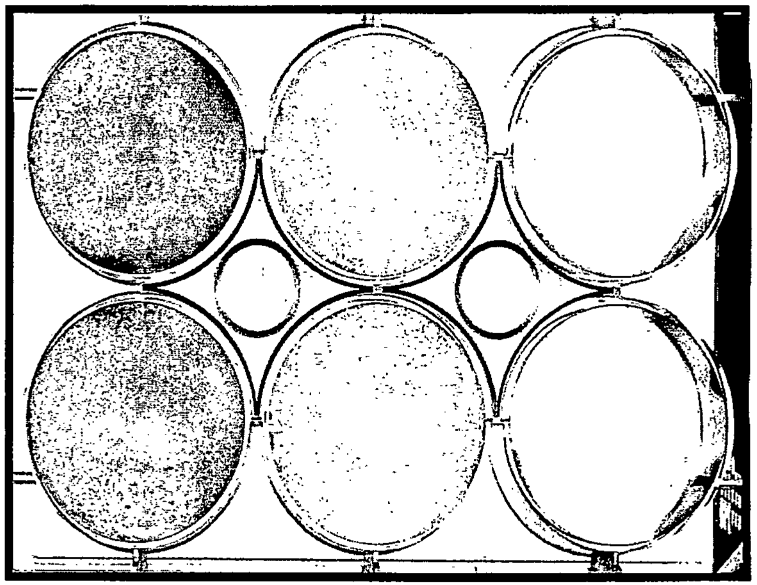

Fig. 12

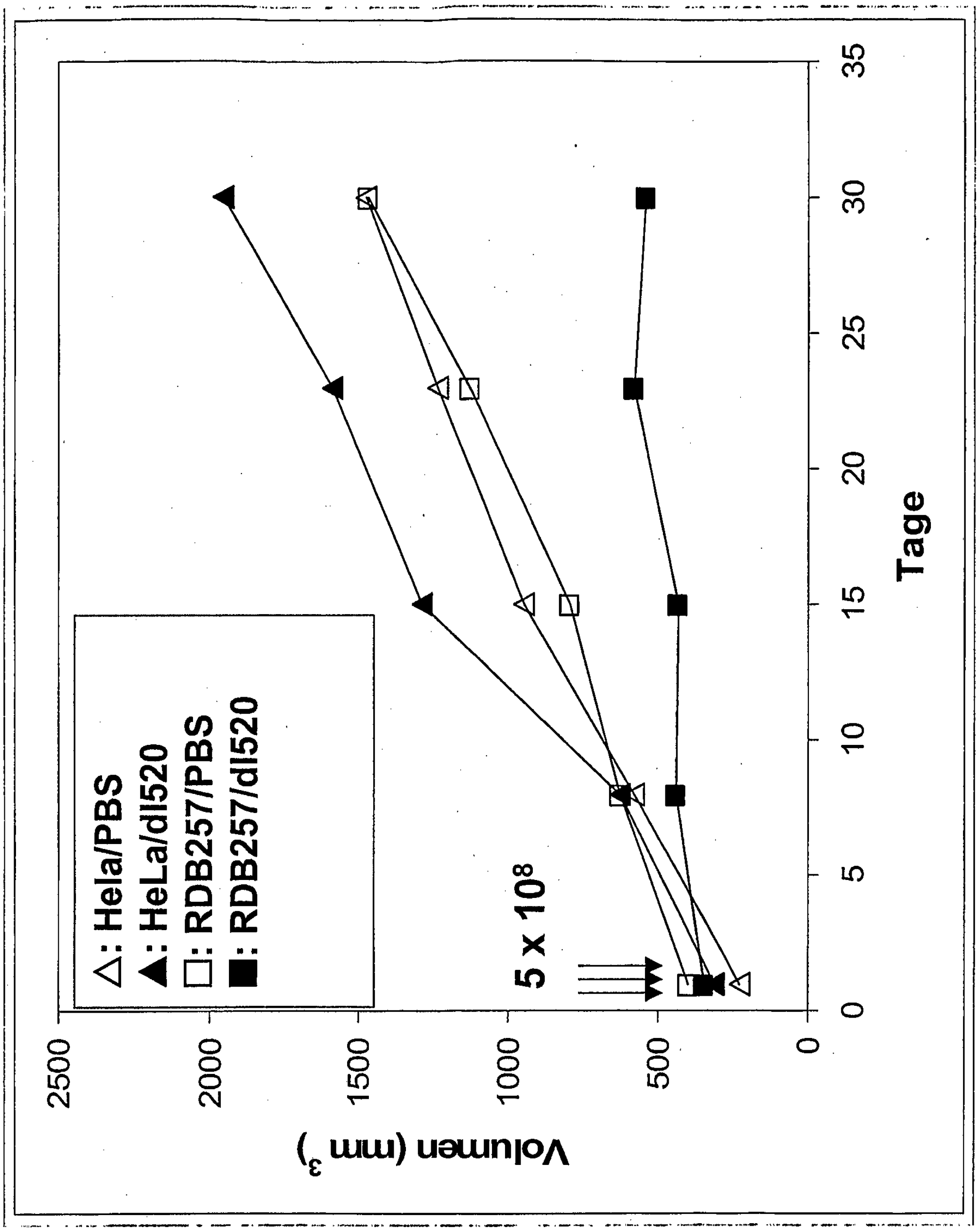
△: Hela/PBS
▲: HeLa/dl520
□: RDB257/PBS
■: RDB257/dl520
5 x $10^8$
Volumen (mm $^3$)
0
500
1000
1500
2000
2500
Tage
0
5
10
15
20
25
30
35

5 x $10^8$ dl520

PBS

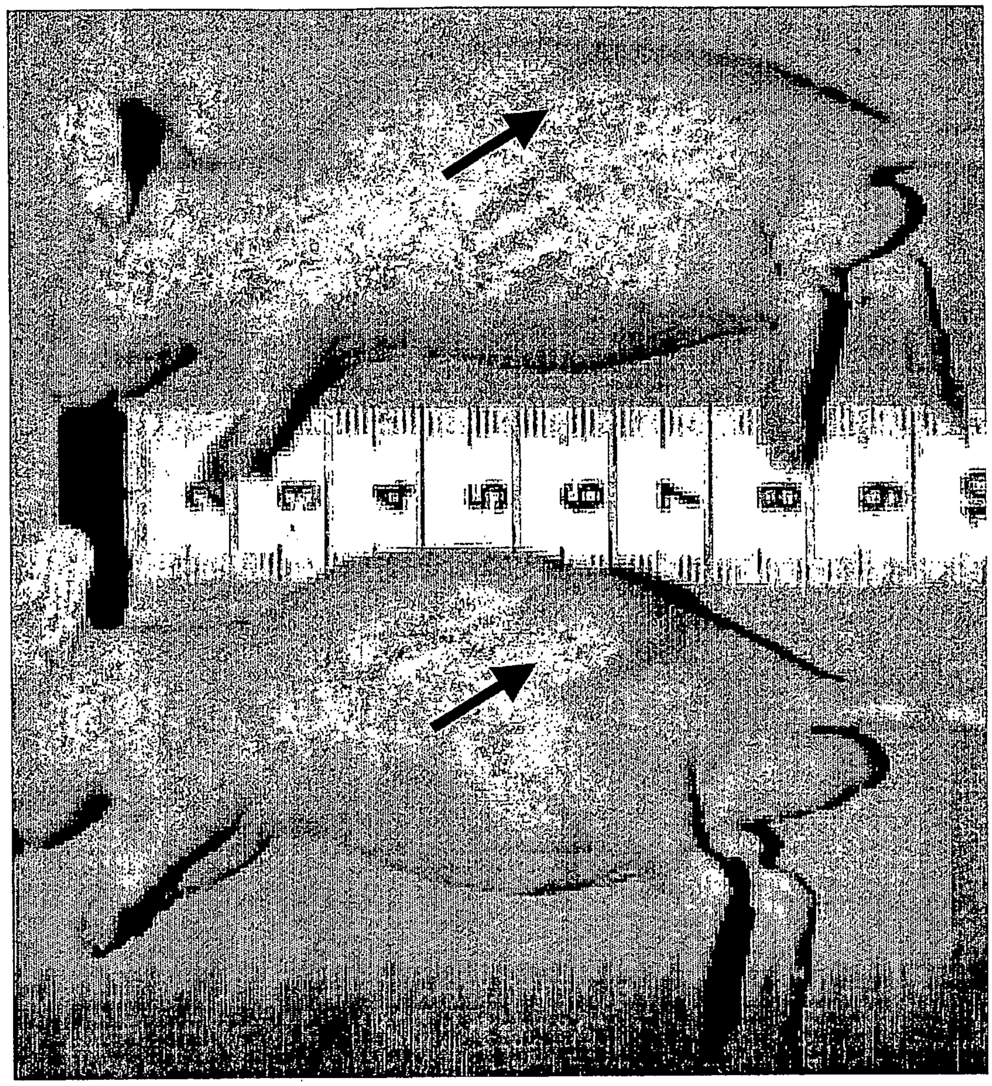

Fig. 13

Fig. 14

257RDB YB-1 Kernpositiv

HeLa YB-1 Kernnegativ

1 2 3 4 5 6 7 8

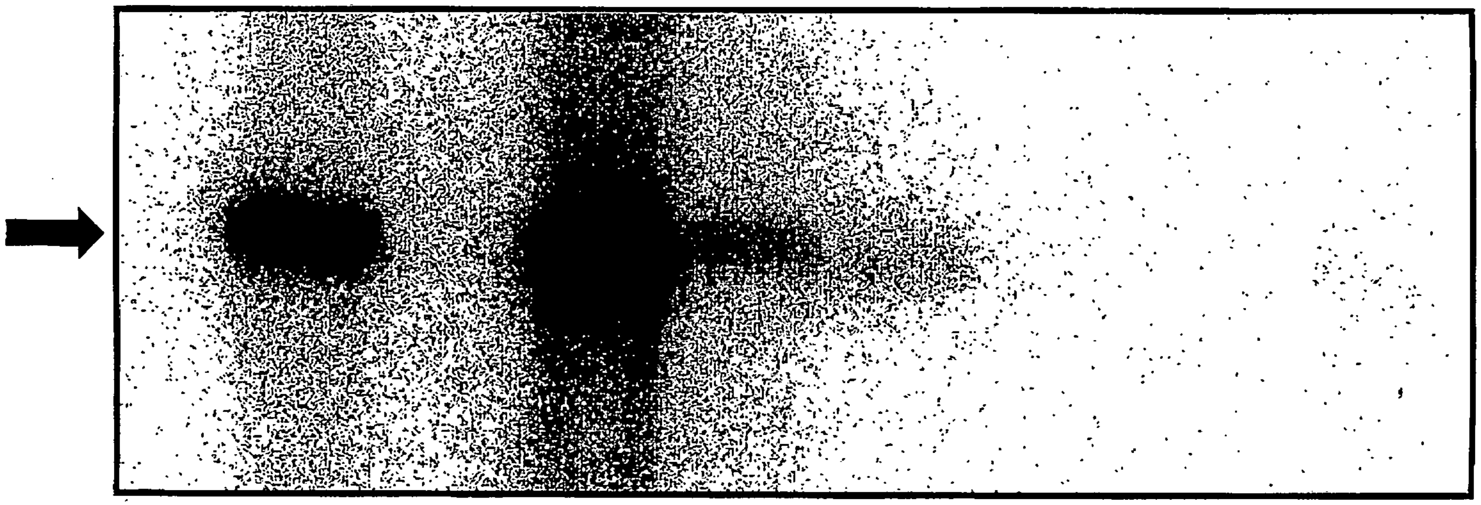

Southern Blot Analysis

Fig. 15

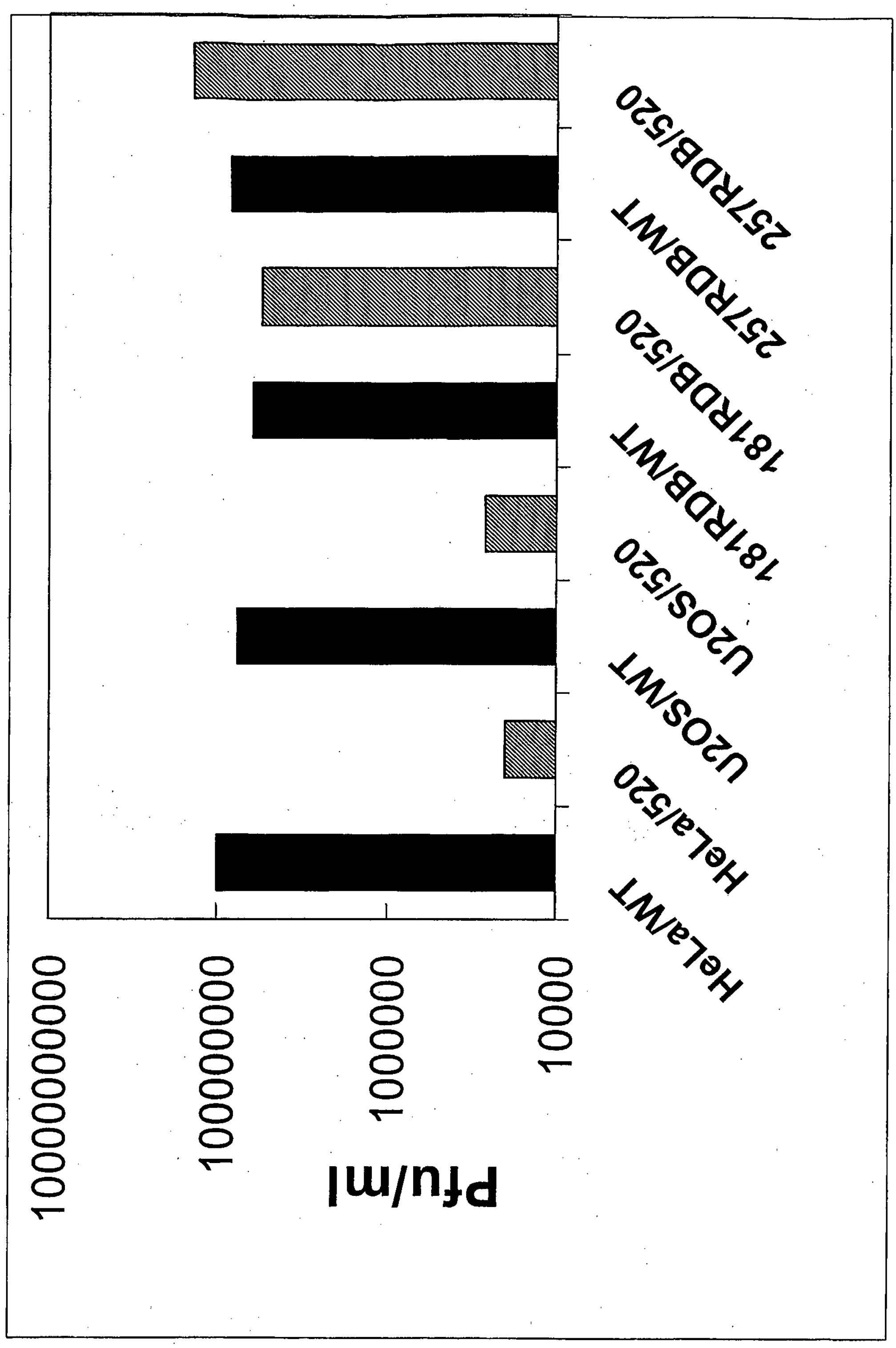

Pfu/ml
10000000000
100000000
1000000
10000
HeLa/WT
HeLa/520
U2OS/WT
U2OS/520
181RDB/WT
181RDB/520
257RDB/WT
257RDB/520

Fig. 16

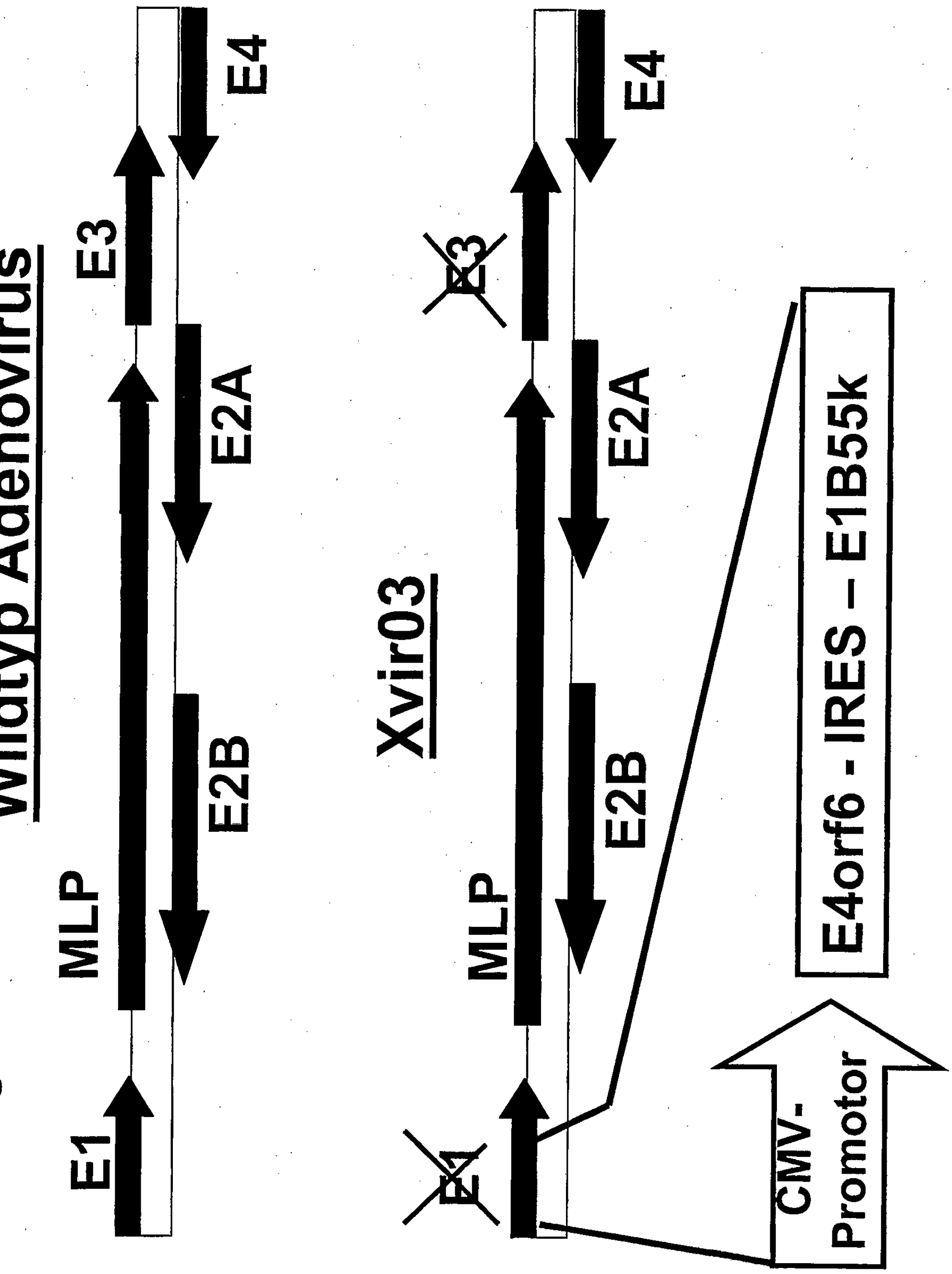

Wildtyp Adenovirus
E1
MLP
E2B
E2A
E3
E4
Xvir03
E1
MLP
E2B
E2A
E3
E4
CMV-Promotor
E4orf6 - IRES – E1B55k

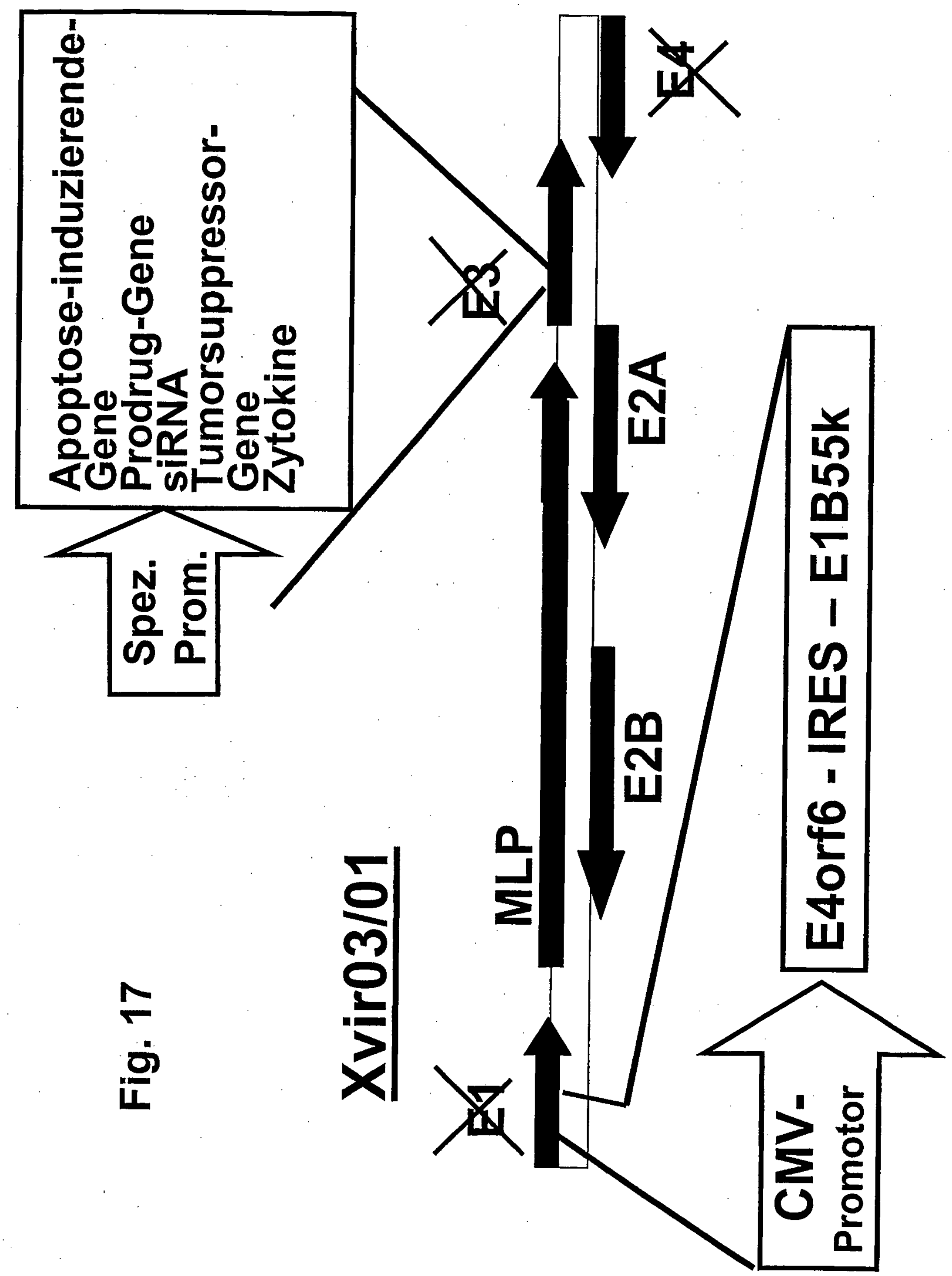
Xvir03/01
Apoptose-induzierende-Gene
Prodrug-Gene
siRNA
Tumorsuppressor-Gene
Zytokine
Spez. Prom.
E4
E3
E2A
E2B
MLP
E1
E4orf6 - IRES – E1B55k
CMV-Promotor

Fig. 17

Fig. 18A

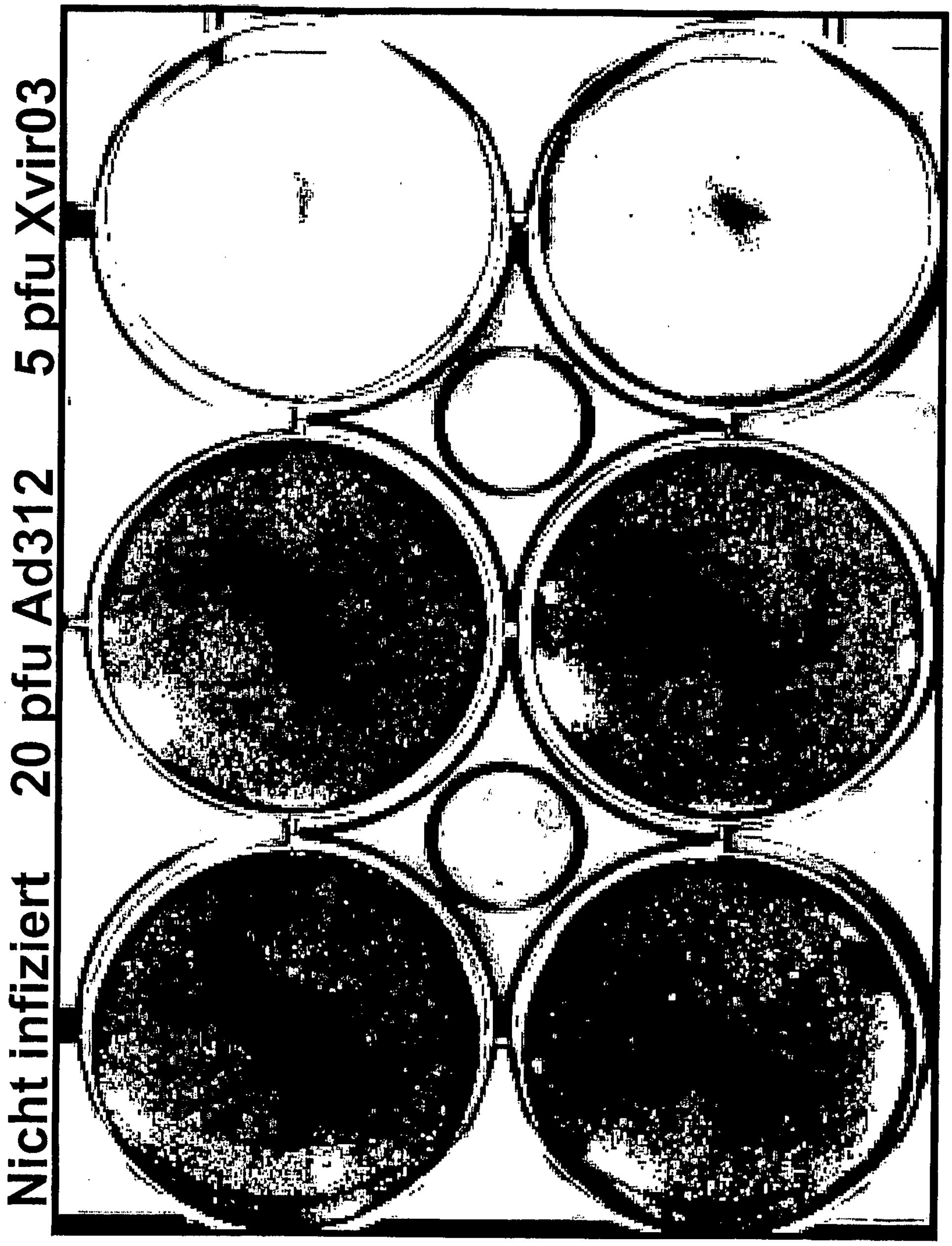

Fig. 18B

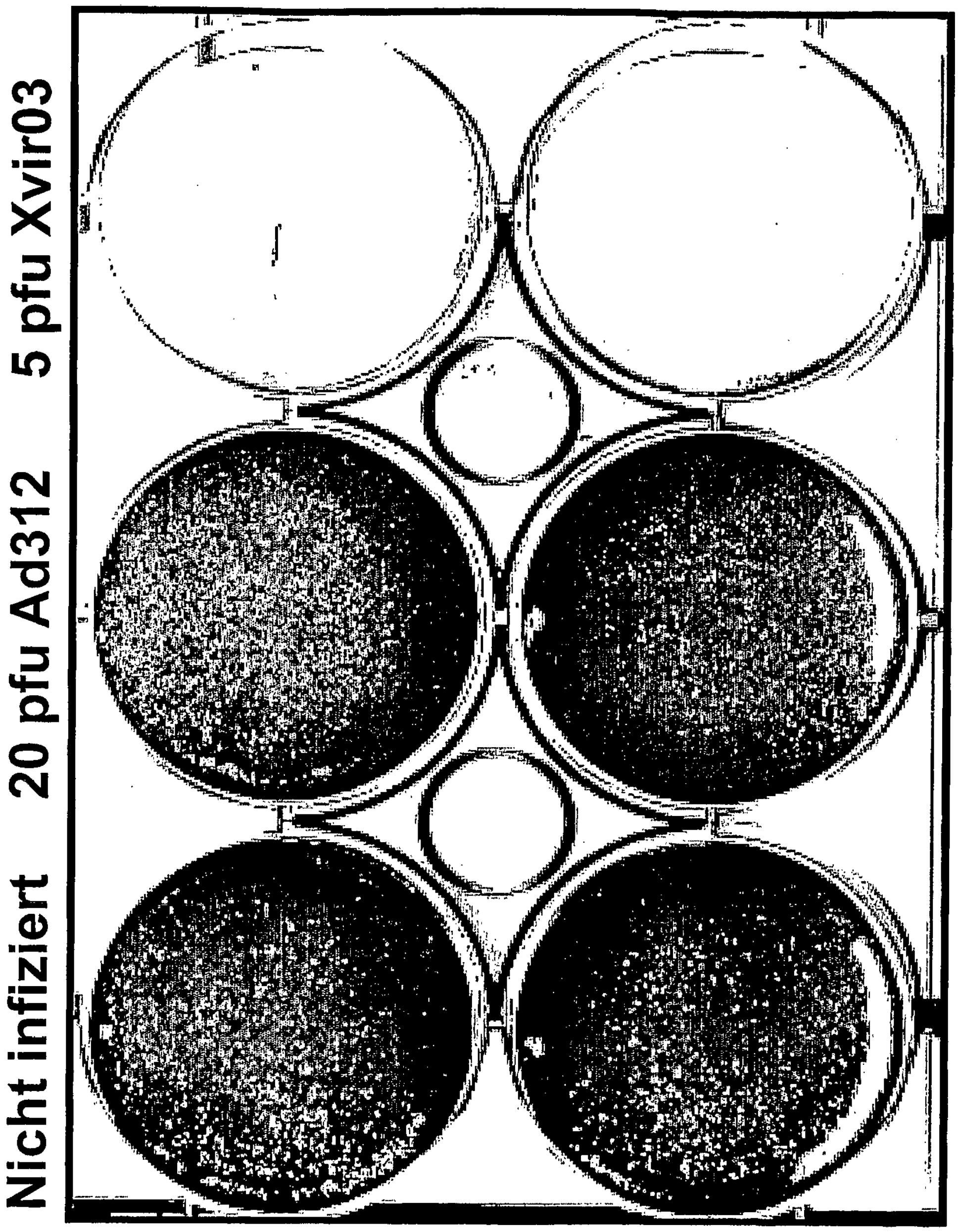

Fig. 19

**U373 Zellen infiziert mit 10 pfu/Zelle dl520 nach 24 h Inkubation mit 5µM Irinotecan**

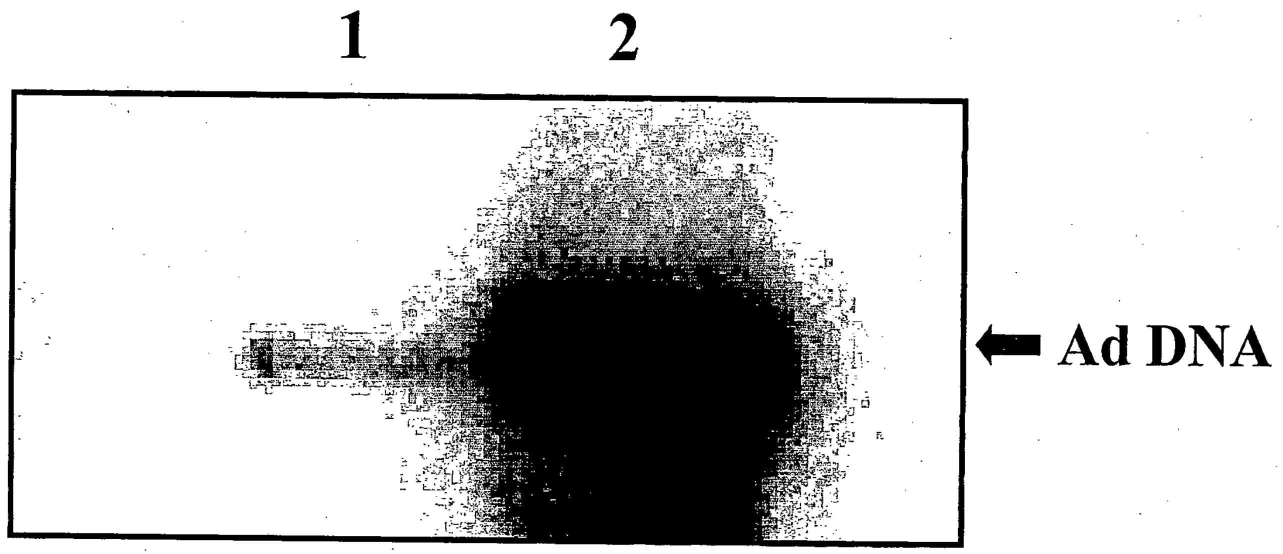


**Southern Blot Analyse**

Fig. 20

U373 Zellen infiziert mit 10 pfu/Zelle dl520 nach 24 h Inkubation mit steigender Trichostatinkonzentrationen

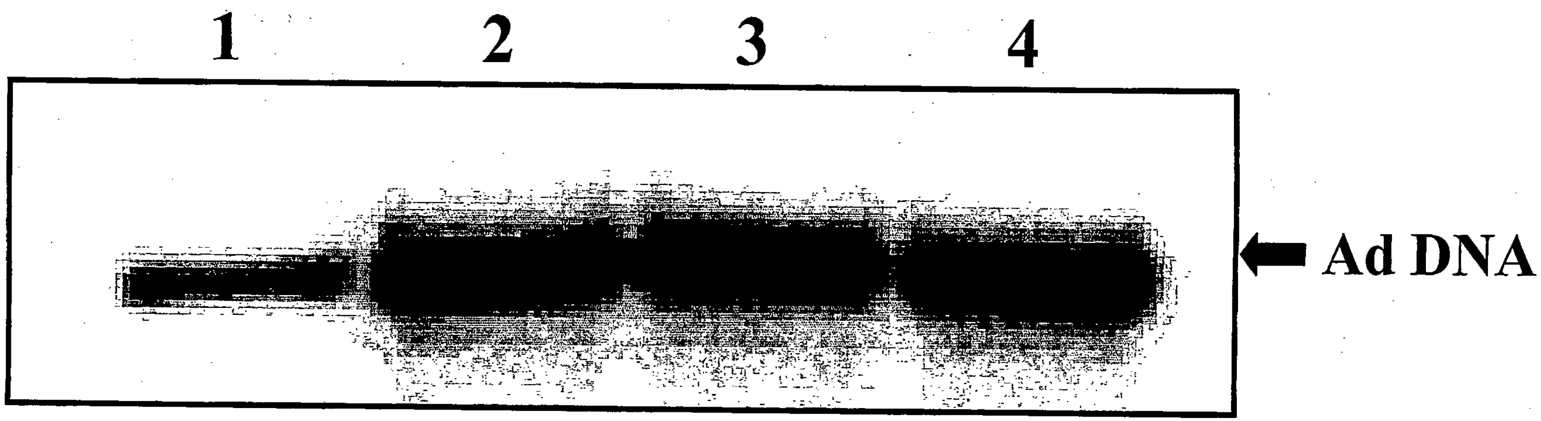


Southern Blot Analyse

Fig. 21

FACS-Analyse: Car-Expression von U373 Zellen nach 24h Inkubation mit Trichostatin

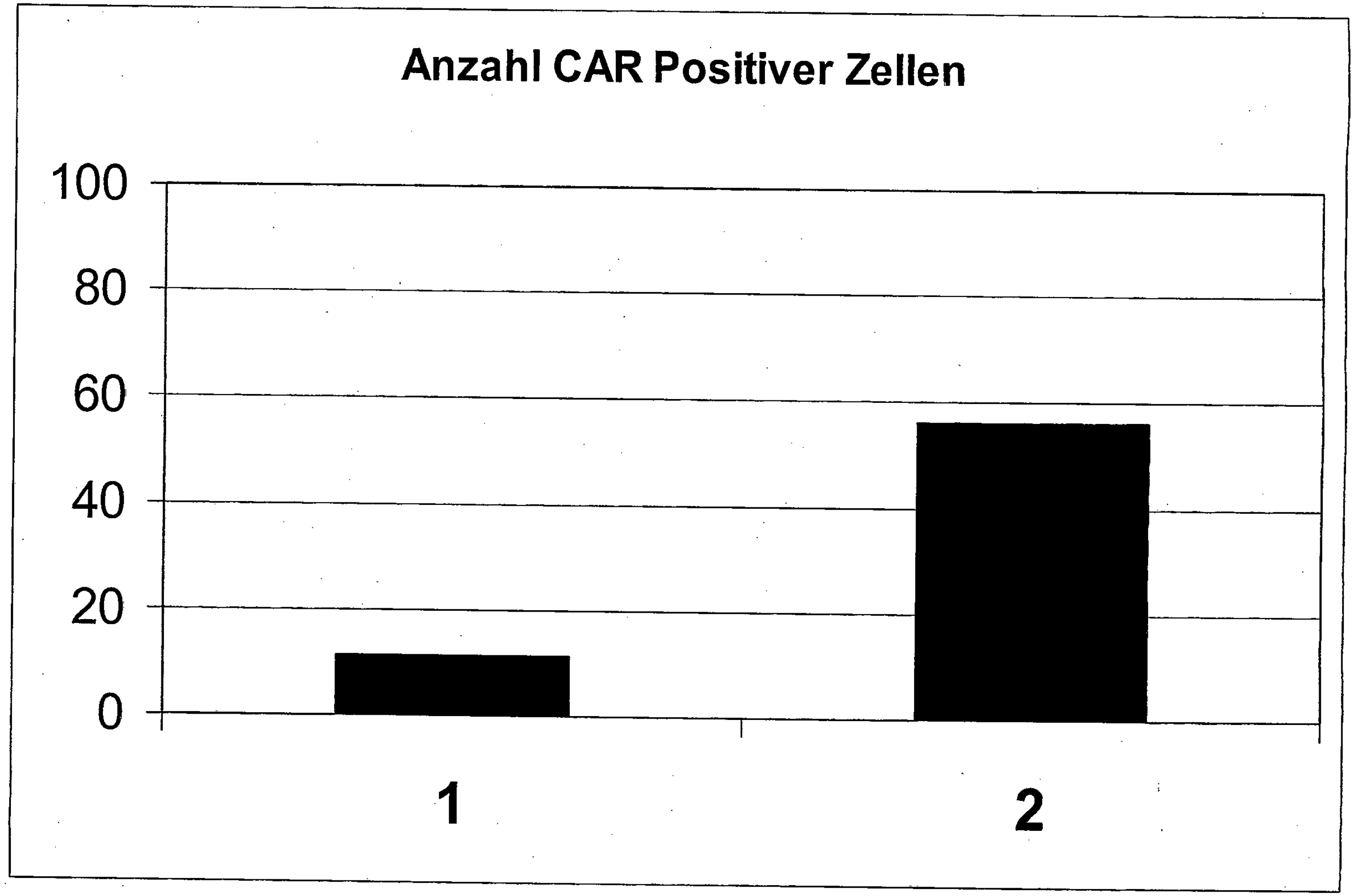

Onkolytischer Effect von dl520 in U373 Zellen in Kombination mit Irinotecan und Trichostatin

Fig. 22

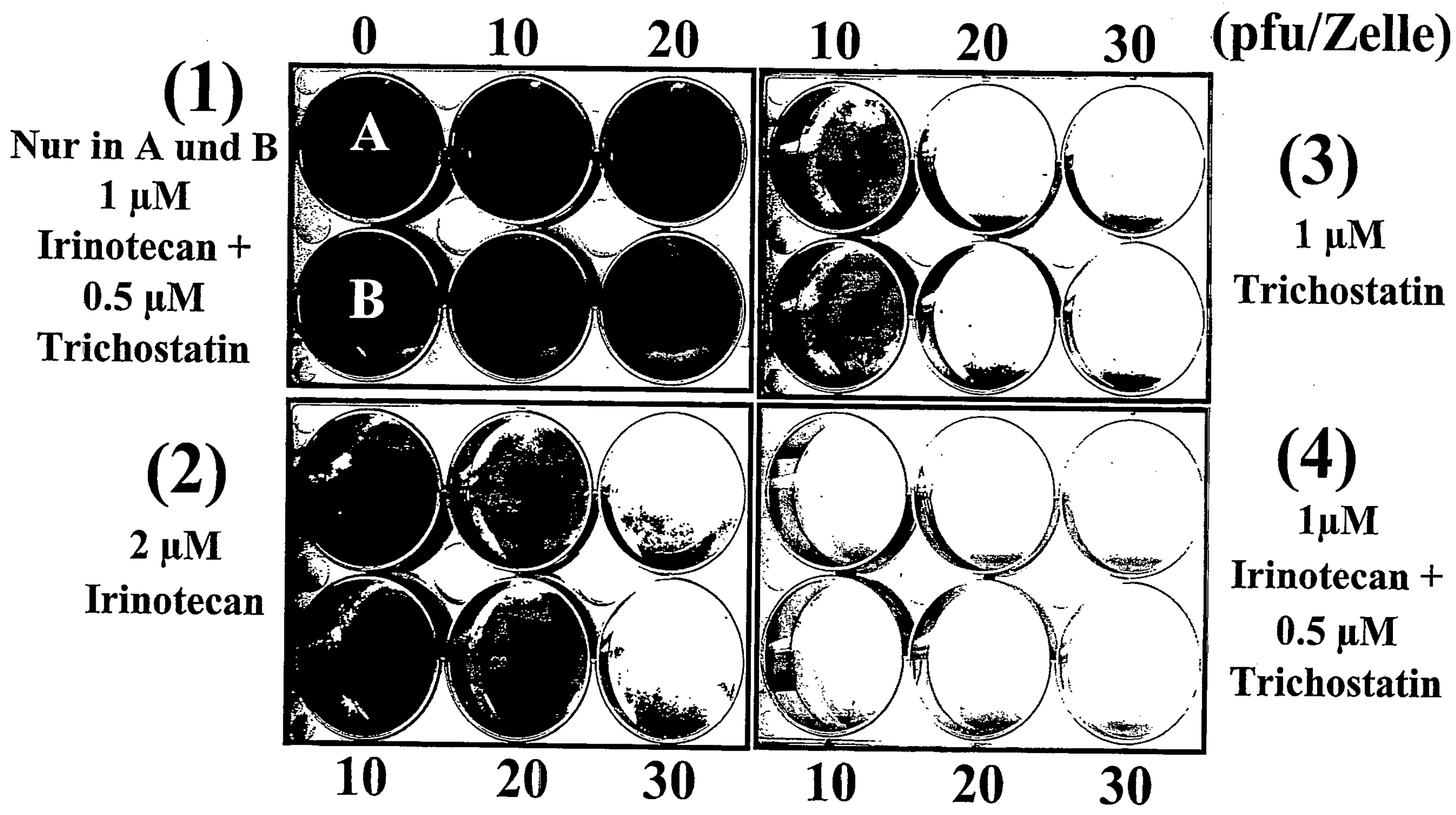

Fig. 23

**E1B55k-Bereich:**
**ORF = 2019 – 3506 bp**
***Bfr*I – Schnittstelle = 3534 bp**
**3'UTR = 3507 – 4107 bp**

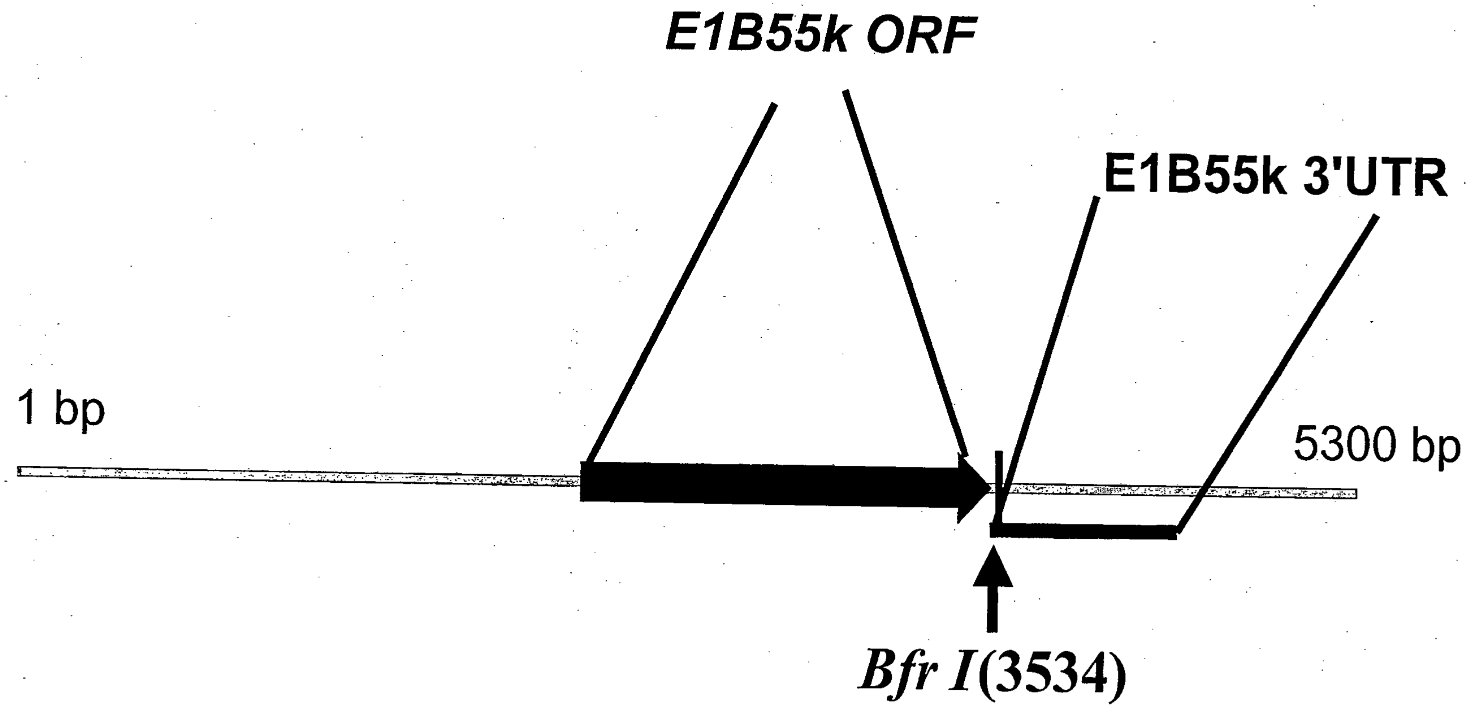

# Fig. 24

5'tgaggtactgaaatgtgtgggcgtggcttaagggtgggaaagaatatataag
gtgggggtcttatgtagttttgtatctgttttgcagcagccgccgccatgagc
accaactcgtttgatggaagcattgtgagctcatatttgacaacgcgcatgcccc
catgggccggggtgcgtcagaatgtgatgggctccagcattgatggtcgcccc
gtcctgcccgcaaactctactaccttgacctacgagaccgtgtctggaacgccg
ttggagactgcagcctccgccgccgcttcagccgctgcagccaccgcccgcg
ggattgtgactgactttgctttcctgagcccgcttgcaagcagtgcagcttcccgt
tcatccgcccgcgatgacaagttgacggctcttttggcacaattggattctttgac
ccgggaacttaatgtcgtttctcagcagctgttggatctgcgccagcaggtttctg
ccctgaaggcttcctcccctcccaatgcggtttaaaacataaataaaaaaccag
actctgtttggatttggatcaagcaagtgtcttgctgtctttatttaggggttttgc

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0931830 A
- US 20020086411 A1
- WO 03033692 A
- DE 10150984
- DE 19742706
- EP 0533838 A
- WO 9808856 A


### In der Beschreibung aufgeführte Nicht-Patentliteratur


- **KHURI, F. et al.** *Nature Medicine,* 2000, vol. 6, 879-885
- **KIRN, D. et al.** *Proc. Am. Soc. Clin. Oncol.,* 1998, vol. 17, 391a
- **ZAMBETTI, G.P. et al.** *FASEB J.,* 1993, vol. 7, 855-865
- **HOWE, J. A. et al.** *Molecular Therapy,* 2000, vol. 2, 485-495
- **FUEYO, J. et al.** *Oncogene,* 2000, vol. 19, 2-12
- **HEISE, C. et al.** *Nature Medicine,* 2001, vol. 6, 11341139
- **BALAGUE, C. et al.** *J. Virol.,* 2001, vol. 75, 7602-7611
- **DYSON, N.** *Genes & Development,* 1998, vol. 12, 2245-2262
- **RODRIGUEZ, R. et al.** *Cancer Res.,* 1997, vol. 57, 2559-2563
- **GOTTESMAN ; PASTAN.** *Annu. Rev. Biochem.,* 1993, vol. 62, 385-427
- **STEIN, U. et al.** *JBC,* 2001, vol. 276, 28562-69
- **J. WIJNHOLDS.** *Novartis Found Symp.,* 2002, vol. 243, 69-79
- **BARGOU, R. C. et al.** *Nature Medicine,* 1997, vol. 3, 447-450
- *Clin. Cancer Res.,* 1998, vol. 4, 2273-2277
- **KOIKE, K. et al.** *FEBS Lett,* 1997, vol. 17, 390-394
- **NOYA, F et al.** *J.Virol.,* 2003, vol. 77 (11), 6533-6540
- **RUSSELL, W. C.** *Journal of Virology,* 2000, vol. 81, 2573-2604
- **TOLLEFSON, A. et al.** *J. Virology,* 1996, vol. 70, 2296-2306
- **RAMYA SUNDARARAJAN ; EILEEN WHITE.** *Journal of Virology,* 2001, vol. 75, 7506-7516
- **KIRN et al.** *Trends in Molecular Medicine,* 2002, vol. 8 (4
- **WYBRANIETZ W.A. et al.** *Gene Therapy,* 2001, vol. 8, 1654-1664
- **NICULESCU-DUVAZ et al.** *Curr. Opin. Mol. Therapy,* 1999, vol. 1, 480-486
- **KOYAMA et al.** *Cancer Gene Therapy,* 2000, vol. 7, 1015-1022
- **ROGERS et al.** *Human Gene Therapy,* 1996, vol. 7, 2235-2245
- **LOCKETT et al.** *Clinical Cancer Res.,* 1997, vol. 3, 2075-2080
- **VIJAYAKRISHNA et al.** *J. Pharmacol. And Exp. Therapeutics,* 2003, vol. 304, 1280-1284
- Gene Therapy, Advances in Pharmacology. Academic Press, vol. 40
- **ZHANG ; DEGROOT.** *Endocrinology,* vol. 144, 1393-1398
- **DESCAMPS et al.** *J. Mol. Med.,* 1996, vol. 74, 183-189
- **MAJUMDAR et al.** *Cancer Gene Therapy,* 2000, vol. 7, 1086-1099
- **TRALHAO et al.** *FASEB J,* 2003, vol. 17, 464-466
- **ZHANG et al.** *Cancer Res.,* 2003, vol. 63, 760-765
- **ZHANG et al.** *Hum. Gene Ther.,* 2002, vol. 20, 2051-2064
- **NOTEBORN ; PIETERSEN.** *Adv. Exp. Med. Biol.,* vol. 465, 153-161
- **TOTH et al.** *Cancer Gene Therapy,* 2003, vol. 10, 193-200
- **SUMANTRAN et al.** *Cancer Res,* 1995, vol. 55, 2507-2512
- **BRAITHWAITE ; RUSSELL.** *Apoptosis,* 2001, vol. 6, 359-370
- **BOEHRINGER MANHEIM.** *Guide to Apoptotic Pathways*
- **ARAI et al.** *PNAC,* 1997, vol. 94, 13862-13867
- **YAMAGUCHI et al.** *Gene Therapy,* 2003, vol. 10, 375-385
- *Oncology News,* 17. Juni 2000
- **OPALKA et al.** *Cell Tissues Organs,* 2002, vol. 172, 126-132
- **JI et al.** *Cancer Res.,* 1999, vol. 59, 3333-3339

- **SU et al.** *Oncogene,* 2003, vol. 22, 1164-1180
- **HAJITOU et al.** *FASEB J.,* 2002, vol. 16, 1802-1804
- **FERRARA, N.** *Semin Oncol,* Dezember 2002, vol. 29 (6), 10-4
- **AHONEN et al.** *Mol Therapy,* 2002, vol. 5, 705-715
- **SOFF et al.** *J. Clin. Invest.,* 1995, vol. 96, 2593-2600
- **BRANDT K.** *Curr. Gene Therapy,* 2002, vol. 2, 255-271
- **ELBASHIR, S ; HARBORTH J ; LENDECKEL W ; YALVCIN, A ; WEBER K ; TUSCHL T.** Duplexes of 21-nucleotide RNAs mediate RNA interference in cultured mammalian cells. *Nature,* 2001, vol. 411, 494-498
- **BRUMMELKAMP et al.** *Science,* 2002, vol. 296, 550-553
- **BEN-ISRAEL ; KLEIBERGER.** *Frontiers in Bioscience,* 2002, vol. 7, 1369-1395
- **HELT ; GALLOWAY.** *Carcinogenesis,* 2003, vol. 24, 159-169
- **WHITTAKER, G.R. et al.** *Virology,* 1998, vol. 246, 1-23
- **FRIEDBERG, E.C.** *TIBS,* 1992, vol. 17, 347
- **JANS, D.A. et al.** *Bioessays,* Juni 2000, vol. 22 (6), 532-44
- **YONEDA, Y.** *J. Biocehm. (Tokyo),* Mai 1991, vol. 121 (5), 811-7
- **BOULIKAS, T.** *Crit. Rev. Eukaryot. Gene Expr.,* 1993, vol. 3 (3), 193-227
- **LYONS RH.** *Mol. Cell Biol.,* 1987, vol. 7, 2451-2456
- **EFTHYMIADIS, A. ; BRIGGS, LJ ; JANS, DA.** *JBC,* 1998, vol. 273, 1623-1628
- **NICKLIN S. A. et al.** *Molecular Therapy,* 2001, vol. 4, 534-542
- **MAGNUSSON, M. K.** *J. of Virology,* 2001, vol. 75, 7280-7289
- **BARNETT B. G. et al.** *Biochimica et Biophysica Acta,* 2002, vol. 1575, 1-14
- **MIZUGUCHI ; HAYAKAWA.** *GENE,* 2002, vol. 285, 69-77
- **VIGUSHIN et al.** *Clinical Cancer Research,* 2001, vol. 7, 971-976
- **KITAZONO et al.** *Cancer Res.,* 2001, vol. 61, 6328-6330
- **JABOIN et al.** *Cancer Res.,* 2002, vol. 62, 6108-6115
- **PLUMB et al.** *Mol. Cancer Ther,* 2003, vol. 8, 721-728
- **ATADJA et al.** *Cancer Res.,* 2004, vol. 64, 689-695
- **GILBERT et al.** *Clinical Cancer Res.,* 2003, vol. 9, 2940-2949
- **VAN HATTUM et al.** *British Journal of Cancer,* 2002, vol. 87, 665-672
- **AVEMANN et al.** *Mol. Cell. Biol.,* 1988, vol. 8, 3026-3034
- **RAJENDRA et al.** *Cancer Res.,* 2003, vol. 63, 3228-3233
- **M. BINASCHI et al.** *Mol. Pharmacol.,* vol. 51, 1053-1059
- Adenoviruses as cloning vectors. **GRUNHAUS, A. ; HORWITZ, M.S.** Seminars in Virology. Saunders Scientific Publications, 1994
- Adenoviridae: The virus and their replication. **SHENK, T. et al.** Fields Virology. Lippincott-Raven Publishers, 1996
- **NEVINS, J. R.** *Cell,* 1981, vol. 26, 213-220
- **WHYTE, P. et al.** *Nature,* 1988, vol. 334, 124-127
- **NEVINS, J. R.** *Science,* 1992, vol. 258, 424-429
- The Molecular Repertoire of Adenoviruses III. **SWAMINATHAN ; THIMMAPAYA.** Current Topics in Microbiology and Immunology. Springer Verlag, 1995, vol. 199, 177-194
- **SWAMINATHAN ; THIMMAPAYA.** *JBC,* 1996, vol. 258, 736-746
- **STEEGENGA, W. T. et al.** *Oncogene,* 1998, vol. 16, 349-357
- **BRIDGE ; KETNER.** *Virology,* 1990, vol. 174, 345-353
- **ORNELLES ; SHENK.** *J. Virology,* 1991, vol. 65, 424-429
- **MARSHALL S. HORWITZ.** *Virololgie,* 2001, vol. 279, 1-8
- **TOLLEFSON.** *J. Virology,* 1996, vol. 70, 2296-2306
- **DORONIN et al.** *J. Virology,* 2000, vol. 74, 6147-6155
- **NEVINS J. R.** *Cell,* 1981, vol. 26, 213-220
- **BOULANGER, P. ; BLAIR, E.** *Biochem. J.,* 1991, vol. 275, 281-299
- **WONG HK ; ZIFF EB.** *J Virol.,* 1994, vol. 68, 4910-20
- **MYMRYK, J. S. ; BAYLEY, S. T.** *Virus Research,* 1994, vol. 33, 89-97
- **WEIGEL, S. ; DOBBELSTEIN , M.** *J. Virology,* 2000, vol. 74, 764-772
- **KEITH N. LEPPARD.** *Seminars in Virology,* 1998, vol. 8, 301-307
- **WOLFFE, A. P.** *Trends in Cell Biology,* 1998, vol. 8, 318-323
- **SWAMYNATHAN, S. K. et al.** *FASEB J.,* 1998, vol. 12, 515-522
- **OKAMOTO, T. et al.** *Oncogene,* 2000, vol. 19, 6194-6202
- **LASHAM, A. et al.** *Gene,* 2000, vol. 252, 1-13
- **MERTENS. P. R. et al.** *JBC,* 1997, vol. 272, 22905-22912

- **SHIBAO, K. et al.** *Int. J. Cancer,* 1999, vol. 83, 732-737
- **CHEN, C-Y. et al.** *Genes & Development,* 2000, vol. 14, 1236-1248
- **OHGA, T. et al.** *Cancer Res.,* 1996, vol. 56, 4224-4228
- **HOLM, P. S. et al.** *JBC,* 2002, vol. 277, 10427-10434
- **GOODRUM, F. D. ; ORNELLES, D. A.** *J. Virology,* 1999, vol. 73, 7474-7488
- **STEIN U ; JURCHOTT K ; WALTHER W ; BERGMANN S ; SCHLAG PM.** *J Biol Chem.,* 2001, vol. 276 (30), 28562-9
- **HU Z ; JIN S ; SCOTTO KW.** *J Biol Chem.,* 28. Januar 2000, vol. 275 (4), 2979-85
- **OHGA T ; UCHIUMI T ; MAKINO Y ; KOIKE K ; WADA M ; KUWANO M ; KOHNO K.** *J Biol Chem.,* 1998, vol. 273 (11), 5997-6000
- **MAKINO Y. et al.** *Nucleic Acids Res.,* 1996, vol. 15, 1873-1878
- **CAO, G. ; KURIYAMA, S. ; GAO, J. ; MITORO, A. ; CUI, L. ; NAKATANI, T. ; ZHANG, X. ; KIKUKAWA, M. ; PAN, X. ; FUKUI, H.** *Int. J. Cancer,* 1998, vol. 78, 242-247
- **CHUNG, I. ; SCHWARTZ, PE. ; CRYSTAL, RC. ; PIZZORNO, G ; LEAVITT, J. ; DEISSEROTH, AB.** *Cancer Gene Therapy,* 1999, vol. 6, 99-106
- **COULSON, JM ; STALEY, J. ; WOLL, PJ.** *British J. Cancer,* 1999, vol. 80, 1935-1944
- **TSUKADA et al.** *Cancer Res.,* vol. 62, 3428-3477
- **ZHANG et al.** *Cancer Res.,* 2002, vol. 62, 3743-3750
- **HALLENBECK PL ; CHANG, YN ; HAY, C ; GOLIGHTLY, D. ; STEWART, D. ; LIN, J. ; PHIPPS, S. ; CHIANG, YL.** *Human Gene Therapy,* 1999, vol. 10, 1721-1733
- **BRAUNSTEIN, I. et al.** *Cancer Research,* 2001, vol. 61, 5529-5536
- **MAJUMDAR, A. S. et al.** *Gene Therapy,* 2001, vol. 8, 568-578
- **DOBBELSTEIN, M. et al.** *EMBO Journal,* 1997, vol. 16, 4276-4284
- **HASAN S. et al.** *Nature,* 2001, vol. 15, 387-391
- **SHIBAO K et al.** *Int. Cancer,* 1999, vol. 83, 732-737
- **ODA Y et al.** *Clin. Cancer Res.,* 1998, vol. 4, 2273-2277
- **KAMIYA, M. ; NAKAZATP, Y.** *J Neurooncology,* 2002, vol. 59, 143-149
- **STIEWE et al.** *J. Biol. Chem.,* 2003, vol. 278, 14230-14236
- **PIGANEAU, N. et al.** *Angew. Chem. Int. Ed.,* 2000, vol. 39 (29), 4369-4373
- **WONG ; ZIFF.** *J. Virol.,* 1994, vol. 68, 4910-4920
- **PELLETIER, J. ; SONENBERG, N.** *Nature,* 1988, vol. 334, 320-325
- **DOBBELSTEIN et al.** *EMBO,* 1997, vol. 16, 4276-4284